(19) Europäisches Patentamt
European Patent Office
Office européen des brevets

(11) EP 3 882 248 A1

(12) EUROPEAN PATENT APPLICATION
published in accordance with Art. 153(4) EPC

(43) Date of publication:
**22.09.2021 Bulletin 2021/38**

(21) Application number: **19881421.2**

(22) Date of filing: **08.11.2019**

(51) Int Cl.:
*C07D 487/04* (2006.01) *C07D 519/00* (2006.01)
*G01N 30/26* (2006.01) *G01N 30/34* (2006.01)
*G01N 30/88* (2006.01)

(86) International application number:
**PCT/JP2019/043926**

(87) International publication number:
**WO 2020/096050 (14.05.2020 Gazette 2020/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.11.2018 PCT/JP2018/041744**
**11.03.2019 JP 2019044236**

(71) Applicant: **Taiho Pharmaceutical Co., Ltd.**
**Chiyoda-ku**
**Tokyo 101-8444 (JP)**

(72) Inventors:
- **KANAMOTO, Yuji**
**Tokushima-shi, Tokushima 771-0194 (JP)**
- **OTA, Reina**
**Tokushima-shi, Tokushima 771-0194 (JP)**
- **HASHIMOTO, Masaya**
**Tsukuba-shi, Ibaraki 300-2611 (JP)**
- **KANDA, Kohei**
**Kodama-gun, Saitama 367-0241 (JP)**

(74) Representative: **Hoffmann Eitle**
**Patent- und Rechtsanwälte PartmbB**
**Arabellastraße 30**
**81925 München (DE)**

# (54) DIMETHOXYBENZENE COMPOUND ANALOGS, METHODS FOR ANALYZING SAID COMPOUNDS AND STANDARD PRODUCTS OF SAID COMPOUNDS

(57) A compounds represented by any one of the following formulas (1) to (5) or a salt thereof, or a combination thereof:
(1) 1,3-bis((S)-3-(4-amino-3-((3,5-dimethoxyphenyl)ethynyl)-1H-pyrazolo[3,4-d]pyrimidin-1-yl)pyrrolidin-1-yl)propan-1-one;
(2) 1-((S)-3-(4-((3-((S)-3-(4-amino-3-((3,5-dimethoxyphenyl)ethynyl)-1H-pyrazolo[3,4-d]pyrimidin-1-yl)pyrrolidin-1-yl)-3-oxopropyl)amino)-3-((3,5-dimethoxyphenyl)ethynyl)-1H-pyrazolo[3,4-d]pyrimidin-1-yl)pyrrolidin-1-yl)prop-2-en-1-one;
(3) (S)-3-((1-(1-acryloylpyrrolidin-3-yl)-6-((3,5-dimethoxyphenyl)ethynyl)-1H-pyrazolo[3,4-d]pyrimidin-4-yl)amino)propanoic acid;
(4) (S)-3-(4-amino-3-((3,5-dimethoxyphenyl)ethynyl)-1H-pyrazolo[3,4-d]pyrimidin-1-yl)pyrrolidine-1-carbaldehyde;
(5)(S)-1-(3-(4-amino-3-((3,5-dimethoxyphenyl)ethynyl)-1H-pyrazolo[3,4-d]pyrimidin-1-yl)pyrrolidin-1-yl)-3-hydroxypropan-1-one.

EP 3 882 248 A1

## Description

Technical Field

Cross-Reference to Related Patent Applications

The present application claims priority to a specification, the international application number of which is PCT/JP2018/041744, filed on November 9, 2018, and Japanese Patent Application No. 2019-044236, filed on March 11, 2019, the entire disclosures of which are hereby incorporated by reference. The present invention relates to related substances of a dimethoxybenzene compound, a method for analyzing the compound, and a standard of the compound.

Background Art

To ensure the constant supply of a pharmaceutical product with appropriate quality assurance, it is necessary to confirm whether the active ingredient is appropriately contained in the pharmaceutical product and whether the impurities contained are controlled to below a certain level. In particular, the control of impurities in pharmaceutical products is very important to ensure not only the quality of pharmaceutical products but also the safety of patients. Thus, an analytical method capable of appropriately assessing the amount of related substances (impurities) contained in pharmaceutical products is one of the methods prioritized over other analytical methods in quality control.

The compound (S)-1-(3-(4-amino-3-((3,5-dimethoxyphenyl)ethynyl)-1H-pyrazolo[3,4-d]pyrimidin-1-yl)pyrrolidin-1-yl)-2-propen-1-one ("compound A" below in this specification) is reported as having excellent inhibitory activity on the fibroblast growth factor receptor (FGFR) and exhibiting antitumor activity (PTL 1 to 5). A method for synthesizing compound A by using (S)-3-((3,5-dimethoxyphenyl)ethynyl)-1-(pyrrolidin-3-yl)-1H-pyrazolo[3,4-d]pyrimidin-4-amine ("compound B" in this specification) and acryloyl chloride has been disclosed. A method for analyzing compound A and like compounds by using ACQUITY SQD (produced by Waters Corporation) has been disclosed (PTL 4). It has also been reported that compound A can be obtained in the form of crystals by using various solvents, and that these crystals were dissolved and analyzed by using the 1200 Series Binary LC System (Agilent Technologies) and ACQUITY SQD (Waters Corporation) (PTL 4).

Citation List

Patent Literature

PTL 1: WO2013/108809A
PTL 2: WO2015/008844A
PTL 3: WO2015/008839A
PTL 4: WO2016/159327A
PTL 5: WO2017/150725A

Summary of Invention

Technical Problem

An object of the present invention is to provide a method for producing compound A or a pharmaceutically acceptable salt thereof that enables mass synthesis of compound A or a pharmaceutically acceptable salt thereof, is simple and excellent in ease of use, and satisfies the quality required for pharmaceutical products.

However, even with this production method, inevitable related substances are present, and quality control is necessary to maintain the quality required for pharmaceutical products. Thus, another object of the present invention is to provide a standard of these related substances for use in quality control for determining whether a pharmaceutical product meets the required quality.

Another object of the present invention is to provide an analysis method for appropriately detecting the content of these related substances in a drug substance or a preparation for use in quality control.

Solution to Problem

The inventors conducted extensive research and found a method for producing compound A or a pharmaceutically acceptable salt thereof that is capable of mass production of compound A or a pharmaceutically acceptable salt with suitable quality as a pharmaceutical product. The inventors also found related substances of compound A usable as a standard in confirming the quality of compound A. The inventors also found an analysis method capable of controlling the quality of compound A using high-performance liquid chromatography.

Specifically, the present invention includes the following [1] to [12].

[1] A compound represented by any one of the following formulas (1) to (5) or a salt thereof, or a combination thereof

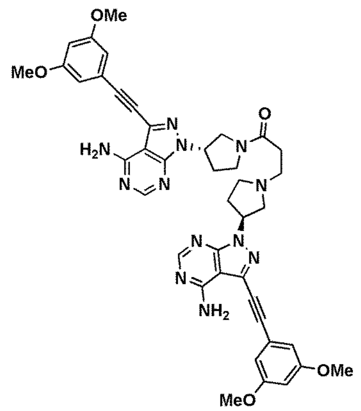

(1)

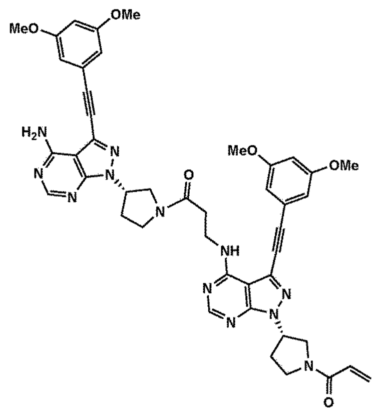

(2)

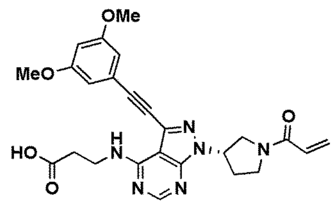

(3)

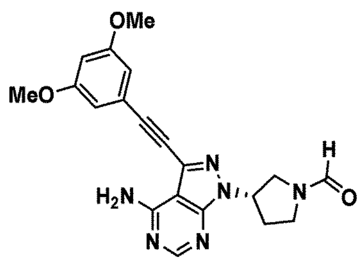

(4)

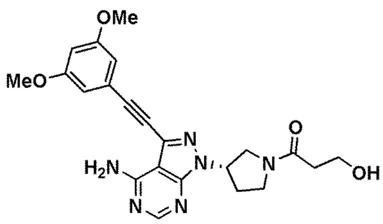

(5)

[2] The compound or a salt thereof, or a combination thereof according to [1], wherein the compound is represented by formula (1) or (2).

[3] A compound represented by any one of the following formulas (1) to (5) or a salt thereof, or a combination thereof, the compound being for use as a standard for controlling quality of (S)-1-(3-(4-amino-3-((3,5-dimethoxyphenyl)ethynyl)-1H-pyrazolo[3,4-d]pyrimidin-1-yl)pyrrolidin-1-yl)-2-propen-1-one

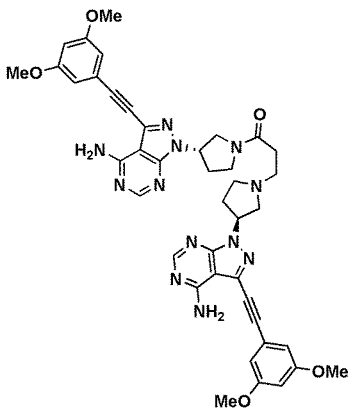

(1)

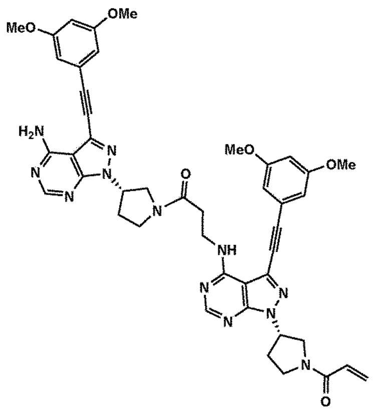

(2)

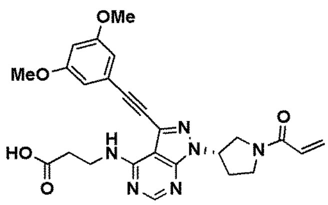

(3)

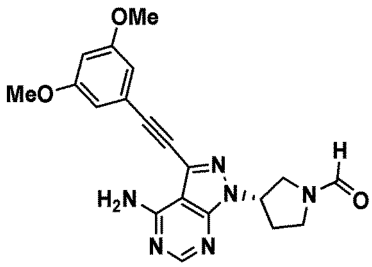

(4)

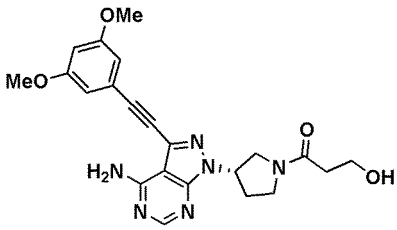

(5)

.

[4] A compound represented by the following formula (1) or (2) or a salt thereof, or a combination thereof, the compound being for use as a standard for controlling quality of (S)-1-(3-(4-amino-3-((3,5-dimethoxyphenyl)ethynyl)-1H-pyrazolo[3,4-dJpyrimidin-1-yl)pyrrolidin-1-yl)-2-propen-1-one

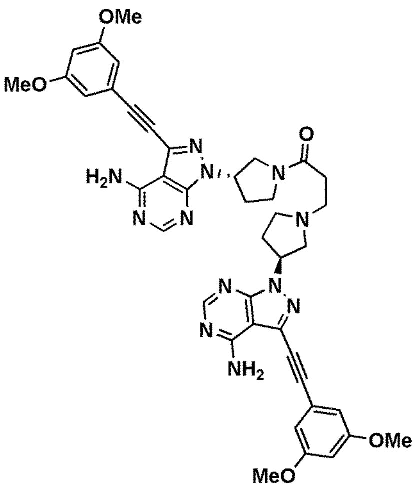

(1)

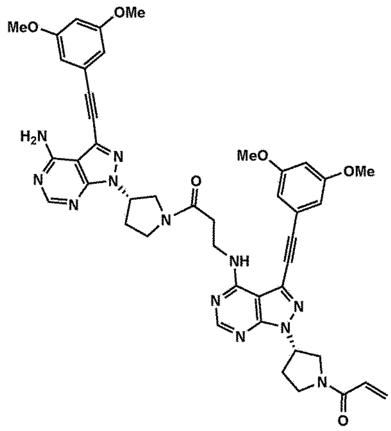

(2)

.

[5] A method for analysis of (S)-1-(3-(4-amino-3-((3,5-dimethoxyphenyl)ethynyl)-1H-pyrazolo[3,4-d]pyrimidin-1-yl)pyrrolidin-1-yl)-2-propen-1-one by high-performance liquid chromatography, wherein resolutions between compounds of the group consisting of a compound represented by the following formula (1), a compound represented by the following formula (2), and (S)-1-(3-(4-amino-3-((3,5-dimethoxyphenyl)ethynyl)-1H-pyrazolo[3,4-d]pyrimidin-1-yl)pyrrolidin-1-yl)-2-propen-1-one are 1.5 or more as measured by high-performance liquid chromatography

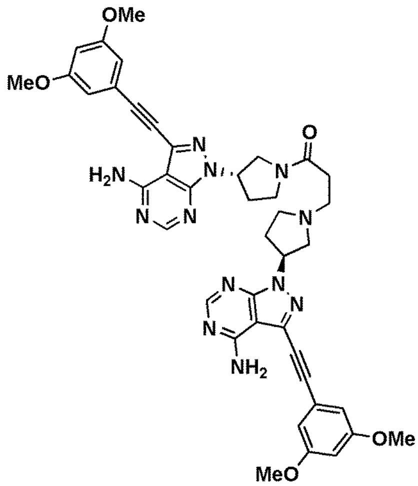

(1)

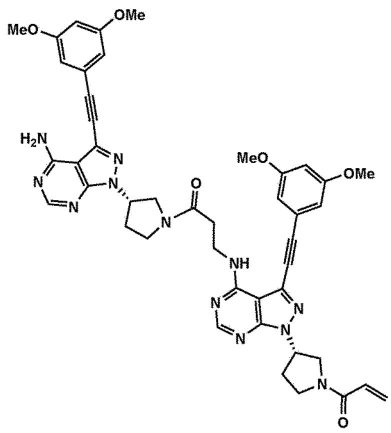

(2)

.

[6] A method for analysis of (S)-1-(3-(4-amino-3-((3,5-dimethoxyphenyl)ethynyl)-1H-pyrazolo[3,4-d]pyrimidin-1-yl)pyrrolidin-1-yl)-2-propen-1-one by high-performance liquid chromatography, wherein resolutions between compounds of the group consisting of a compound represented by the following formula (1), a compound represented by the following formula (2), a compound represented by the following formula (3), a compound represented by the following formula (4), a compound represented by the following formula (5), and (S)-1-(3-(4-amino-3-((3,5-dimethoxyphenyl)ethynyl)-1H-pyrazolo[3,4-d]pyrimidin-1-yl)pyrrolidin-1-yl)-2-propen-1-one are 1.5 or more as measured by high-performance liquid chromatography

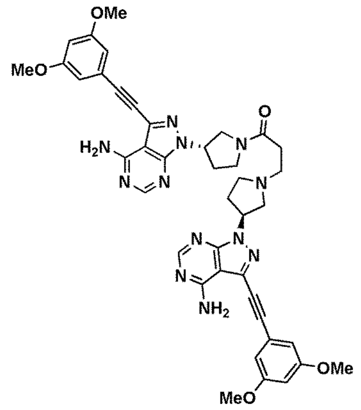

(1)

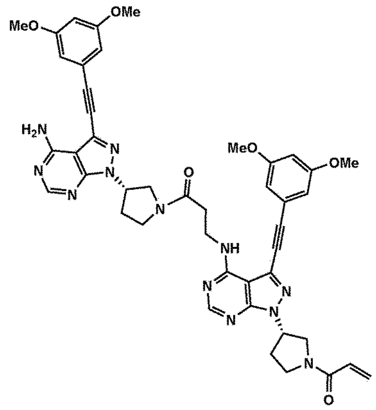

(2)

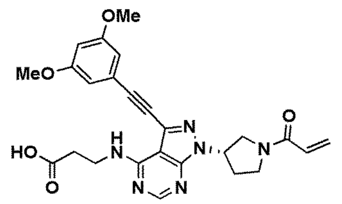

(3)

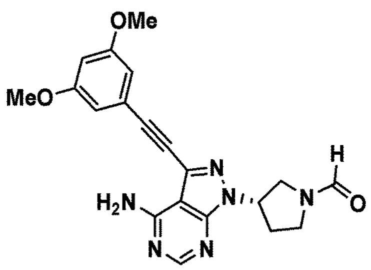

(4)

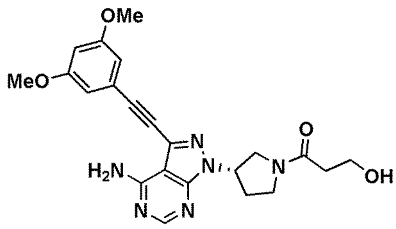

(5)

.

[7] The analysis method according to [5] or [6], wherein a mobile phase comprises a buffer solution whose pH is adjusted to 6.4 or more and 6.8 or less using a phosphoric acid salt.
[8] The analysis method according to any one of [5] to [7], wherein the mobile phase is a mixture of an organic phase and an aqueous phase and comprises a first gradient, a second gradient, and a third gradient, in each of which the percentage of the organic phase in the mobile phase is increased over time at a constant increase rate.
[9] The analysis method according to any one of [5] to [8], wherein the increase rate of the organic phase in the first

gradient is 0.7 to 2.0 volume%/minute, a starting time point of the first gradient is 0 to 5 minutes after a starting time point of the measurement, an ending time point of the first gradient is 5 to 15 minutes after the starting time point of the first gradient, the percentage of the organic phase at the starting time point of the first gradient is 0 to 15 mass% of the entire mobile phase, and the percentage of the organic phase at the ending time point of the first gradient is 5 to 15 mass% of the entire mobile phase;
the increase rate of the organic phase in the second gradient is 0.3 to 2.0 volume%/minute, a starting time point of the second gradient is 0 to 10 minutes after the ending time point of the first gradient, an ending time point of the second gradient is 10 to 20 minutes after the starting time point of the second gradient, the percentage of the organic phase at the starting time point of the second gradient is the same as the percentage of the organic phase at the ending time point of the first gradient, and the percentage of the organic phase at the ending time point of the second gradient is 15 to 45 mass% of the entire mobile phase; and
the increase rate of the organic phase in the third gradient is 1.0 to 6.0 volume%/minute, a starting time point of the third gradient is 0 to 10 minutes after the ending time point of the second gradient, an ending time point of the third gradient is 10 to 20 minutes after the starting time point of the third gradient, the percentage of the organic phase at the starting time point of the third gradient is the same as the percentage of the organic phase at the ending time point of the second gradient, and the percentage of the organic phase at the ending time point of the third gradient is 45 to 75 mass% of the entire mobile phase.

[10] The analysis method according to any one of [5] to [9], wherein an octadecylsilanized silica gel column is used.
[11] The analysis method according to any one of [5] to [10], wherein an aqueous phase comprising acetonitrile in an amount of 15 to 40 volume% based on the total amount of the aqueous phase is used.
[12] The analysis method according to any one of [5] to [11], wherein a mobile phase cleaner is used.

Advantageous Effects of Invention

The present invention enables reliable control of the quality of compound A by using a related substance of compound A as a standard. The present invention also enables efficient control of the quality of compound A or a pharmaceutically acceptable salt thereof required as a pharmaceutical product.

Brief Description of Drawings

Fig. 1 illustrates a chromatogram in Example 1.

Description of Embodiments

In the present invention, compound A is (S)-1-(3-(4-amino-3-((3,5-dimethoxyphenyl)ethynyl)-1H-pyrazolo[3,4-d]pyrimidin-1-yl)pyrrolidin-1-yl)-2-propen-1-one. The structure of compound A is shown below.

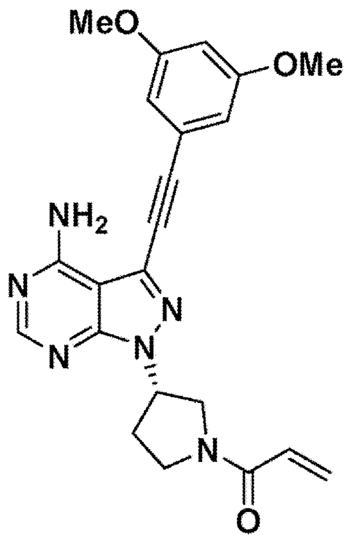

(A)

Compound A or a pharmaceutically acceptable salt thereof may be a solvate (e.g., a hydrate) or a non-solvate. In the present invention, any of such forms are included within the scope of "compound A or a pharmaceutically acceptable salt thereof." The pharmaceutically acceptable salt of compound A is not particularly limited, and examples include addition salts with inorganic acids such as hydrochloric acid and sulfuric acid; addition salts with organic acids such as acetic acid, citric acid, tartaric acid, and maleic acid; salts with alkali metals such as potassium and sodium; salts with alkaline earth metals such as calcium and magnesium; salts with organic bases, such as ammonium salts, ethylamine salts, and arginine salts; and the like. In the present specification, the term "compound A" may be intended to include a pharmaceutically acceptable "salt" and a "solvate" of compound A.

In the present invention, compound B is (S)-3-((3,5-dimethoxyphenyl)ethynyl)-1-(pyrrolidin-3-yl)-1H-pyrazolo[3,4-d]pyrimidin-4-amine. The structure of compound B is shown below.

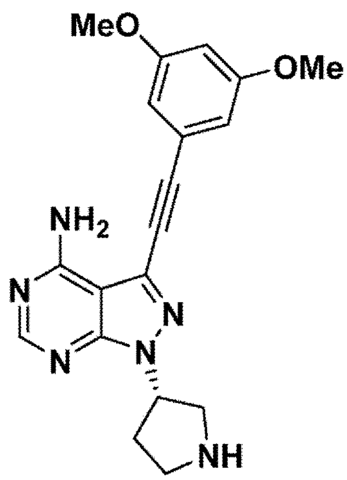

(B)

Compound B or a salt thereof may be a solvate (e.g., a hydrate) or a non-solvate. In the present invention, any of such forms are included within the scope of "compound B or a salt thereof." The salt of compound B is not particularly limited, and examples include addition salts with inorganic acids such as hydrochloric acid, hydrobromic acid, hydroiodic acid, hydrofluoric acid, and sulfuric acid; addition salts with alkyl sulfuric acids such as methanesulfonic acid, p-toluenesulfonic acid, and benzenesulfonic acid; addition salts with organic acids such as acetic acid, citric acid, tartaric acid, and maleic acid; salts with alkali metals such as potassium and sodium; salts with alkaline earth metals such as calcium and magnesium; salts with organic bases, such as ammonium salts, ethylamine salts, and arginine salts; and the like. In the present specification, the term "compound B" may be intended to include a "salt" and "solvate" of compound B.

In the present invention, compound B used for producing compound A may be in free or salt form and is preferably an addition salt with an inorganic acid, an alkyl sulfuric acid, or an organic acid, more preferably an addition salt with an alkyl sulfuric acid, and even more preferably an addition salt with methanesulfonic acid.

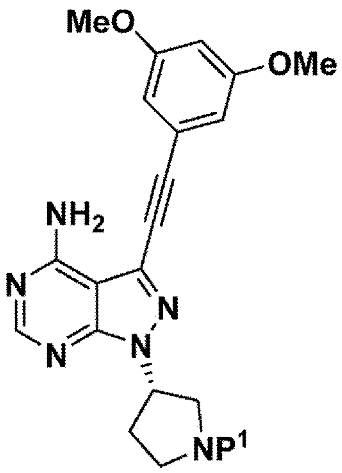

(C)

In the present invention, compound B or a salt thereof is obtained by deprotecting $P^1$ ($P^1$ representing a protecting group of an amino group) of a compound represented by formula (C). The compound represented by formula (C) can be obtained by the method disclosed in WO2013/108809.

Compound B in free form is easily soluble in water, highly water-soluble organic solvents, and highly fat-soluble organic solvents, whereas acid addition salts or base addition salts of compound B have low solubility in organic solvents and tend to be easily isolated and purified.

Examples of the protecting group of an amino group represented by $P^1$ include protecting groups that can be deprotected under acidic conditions, such as a tert-butoxycarbonyl group (Boc group). The method for deprotection of $P^1$, which is a protecting group, can be suitably selected by those skilled in the art. When $P^1$ is a protecting group that can be deprotected under acidic conditions, such as a tert-butoxycarbonyl, the deprotection is preferably performed under acidic conditions. An acid such as hydrochloric acid, methanesulfonic acid, hydrogen iodide, or trifluoroacetic acid may be selected. Methanesulfonic acid is preferable in terms of reaction conditions, ease of use, burden on production equipment, and the like. The amount of acid used is, for example, preferably 1 to 100 moles per mole of the compound represented by formula (C) .

For example, when $P^1$ is a protecting group that can be deprotected under acidic conditions, such as a tert-butoxycarbonyl, compound B can be obtained as an acid addition salt and can be converted to compound A or a pharmaceutically acceptable salt thereof.

In the present invention, compound A or a salt thereof is produced from compound B or a salt thereof by using an acryloylating reagent. As one embodiment of the acryloylating reagent of the present invention, a compound represented by the following formula (I-1-A) or formula (I-2-A) may be used.

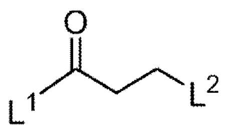

(I－1－A)

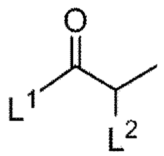

(I－2－A)

wherein $L^1$ and $L^2$ are the same or different, and each represents a leaving group.

In the compound represented by formula (I-1-A), leaving group $L^2$ is attached to the β-position of the carbonyl. In the compound represented by formula (I-2-A), leaving group $L^2$ is attached to the α-position of the carbonyl. In both cases, acryloyl group can be derived under basic conditions, and an acrylamide structure in compound A can be constructed.

Examples of $L^1$, which is a leaving group, include halogen atoms and the like. $L^1$ is preferably a chlorine atom.

Examples of $L^2$, which is a leaving group, include halogen atoms, $-OSO_2C_nF_{n+2}$ (n representing an integer of 1 to 4), mesylate (-OMs; Ms representing mesyl), tosylate (-OTs; Ts representing p-tosyl), nosylate (-ONs; Ns representing p-nosyl), $-OSO_2Ph$ (Ph representing phenyl), phenoxy (-OPh), and the like. $L^2$ is preferably a halogen atom and more preferably a chlorine atom.

Examples of halogen atoms include a fluorine atom, a chlorine atom, a bromine atom, and an iodine atom.

Examples of the compound represented by formula (I-1-A) include 3-chloropropionyl chloride and 3-bromopropionyl chloride.

Examples of the compound represented by formula (I-2-A) include 2-chloropropionyl chloride and 2-bromopropionyl chloride.

As another embodiment of the acryloylating reagent of the present invention, a compound represented by the following formula (I-1-B), formula (I-1-C), formula (I-2-B), or formula (I-2-C) may be used. The compound represented by the following formula (I-1-B), formula (I-1-C), formula (I-2-B), or formula (I-2-C) is an acid anhydride.

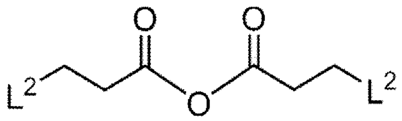

(I-1-B)

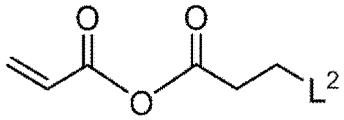

(I-1-C)

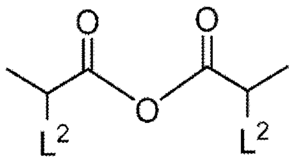

(I-2-B)

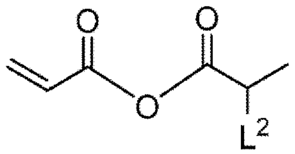

(I-2-C)

wherein each $L^2$ is the same or different, and each represents a leaving group.

Examples of $L^2$ include the leaving groups described above. $L^2$ is preferably a halogen atom and more preferably a chlorine atom.

Examples of the compound represented by formula (I-1-B) include 3-chloropropionic anhydride, 3-bromopropionic anhydride, 3-chloropropionic 3-bromopropionic anhydride, and the like. The compound represented by formula (I-1-B) is preferably 3-chloropropionic anhydride.

Examples of the compound represented by formula (I-1-C) include acrylic 3-chloropropionic anhydride, acrylic 3-bromopropionic anhydride, and the like. The compound represented by formula (I-1-C) is preferably acrylic 3-chloropropionic anhydride.

Examples of the compound represented by formula (I-2-B) include 2-chloropropionic anhydride, 2-bromopropionic anhydride, 2-chloropropionic 2-bromopropionic anhydride, and the like. The compound represented by formula (I-2-B) is preferably 2-chloropropionic anhydride.

Examples of the compound represented by formula (I-2-C) include acrylic 2-chloropropionic anhydride, acrylic 2-bromopropionic anhydride, and the like. The compound represented by formula (I-2-C) is preferably acrylic 2-chloropropionic anhydride.

In the present invention, the acryloylating reagent is preferably a compound represented by formula (I-1-A) or formula (I-2-A), more preferably a compound represented by formula (I-1-A), and even more preferably 3-chloropropionyl chloride.

In the present invention, the amount of the acryloylating reagent, which is a compound represented by formula (I-1-A), formula (I-1-B), formula (I-1-C), formula (I-2-A), formula (I-2-B), or formula (I-2-C), is 1.0 to 1.3 molar equivalents, more preferably 1.05 to 1.3 molar equivalents, even more preferably 1.1 to 1.2 molar equivalents, and particularly preferably 1.1 molar equivalents, per molar equivalent of compound B or a salt thereof. When a base containing a hydroxide ion is used, the acryloylating reagent may be used in an amount of not less than 1.0 molar equivalent, per molar equivalent of compound B or a salt thereof; the acryloylating reagent is preferably used in an amount of 1.0 to 3.0 molar equivalents, more preferably 1.1 to 2.0 molar equivalents, and particularly preferably 1.1 molar equivalents or 1.8 molar equivalents. In the method of the present invention, one $-C(=O)-CH_2-CH_2-L^2$ group is intended to be added per

molecule of compound B or a salt thereof. Accordingly, in the present specification, for example, using 1.0 molar equivalent of the compound represented by formula (I-1-A), the compound represented by formula (I-1-C), or the compound represented by formula (I-2-C) per molar equivalent of compound B or a salt thereof means using 1.0 mole of the acryloylating reagent (acryloylating reagent having one $-C(=O)-CH_2-CH_2-L^2$ group per molecule) per mole of compound B or a salt thereof. Moreover, for example, using 1.0 molar equivalent of the compound represented by formula (I-1-B) or the compound represented by formula (I-2-B) per molar equivalent of compound B or a salt thereof means using 0.5 moles of the acryloylating reagent (acryloylating reagent having two $-C(=O)-CH_2-CH_2-L^2$ groups per molecule) per mole of compound B or a salt thereof. When these acryloylating reagents are used in combination, the above calculations are combined.

In the present specification, "equivalent" means molar equivalent unless it is obvious that it is meant otherwise.

In the present invention, the compound represented by formula (I-1-A), formula (I-1-B), formula (I-1-C), formula (1-2-A), formula (I-2-B), or formula (I-2-C) can be used as the acryloylating reagent. Thus, when the compound represented by formula (I-1-A) or formula (I-2-A) is used, the reaction proceeds in the following two steps, and compound A or a pharmaceutically acceptable salt thereof can be produced.

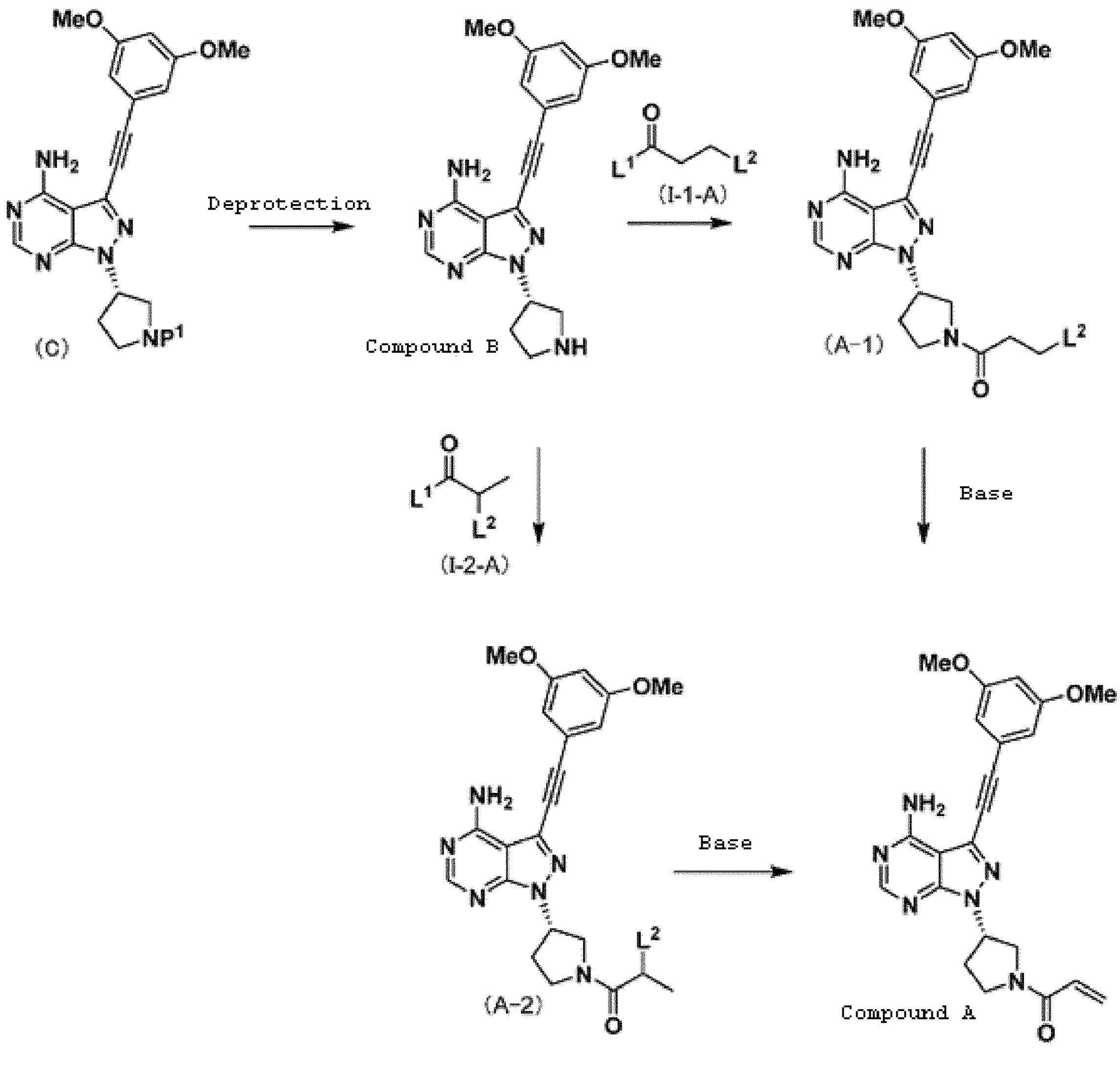


wherein $L^1$ and $L^2$ are the same as above.

When compound A or a pharmaceutically acceptable salt thereof is produced from compound B or a salt thereof, a compound represented by formula (A-1) or formula (A-2), or a salt thereof is obtained as an intermediate. In the present invention, $L^2$ can be eliminated from these intermediates to produce compound A without isolating the intermediates.

For example, when 3-chloropropionyl chloride is used as the compound represented by formula (I-1-A), the compound represented by formula (A-1) is (S)-1-(3-(4-amino-3-((3,5-dimethoxyphenyl)ethynyl)-1H-pyrazolo[3,4-d]pyrimidin-1-yl)pyrrolidin-1-yl)-3-chloropropan-1-one (which hereinafter may be referred to as "A-1-3CP compound").

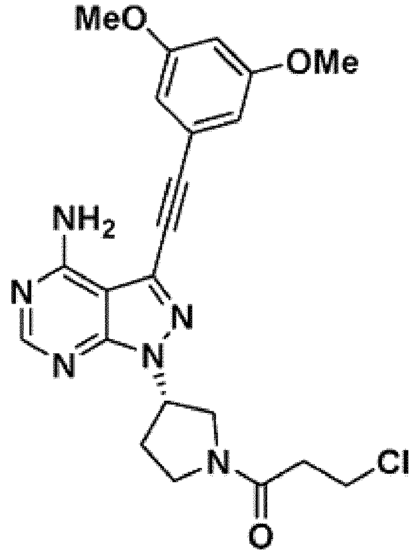

(A-1-3CP compound)

When 2-chloropropionyl chloride is used as the compound represented by formula (I-2-A), the compound represented by formula (A-2) as an intermediate is 1-((S)-3-(4-amino-3-((3,5-dimethoxyphenyl)ethynyl)-1H-pyrazolo[3,4-d]pyrimidin-1-yl)pyrrolidin-1-yl)-2-chloropropan-1-one (which hereinafter may be referred to as "A-1-2CP compound").

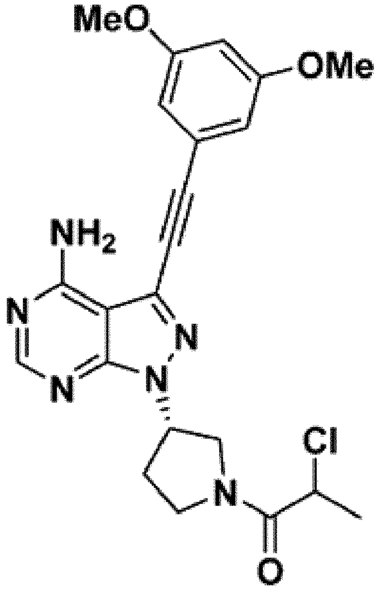

(A-1-2CP compound)

Compound B or a salt thereof, and the compound represented by formula (A-1) or formula (A-2), or a salt thereof can be used to confirm whether the reaction has proceeded when compound A is derived from compound B. Furthermore, since these compounds or salts thereof are possibly contained as impurities in a drug substance and/or pharmaceutical preparation of compound A, they can also be used to determine the presence of impurities.

In the case of using compound A as a pharmaceutical product, the guideline on the amount of compound that can be contained as impurities in a drug substance and/or pharmaceutical preparation of the pharmaceutical product is indicated in the ICH-Q3 guideline of the International Council for Harmonisation of Technical Requirements for Pharmaceuticals for Human Use.

When acryloyl chloride is used in producing compound A or a pharmaceutically acceptable salt thereof from compound B or a salt thereof, (S)-N-(1-(1-acryloylpyrrolidin-3-yl)-3-((3,5-dimethoxyphenyl)ethynyl)-1H-pyrazolo[3,4-d]pyrimidin-4-yl)acrylamide (which hereinafter may be referred to as "diamide compound") may be contained in compound A produced (drug substance of compound A).

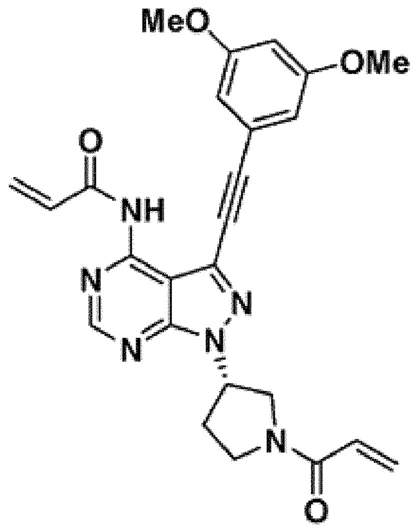

(Diamide compound)

The present inventors tried a method in which acryloyl chloride is used in a smaller amount in order to remove the diamide compound or suppress the formation of the diamide compound, and found that with this method, compound B remained, resulting in a decrease in yield. The inventors also tried a method in which diamide is decomposed by adjusting the pH, and found that with this method, compound A could not be produced in large quantities efficiently because the number of steps was increased and that further, compound A was also decomposed, resulting in a decrease in yield. Furthermore, although crystallization conditions were examined, it was difficult to efficiently remove the diamide compound. In light of the above, it is believed that it is difficult to produce compound A in large quantities while maintaining the quality as a pharmaceutical product in the method for producing compound A by using acryloyl chloride.

Also when the compound represented by formula (A-1) or formula (A-2), or a salt thereof is derived from compound B or a salt thereof using the compound represented by formula (I-1-A), formula (I-1-B), formula (I-1-C), formula (I-2-A), formula (1-2-B), or formula (I-2-C) as the acryloylating reagent, the diamide compound may be obtained as a by-product via a compound represented by formula (A-1-diamide) or formula (A-2-diamide), as shown below.

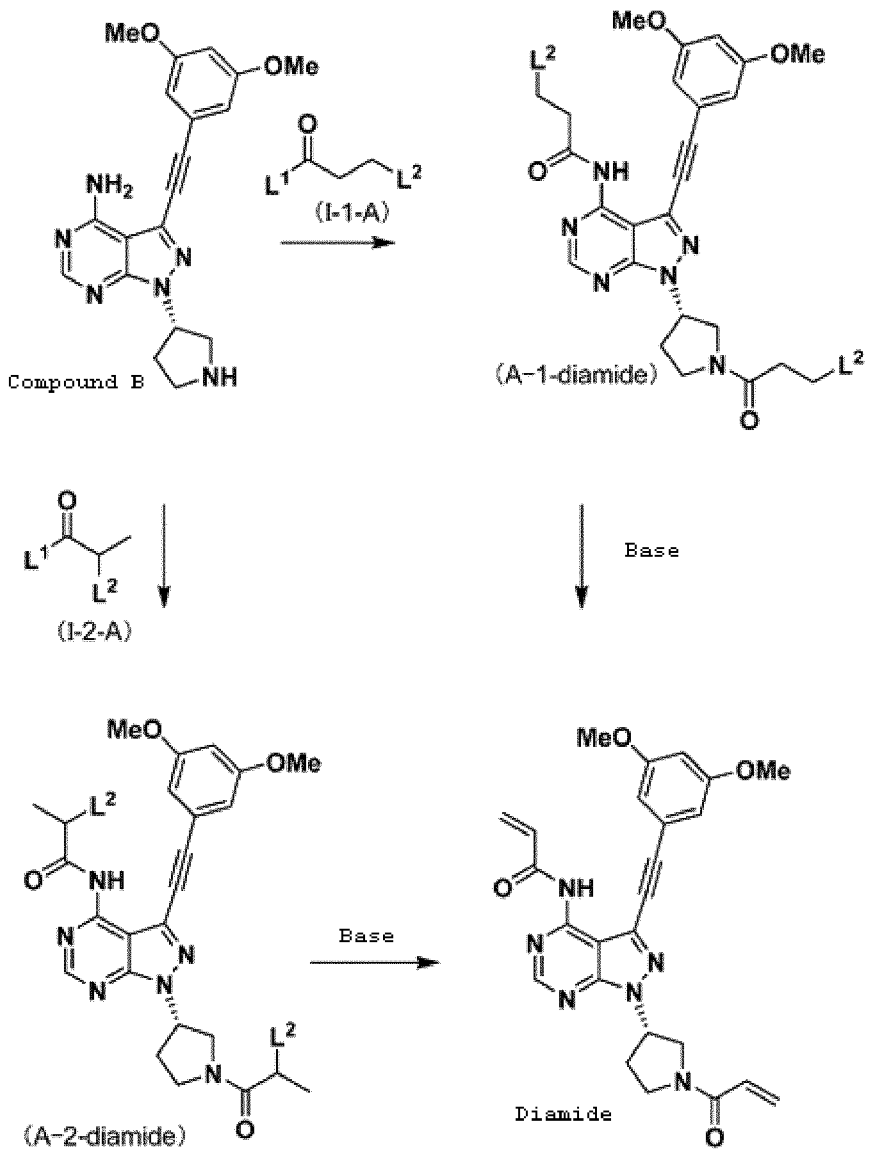


Compound B (I-1-A) (A-1-diamide) (I-2-A) (A-2-diamide) Diamide

wherein $L^1$ and $L^2$ are the same as above.

The diamide or the compound represented by formula (A-1-diamide) or formula (A-2-diamide) can be used to determine the presence of impurities contained in compound A or a pharmaceutically acceptable salt thereof, the compound of formula (A-1) or a salt thereof, or the compound of formula (A-1) or a salt thereof.

For example, when $L^1$ and $L^2$ in the compound represented by formula (I-1-A), formula (I-1-B), formula (I-1-C), formula (I-2-A), formula (I-2-B), or formula (I-2-C) are chlorine, formula (A-1-diamide) is (S)-3-chloro-N-(1-(1-(3-chloropropanoyl)pyrrolidin-3-yl)-3-((3,5-dimethoxyphenyl)ethynyl)-1H-pyrazolo[3,4-d]pyrimidin-4-yl)propanamide (which hereinafter may be referred to as "3CP diamide").

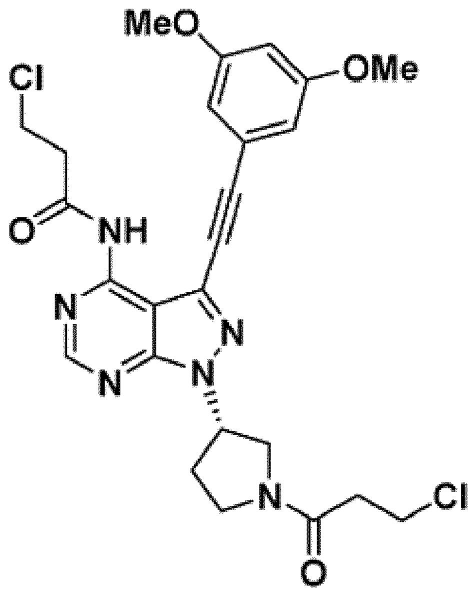

3CP diamide

When $L^1$ and $L^2$ in the compound represented by formula (I-1-A), formula (I-1-B), formula (I-1-C), formula (I-2-A), formula (I-2-B), or formula (I-2-C) are chlorine, formula (A-2-diamide) is 2-chloro-N-(1-((3S)-1-(2-chloropropanoyl)pyrrolidin-3-yl)-3-((3,5-dimethoxyphenyl)ethynyl)-1H-pyrazolo[3,4-d]pyrimidin-4-yl)propanamide (which hereinafter may be referred to as "2CP diamide").

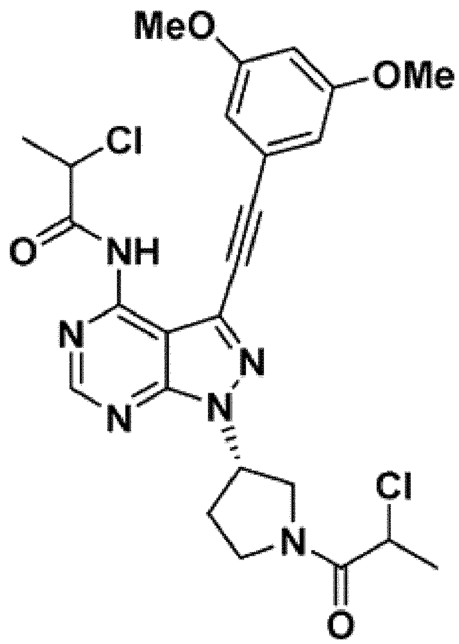

(2CP diamide)

In the present invention, when the compound represented by formula (A-1) or formula (A-2), or a salt thereof is derived as an intermediate from compound B, it may be derived in the presence of a base (e.g., a base in an amount that is at least equivalent to that of compound B). Furthermore, when compound A is derived from these intermediates, it may be derived in the presence of a base (e.g., a base in an amount that is at least equivalent to that of the intermediate). When both steps are performed in the presence of a base, the bases in the steps may be the same or different from each other.

Examples of bases that can be used when the compound represented by formula (A-1) or formula (A-2), or a salt thereof is derived from compound B or a salt thereof include organic amine bases such as trimethylamine, triethylamine, diisopropylethylamine, N-methylmorpholine, diazabicycloundecene (DBU), diazabicyclononene (DBN), pyridine, and 4-dimethylaminopyridine (DMAP); inorganic bases such as lithium hydroxide, sodium hydroxide, magnesium hydroxide, potassium hydroxide, calcium hydroxide, cesium hydroxide, lithium acetate, sodium acetate, magnesium acetate, potassium acetate, calcium acetate, cesium acetate, lithium carbonate, sodium carbonate, magnesium carbonate, potassium carbonate, calcium carbonate, cesium carbonate, lithium hydrogencarbonate, sodium hydrogencarbonate, potassium hydrogencarbonate, cesium hydrogencarbonate, lithium phosphate, sodium phosphate, magnesium phosphate, potassium phosphate, calcium phosphate, and cesium phosphate; and the like. The organic amine bases or inorganic bases are preferable, bases containing a hydroxide ion are more preferable, bases containing an alkali metal ion (e.g., sodium ion or potassium ion) and a hydroxide ion are even more preferable, and sodium hydroxide or potassium hydroxide is still even more preferable. These bases may be used singly or in a combination of two or more.

Examples of bases that can be used when $L^2$ is eliminated from the compound represented by formula (A-1) or formula (A-2) to obtain compound A include the bases described above. The inorganic bases are preferable, bases containing a hydroxide ion are more preferable, bases containing an alkali metal ion (e.g., sodium ion or potassium ion) and a hydroxide ion are even more preferable, and sodium hydroxide or potassium hydroxide is still even more preferable.

The bases described above are roughly classified into monovalent bases, divalent bases, or trivalent bases. A monovalent base is a base that can accept one proton per molecule, and examples include triethylamine, diisopropylethylamine, sodium hydroxide, potassium hydroxide, sodium hydrogencarbonate, and the like. A divalent base is a base that can accept two protons per molecule, and examples include sodium carbonate and the like. A trivalent base is a base that can accept three protons per molecule, and examples include potassium phosphate and the like.

In the present invention, when a monovalent base is used in deriving the compound represented by formula (A-1) or formula (A-2), or a salt thereof from compound B or a salt thereof, the amount of the base is preferably 0.5 to 10 equivalents, more preferably 1 to 10 equivalents, even more preferably 1 to 5 equivalents, still even more preferably 1 to 3 equivalents, further still even more preferably 1 to 2 equivalents, and particularly preferably 1.1 equivalents or 1.9 equivalents, after subtracting the equivalent amount neutralized with an acid addition salt of compound B, per equivalent of compound B or a salt thereof, i.e., relative to compound B in free form. When a monovalent base is used, the amount of the base used in eliminating $L^2$ from the compound represented by formula (A-1) or formula (A-2), or a salt thereof to obtain compound A or a pharmaceutically acceptable salt thereof is preferably 1 to 10 equivalents, more preferably 1 to 5 equivalents, even more preferably 3 to 4 equivalents, and particularly preferably 3.4 equivalents or 3.6 equivalents, per equivalent of compound B or a salt thereof, i.e., relative to compound B in free form. Similarly, for divalent bases and trivalent bases, the optimal equivalent amounts can be calculated according to the above, taking the valence into account.

In the present invention, when a monovalent base is used in eliminating $L^2$ from the compound represented by formula (A-1) or formula (A-2), or a salt thereof to obtain compound A or a pharmaceutically acceptable salt thereof, the amount of the base may be 1 to 5 equivalents, after subtracting the equivalent amount neutralized with an acid addition salt of the compound represented by formula (A-1) or formula (A-2), per equivalent of the compound represented by formula (A-1) or formula (A-2), or a salt thereof, i.e., relative to the compound represented by formula (A-1) or formula (A-2) that is in free form. When the above process is performed without isolating the compound represented by formula (A-1) or formula (A-2), the amount of the base may be 1 to 10 equivalents relative to the theoretical yield of the compound represented by formula (A-1) or formula (A-2) that is in free form (i.e., the amount of compound B in free form). Similarly, for divalent bases and trivalent bases, the optimal equivalent amounts can be calculated according to the above, taking the valence into account.

In the present invention, the solvent used when the compound represented by formula (A-1) or formula (A-2), or a salt thereof is derived from compound B or a salt thereof is not particularly limited as long as it does not interfere with bonding of compound B to the acryloylating reagent. Examples of solvents include acetonitrile, water, N-methyl-2-pyrrolidone, tetrahydrofuran, acetone, N,N-dimethylformamide (DMF), N,N-dimethylacetamide, dimethyl sulfoxide (DMSO), 1,4-dioxane, and mixtures thereof. Acetonitrile, water, or a mixture thereof is preferable. The volume of the solvent is not particularly limited, and the amount of the solvent is preferably 1 to 50 times by volume (v/w), more preferably 2 to 30 times by volume (v/w), even more preferably 10 to 20 times by volume (v/w), and particularly preferably 12 times by volume (v/w) or 14 times by volume (v/w), per 1 weight of compound B or a salt thereof. When a mixture of solvents is used, the proportion of each solvent is not particularly limited. For example, when a mixture of acetonitrile and water is used, the proportion of each solvent is not particularly limited, and the amount of water is preferably 0.1 to 2 times by volume (v/v), more preferably 0.1 to 1 time by volume (v/v), even more preferably 0.5 to 1 time by volume (v/v), and particularly preferably 0.5 times by volume (v/v) or 1 time by volume (v/v), per 1 volume of acetonitrile.

Examples of solvents that can be used when $L^2$ is eliminated from the compound represented by formula (A-1) or formula (A-2), or a salt thereof to obtain compound A or a pharmaceutically acceptable salt thereof include the same solvents as described above. Further, examples of solvents that can be used when this process is performed without isolating the compound represented by formula (A-1) or formula (A-2), or a salt thereof also include the same solvents as described above.

In the present invention, the temperature of the solvent used when the compound represented by formula (A-1) or formula (A-2), or a salt thereof is derived from compound B or a salt thereof is not particularly limited as long as it is between the melting point and the boiling point of the solvent and is within the range in which compound B can be

stably present, and is preferably 0 to 50°C, and more preferably 25 to 35°C.

The temperature used when $L^2$ is eliminated from the compound represented by formula (A-1) or formula (A-2), or a salt thereof to obtain compound A or a pharmaceutically acceptable salt thereof is, for example, within the same range as described above.

In the present invention, a carboxylic acid represented by the following formula (I-1-D) or formula (I-2-D) may be used as a way to derive the compound represented by formula (A-1) or formula (A-2), or a salt thereof from compound B or a salt thereof.

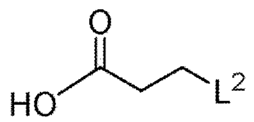

(I-1-D)

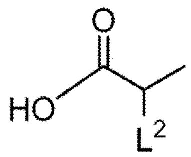

(I-2-D)

wherein $L^1$ and $L^2$ are the same as above.

In this case, the step in which the compound represented by formula (A-1) or formula (A-2), or a salt thereof can be derived from compound B or a salt thereof is as shown below.

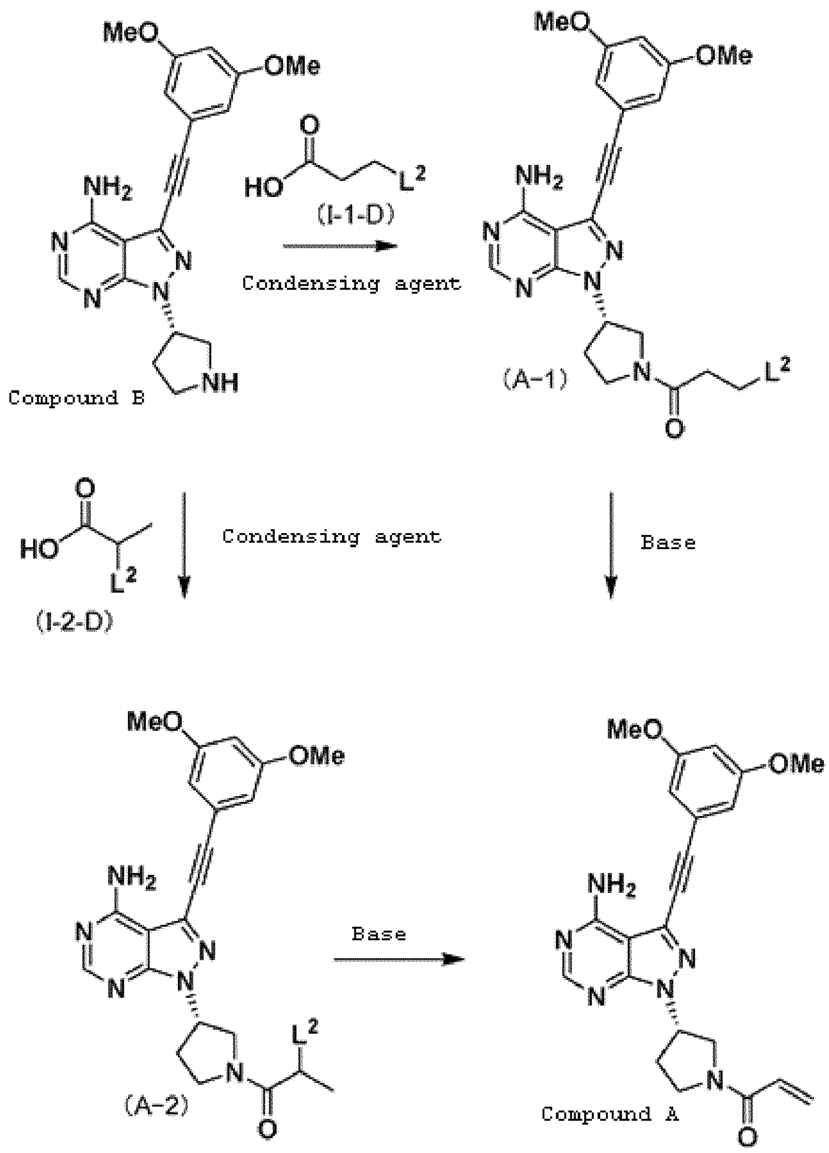


wherein $L^1$ and $L^2$ are the same as above.

Since this step is the condensation of compound B with the carboxylic acid of formula (I-1-D) or formula (I-2-D), a condensing agent can be used. Examples of condensing agents include dicyclohexylcarbodiimide (DCC), diisopropylcarbodiimide (DIC), 1-ethyl-3-(3-dimethylaminopropyl)carbodiimide hydrochloride (EDC, WSCI), benzotriazol-1-yloxy-tris-(dimethylamino)phosphonium hexafluorophosphate (BOP), benzotriazol-1-yloxy-tris-pyrrolidino-phosphonium hexafluorophosphate (PyBOP), (2-(7-aza-1H-benzotriazole-1-yl)-1,1,3,3,-tetramethyluronium hexafluorophosphate (HATU), O-(1H-benzotriazol-1-yl)-N,N,N' ,N'-tetramethyluronium hexafluorophosphate (HBTU), carbonyldiimidazole (CDI), 4-(4,6-dimethoxy-(1,3,5)triazin-2-yl)-4-methylmorpholinium chloride (DMT-MM), and the like.

In this step, p-nitrophenol, pentafluorophenol, 2,4,5-trichlorophenol, 1-hydroxybenzotriazole (HOBt), 1-hydroxy-7-azabenzotriazole (HOAt), N-hydroxysuccinimide (HOSu), or the like may be added in order to convert the carboxylic acid to an activated ester.

Further, in this step, the bases listed above can be used as appropriate.

In the present invention, the reactions in deriving the compound represented by formula (A-1) or formula (A-2), or a salt thereof from compound B or a salt thereof and in eliminating $L^2$ from the compound represented by formula (A-1) or formula (A-2), or a salt thereof to obtain compound A or a pharmaceutically acceptable salt thereof may be confirmed by using, for example, chromatography, such as high-performance liquid chromatography (which hereinafter may be referred to as "HPLC") and thin-layer chromatography (TLC). In the case of using HPLC, when the peak area of compound B is 1% or less of the total peak area, it can be determined that the step of deriving the compound represented by formula (A-1) or formula (A-2), or a salt thereof from compound B or a salt thereof is complete. Further, when the peak area of the compound represented by formula (A-1) or formula (A-2) is 1% or less of the total peak area,

it can be determined that the step of eliminating $L^2$ from the compound represented by formula (A-1) or formula (A-2), or a salt thereof to obtain compound A is complete. The measurement conditions of HPLC are not particularly limited as long as compound A, compound B, and the compound represented by formula (A-1) or formula (A-2) can be detected.

In addition, in the case of using HPLC to confirm the reaction in deriving the compound represented by formula (A-1) or formula (A-2), or a salt thereof from compound B or a salt thereof, when the peak area of impurities and 3CP diamide represented by formula (A-1-diamide) or impurities and 2CP diamide represented by formula (A-2-diamide) is 2% or less of the total peak area, the diamide compound is less likely to be contained in compound A or a pharmaceutically acceptable salt thereof.

In the present invention, when the compound represented by formula (A-1) or formula (A-2), or a salt thereof is isolated, these compounds may be purified by a method such as recrystallization or may be used in the next step without purification. In addition, after the completion of the process in which $L^2$ is eliminated from the compound represented by formula (A-1) or formula (A-2), or a salt thereof to obtain compound A or a pharmaceutically acceptable salt thereof, compound A or a pharmaceutically acceptable salt thereof may be purified. As a purification method, from the viewpoint that the present invention is used for production in large quantities, it is preferable to use crystallization without performing purification by column chromatography.

In the present invention, when compound A or a pharmaceutically acceptable salt thereof dissolved in the reaction solvent is crystallized, for example, a solvent in which compound A or a pharmaceutically acceptable salt thereof has low solubility may be added. Examples of the solvent added include water and the like. The amount of the solvent added is not particularly limited as long as compound A or a pharmaceutically acceptable salt thereof is precipitated, and is preferably 0.5 to 5 times by volume (v/v), more preferably 1 to 3 times by volume (v/v), even more preferably 1 to 2 times by volume (v/v), and particularly preferably 1.1 times by volume (v/v) or 1.8 times by volume (v/v), relative to the volume of the reaction solvent. When compound A or a pharmaceutically acceptable salt thereof is produced from compound B or a salt thereof without isolating the compound represented by formula (A-1) or formula (A-2), or a salt thereof, the amount of the solvent added is 5 to 50 times by volume (v/w), preferably 10 to 40 times by volume (v/w), more preferably 15 to 30 times by volume (v/w), and particularly preferably 15 times by volume (v/w) or 22 times by volume (v/w), relative to the weight of compound B or a salt thereof.

In the present invention, the temperature at which crystallization is performed is not particularly limited as long as compound A or a pharmaceutically acceptable salt thereof is precipitated after the addition of the above solvent, and is preferably 0 to 40°C, and more preferably 20 to 30°C.

Furthermore, in the present invention, the time required for crystallization is, for example, 1 hour or more, and preferably 2 to 72 hours.

In the present invention, compound A or a pharmaceutically acceptable salt thereof may be isolated as a solid by crystallization and filtration. Since compound A or a pharmaceutically acceptable salt thereof is used as a pharmaceutical product, the time required for filtration is preferably short in order to efficiently produce it in large quantities. Since whether filterability is good or bad cannot be determined according to the absolute values of the filtration time, the filtration rate, and the like, it is determined relatively by comparing process conditions. Thus, the filtration areas, filter paper used for filtration, and pressures during suction are made uniform for comparison. No filter paper clogging caused by the precipitation of particles due to their large size, a small amount of solvent filtered, and the like are factors from which it can be determined that the filterability is excellent.

In the present invention, the filterability in filtration of compound A or a pharmaceutically acceptable salt thereof is better when the compound of formula (I-1-A) or formula (I-2-A) is used in producing compound A or a pharmaceutically acceptable salt thereof from compound B or a salt thereof without isolating the compound represented by formula (A-1) or formula (A-2), or a salt thereof, than when acryloyl chloride is used. This would not have been predicted when producing compound A or a salt thereof. In the present invention, the compound represented by formula (I-1-A) or formula (I-2-A) that can be used in terms of filterability is not particularly limited as long as the filterability is improved compared with the case of using acryloyl chloride, and is preferably the compound represented by formula (I-1-A), and more preferably 3-chloropropionyl chloride.

The thus obtained compound represented by formula (A-1) or formula (A-2), or a salt thereof, and compound A or a pharmaceutically acceptable salt thereof can be analyzed by various quantitative analyses and qualitative analyses.

In the present invention, as one embodiment, a method for producing the compound represented by formula (A-1) or a salt thereof, the method comprising reacting 1 equivalent of compound B or a salt thereof with 1.0 to 1.3 equivalents of the compound represented by formula (I-1-A), may be used.

Preferably, the method is for producing the compound represented by formula (A-1) or a salt thereof, comprising reacting 1 equivalent of compound B or a salt thereof with 1.0 to 1.3 equivalents of the compound represented by formula (I-1-A) wherein $L^1$ and $L^2$ are the same or different, and each is selected from the group consisting of halogen atoms.

More preferably, the method is for producing the compound represented by formula (A-1) or a salt thereof, comprising reacting 1 equivalent of compound B or a salt thereof with 1.0 to 1.3 equivalents of 3-chloropropionyl chloride.

More preferably, the method is for producing the compound represented by formula (A-1) or a salt thereof,

comprising reacting 1 equivalent of compound B or a salt thereof with 1.0 to 1.3 equivalents of 3-chloropropionyl chloride in the presence of at least one base selected from the group consisting of organic amine bases and inorganic bases.

More preferably, the method is for producing the compound represented by formula (A-1) or a salt thereof, comprising reacting 1 equivalent of compound B or a salt thereof with 1.0 to 1.3 equivalents of 3-chloropropionyl chloride in the presence of at least one base selected from the group consisting of organic amine bases and bases containing a hydroxide ion.

More preferably, the method is for producing the compound represented by formula (A-1) or a salt thereof, comprising reacting 1 equivalent of compound B or a salt thereof with 1.0 to 1.3 equivalents of 3-chloropropionyl chloride in the presence of at least one base selected from the group consisting of organic amine bases and bases containing a hydroxide ion, the amount of the base after subtracting the equivalent amount neutralized with an acid addition salt of compound B or a salt thereof being 1.0 to 10 equivalents.

More preferably, the method is for producing the compound represented by formula (A-1) or a salt thereof, comprising reacting 1 equivalent of compound B or a salt thereof with 1.0 to 1.3 equivalents of 3-chloropropionyl chloride in the presence of at least one base selected from the group consisting of organic amine bases and bases containing an alkali metal ion and a hydroxide ion, the amount of the base after subtracting the equivalent amount neutralized with an acid addition salt of compound B or a salt thereof being 0.5 to 10 equivalents.

More preferably, the method is for producing the compound represented by formula (A-1) or a salt thereof, comprising reacting 1 equivalent of compound B or a salt thereof with 1.0 to 1.3 equivalents of 3-chloropropionyl chloride in the presence of at least one base selected from the group consisting of diisopropylethylamine, sodium hydroxide, and potassium hydroxide, the amount of the base after subtracting the equivalent amount neutralized with an acid addition salt of compound B or a salt thereof being 0.5 to 10 equivalents.

More preferably, the method is for producing the compound represented by formula (A-1) or a salt thereof, comprising reacting 1 equivalent of compound B or a salt thereof with 1.05 to 1.2 equivalents of 3-chloropropionyl chloride in the presence of at least one base selected from the group consisting of diisopropylethylamine, sodium hydroxide, and potassium hydroxide, the amount of the base after subtracting the equivalent amount neutralized with an acid addition salt of compound B or a salt thereof being 0.5 to 10 equivalents.

Particularly preferably, the method is for producing the compound represented by formula (A-1) or a salt thereof, comprising reacting 1 equivalent of compound B or a salt thereof with 1.1 equivalents of 3-chloropropionyl chloride in the presence of sodium hydroxide, the amount of sodium hydroxide after subtracting the equivalent amount neutralized with an acid addition salt of compound B or a salt thereof being 1.1 equivalents.

In the present invention, as another embodiment, a method for producing the compound represented by formula (A-1) or a salt thereof, the method comprising reacting compound B or a salt thereof with the compound represented by formula (I-1-A) in the presence of a base containing a hydroxide ion, may be used.

Preferably, the method is for producing the compound represented by formula (A-1) or a salt thereof, comprising reacting compound B or a salt thereof with the compound represented by formula (I-1-A) wherein $L^1$ and $L^2$ are the same or different, and each is a halogen atom, in the presence of a base containing a hydroxide ion.

More preferably, the method is for producing the compound represented by formula (A-1) or a salt thereof, comprising reacting compound B or a salt thereof with 3-chloropropionyl chloride in the presence of a base containing a hydroxide ion.

More preferably, the method is for producing the compound represented by formula (A-1) or a salt thereof, comprising reacting 1 equivalent of compound B or a salt thereof with 3-chloropropionyl chloride in the presence of a base containing a hydroxide ion, the amount of the base after subtracting the equivalent amount neutralized with an acid addition salt of compound B or a salt thereof being 0.5 to 10 equivalents.

More preferably, the method is for producing the compound represented by formula (A-1) or a salt thereof, comprising reacting 1 equivalent of compound B or a salt thereof with 3-chloropropionyl chloride in the presence of a base containing an alkali metal ion and a hydroxide ion, the amount of the base after subtracting the equivalent amount neutralized with an acid addition salt of compound B or a salt thereof being 0.5 to 10 equivalents.

More preferably, the method is for producing the compound represented by formula (A-1) or a salt thereof, comprising reacting 1 equivalent of compound B or a salt thereof with 3-chloropropionyl chloride in the presence of at least one base selected from the group consisting of sodium hydroxide and potassium hydroxide, the amount of the base after subtracting the equivalent amount neutralized with an acid addition salt of compound B or a salt thereof being 0.5 to 10 equivalents.

More preferably, the method is for producing the compound represented by formula (A-1) or a salt thereof, comprising reacting 1 equivalent of compound B or a salt thereof with 3-chloropropionyl chloride in the presence of at least one base selected from the group consisting of sodium hydroxide and potassium hydroxide, the amount of the base after subtracting the equivalent amount neutralized with an acid addition salt of compound B or a salt thereof being 1.0 to 5 equivalents.

Particularly preferably, the method is for producing the compound represented by formula (A-1) or a salt thereof,

comprising reacting 1 equivalent of compound B or a salt thereof with 1.8 equivalents of 3-chloropropionyl chloride in the presence of at least one base selected from the group consisting of sodium hydroxide and potassium hydroxide, the amount of the base after subtracting the equivalent amount neutralized with an acid addition salt of compound B or a salt thereof being 3.5 equivalents.

Compound A produced according to the above methods may contain various impurities. In particular, when compound A is produced from compound B using 3-chloropropionyl chloride, compound A may contain the following related substances.

Table 1

| Related substance | Compound Name | Structural Formula |
|---|---|---|
| 1 | (S)-N-(1-(1-acryloylpyrrolidin-3-yl)-3-((3,5-dimethoxyphenyl) ethynyl)-1H-pyrazolo[3,4-d]pyrimidin-4-yl)acrylamide (diamide compound) | 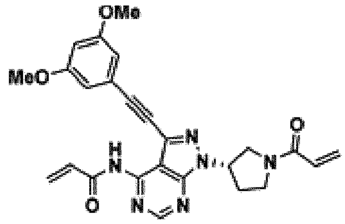 |
| 2 | (S)-1-(3-(4-amino-3-((3,5-dimethoxyphenyl)ethynyl)-1H-pyrazolo[3,4-d]pyrimidin-1-yl)pyrrolidin-1-yl)-3-chloropropan-1-one (A-1-3CP compound) | 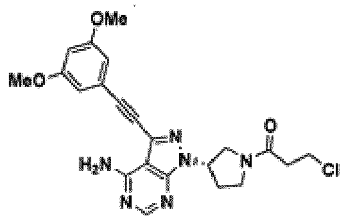 |
| 3 | (S)-3-((3,5-dimethoxyphenyl)ethynyl)-1-(pyrrolidin-3-yl)-1H-pyrazolo[3,4-d]pyrimidin-4-amine (compound B) | 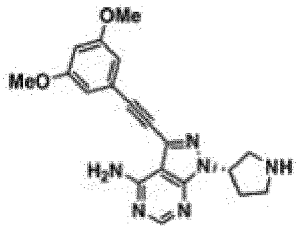 |
| 4 | (S)-3-((1-(1-acryloylpyrrolidin-3-yl)-6-((3,5-dimethoxyphenyl) ethynyl)-1H-pyrazolo[3,4-d]pyrimidin-4-yl)amino)propanoic acid (CE compound) | 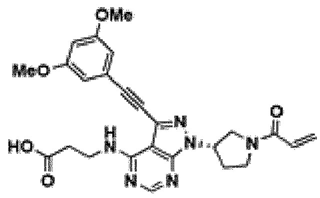 |

Table 2

| | | |
|---|---|---|
| 5 | (S)-1-(3-(4-amino-3-((3,5-dimethoxyphenyl)ethynyl)-1H-pyrazolo [3,4-d]pyrimidin-1-yl)pyrrolidin-1-yl)-3-hydroxypropan-1-one (OH compound) | 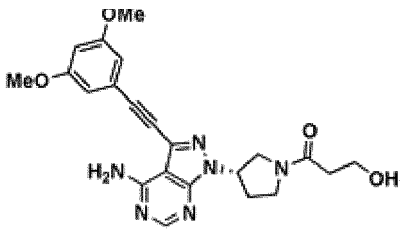 |

(continued)

| | | |
|---|---|---|
| 6 | (S)-8-(1-acryloylpyrrolidin-3-yl)-10- ((3,5-dimethoxyphenyl) ethynyl)-3,4-dihydropyrazolo[4,3-e]pyrimido[1,2-c]pyrimidin-2 (8H)-one (CDA1 compound) | 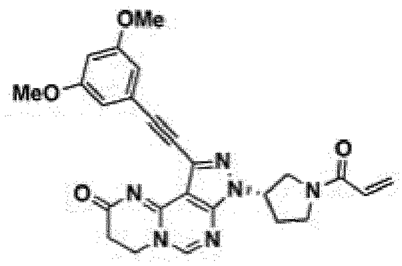 |
| 7 | (S)-3-(4-amino-3-((3,5-dimethoxyphenyl)ethynyl)-1H-pyrazolo[3,4-d]pyrimidin-1-yl)pyrrolidine-1-carbaldehyde (CHO compound) | 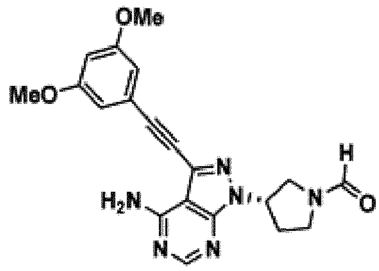 |

Table 3

| | | |
|---|---|---|
| 8 | 1,3-bis((S)-3-(4-amino-3-((3,5-dimethoxyphenyl)ethynyl)-1H-pyrazolo [3,4-d]pyrimidin-1-yl)pyrrolidin-1-yl)propan-1-one (MA compound) | 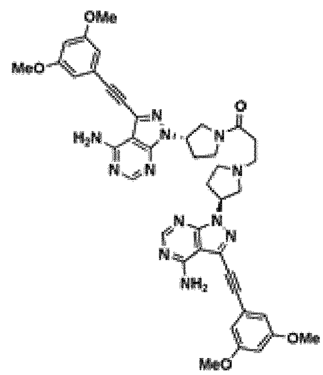 |
| 9 | 1-((S)-3-(4-((3-((S)-3-(4-amino-3-((3,5-dimethoxyphenyl)ethynyl)-1H-pyrazolo[3,4-d]pyrimidin-1-yl)pyrrolidin-1-yl)-3-oxopropyl)amino)-3-((3,5-dimethoxyphenyl)ethynyl)-1H-pyrazolo[3,4-d]pyrimidin-1-yl)pyrrolidin-1-yl)prop-2-en-1-one (Dimer compound) | 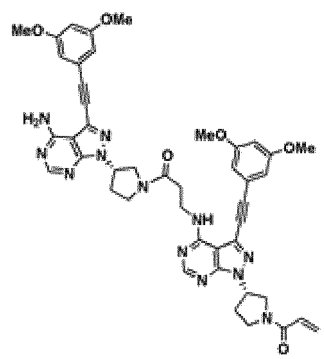 |
| 10 | (S)-1-(1-acryloylpyrrolidin-3-yl)-5-amino-3-((3,5-dimethoxyphenyl) ethynyl)-1H-pyrazole-4-carbonitrile (UK compound) | 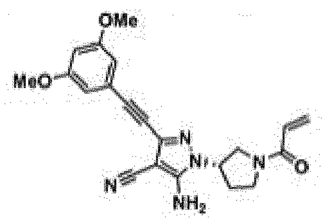 |

Related substance 1 is the diamide compound described above.

Related substance 2 is the A-1-3CP compound described above.

Related substance 3 is compound B described above.

Related substance 4 is (S)-3-((1-(1-acryloylpyrrolidin-3-yl)-6-((3,5-dimethoxyphenyl)ethynyl)-1H-pyrazolo[3,4-d]pyrimidin-4-yl)amino)propanoic acid (which hereinafter may be referred to as "CE compound").

Related substance 5 is (S)-1-(3-(4-amino-3-((3,5-dimethoxyphenyl)ethynyl)-1H-pyrazolo[3,4-d]pyrimidin-1-yl)pyrrolidin-1-yl)-3-hydroxypropan-1-one (which hereinafter may be referred to as "OH compound"). This is a compound in which the chlorine of the A-1-3CP compound is replaced by hydroxyl.

Related substance 6 is (S)-8-(1-acryloylpyrrolidin-3-yl)-10-((3,5-dimethoxyphenyl)ethynyl)-3,4-dihydropyrazolo[4,3-e]pyrimido[1,2-c]pyrimidin-2(8H)-one (which hereinafter may be referred to as "CDA1 compound"). This is a compound in which the acrylamide at the 4-position and the nitrogen at the 5-position of pyrazolo[3,4-d]pyrimidine in related substance 1 form a ring.

Related substance 7 is (S)-3-(4-amino-3-((3,5-dimethoxyphenyl)ethynyl)-1H-pyrazolo[3,4-d]pyrimidin-1-yl)pyrrolidine-1-carbaldehyde (which hereinafter may be referred to as "CHO compound"). This is a compound in which the acryloyl of compound A is replaced by formyl.

Related substance 8 is 1,3-bis((S)-3-(4-amino-3-((3,5-dimethoxyphenyl)ethynyl)-1H-pyrazolo[3,4-d]pyrimidin-1-yl)pyrrolidin-1-yl)propan-1-one (which hereinafter may be referred to as "MA compound"). This is a compound in which the pyrrolidine portion of compound B is bonded to the acryloyl of compound A.

Related substance 9 is 1-((S)-3-(4-((3-((S)-3-(4-amino-3-((3,5-dimethoxyphenyl)ethynyl)-1H-pyrazolo[3,4-d]pyrimidin-1-yl)pyrrolidin-1-yl)-3-oxopropyl)amino)-3-((3,5-dimethoxyphenyl)ethynyl)-1H-pyrazolo[3,4-d]pyrimidin-1-yl)pyrrolidin-1-yl)prop-2-en-1-one (which hereinafter may be referred to as "Dimer compound"). This is a compound in which the nitrogen of the amino group of compound B is bonded to the acryloyl of compound A.

Related substance 10 is (S)-1-(1-acryloylpyrrolidin-3-yl)-5-amino-3-((3,5-dimethoxyphenyl)ethynyl)-1H-pyrazole-4-carbonitrile (which hereinafter may be referred to as "UK compound"). This is a compound in which the pyrimidine portion of compound A is ring-opened.

The present invention also encompasses salts and the like of the related substances. The related substances or salts thereof may be solvates (e.g., hydrates) or non-solvates. In the present invention, any of such forms are included within the scope of "compounds or salts thereof." The salts of the compounds are not particularly limited, and examples include addition salts with inorganic acids such as hydrochloric acid, hydrobromic acid, hydriodic acid, hydrofluoric acid, and sulfuric acid; addition salts with alkyl sulfuric acids such as methanesulfonic acid, p-toluenesulfonic acid, and benzenesulfonic acid; addition salts with organic acids such as acetic acid, citric acid, tartaric acid, and maleic acid; salts with alkali metals such as potassium and sodium; salts with alkaline earth metals such as calcium and magnesium; salts with organic bases, such as ammonium salts, ethylamine salts, and arginine salts; and the like. In the present specification, the terms "related substance 1," "related substance 2," ... "related substance 10" may be intended to include "salts" and "solvates" of the related substances.

The present invention also encompasses a combination of one or more of the related substances described above and/or one or more of salts of the related substances. The combination comprises two or more of the related substances and salts thereof. The combination encompasses all of the following cases: a case in which the combination are composed of two or more of the related substances, a case in which the combination is composed of one or more of the related substances and one or more of salts of the related substances, and a case in which the combination is composed of two or more of salts of the related substances. The combination may comprise any one of the related substances and a salt of the related substance.

In the present invention, the standard refers to a standard used in quantitative or qualitative evaluation of a pharmaceutical preparation and/or drug substance of a pharmaceutical product. The related substances or salts thereof in the present invention may be used as a standard for controlling quality by examining the retention time etc. of each related substance in HPLC measurement, in a stability test of a pharmaceutical preparation and/or drug substance containing compound A, process inspection of its production process, or the like.

Examples of related substances that can be used for quality control include compounds that are starting materials and/or intermediates, compounds that are by-products in the production of a drug substance, compounds that are formed when a pharmaceutical preparation is produced from a drug substance, and the like. However, the related substances that can be used for quality control are not particularly limited as long as they are compounds that can be contained in a drug substance and/or pharmaceutical preparation actually produced. In the production of a drug substance, such related substances include not only compounds actually contained in the drug substance, but also compounds removed in a purification process of the drug substance.

Thus, in the present invention, the related substances described above may be used for the quality control of a drug substance of compound A and a pharmaceutical composition (e.g., a pharmaceutical preparation) containing compound A or a salt thereof (e.g., for the management (adjustment) of the amount of the related substances). Further, according to the present invention, the related substances may also be used as a standard in the detection of impurities

in a sample containing compound A. Moreover, the present invention can provide a method for producing the related substances, the method comprising isolating the related substances from a sample containing compound A. The present invention can also provide a method for analysis of compound A. In the present invention, analysis of compound A means analyzing not only whether a sample containing compound A contains compound A, but also whether the sample contains related substances of compound A, and if an related substance is detected, then measuring the content thereof.

The HPLC system used in the present invention may be generally a commercially available one. The HPLC system comprises at least a separation column and a detector.

In the present invention, the peak resolution Rs as measured by HPLC indicates the relationship between the retention times of peaks and the widths of the peaks in a chromatogram, and is calculated using the following equation.

$$Rs = 1.18 \times (tR2 - tR1) / (W0.5h1 + W0.5h2)$$

tR1 and tR2: retention times of two substances used in resolution measurement, provided that tR1<tR2
W0.5h1 and W0.5h2: peak widths at half height of peaks

It should be noted that the same unit is used for tR1, tR2, W0.5h1, and W0.5h2.

The Japanese Pharmacopoeia states that when the resolution is 1.5 or more, the peaks are completely separated. In the present invention as well, a resolution of 1.5 or more may be used as an index.

In the present invention, the measurement conditions used for HPLC are not particularly limited as long as the resolution between one of related substances 1 to 10 and compound A is 1.5 or more. Preferably, the resolutions between related substance 8 (MA compound), related substance 9 (Dimer compound), and compound A are 1.5 or more. More preferably, the resolutions between related substance 8 (MA compound), related substance 9 (Dimer compound), related substance 4 (CE compound), related substance 5 (OH compound), related substance 7 (CHO compound), and compound A are 1.5 or more. Even more preferably, the resolutions between related substance 3 (compound B), related substance 4 (CE compound), related substance 5 (OH compound), related substance 6 (CDA1 compound), related substance 7 (CHO compound), related substance 2 (A-1-3CP compound), related substance 8 (MA compound), related substance 9 (Dimer compound), and compound A are 1.5 or higher.

In the present invention, the measurement conditions used for HPLC, such as gradient of a mobile phase, a silica gel column, the Injection Volume of a measurement sample, the presence or absence of a mobile phase cleaner, the measurement wavelength, the column temperature, the mobile phase flow rate, and the mixer volume of a HPLC system, may be suitably set.

Known columns for HPLC include normal-phase columns, in which an organic phase is used as a mobile phase to separate compounds according to their polarity, and reversed-phase columns, in which an aqueous phase is used as a mobile phase to separate compounds. In the present invention, reversed-phase chromatography, which uses a reversed-phase column, is preferable in terms of the properties of compound A.

In general, in HPLC measurement, the time at which a measurement sample is injected (introduced) into the mobile phase and detection in the detector starts is defined as the starting time point of the measurement. Below, in the present invention, time points during HPLC measurement may be defined with reference to the starting time point of the measurement.

The HPLC separation column that can be used in the present invention is selected from silica gel columns, columns containing silica gel whose surface is modified with octadecylsilyl groups (ODS columns or C18 columns), columns containing silica gel whose surface is modified with octyl groups (C8 columns), columns containing silica gel whose surface is modified with cyanopropyl groups (CN columns), columns containing silica gel whose surface is modified with phenethyl groups (Ph columns), columns containing silica gel whose surface is modified with aminopropyl groups (NH columns), columns containing silica gel whose surface is modified with dihydroxypropyl groups (Diol columns), columns packed with various polymers (polymer columns), columns packed with ion-exchange resin (ion-exchange columns), and the like. In the present invention, ODS columns are preferable.

It is possible to use various types of ODS columns with different silica gel particle sizes, different pore sizes, different types of bonding of octadecylsilyl groups, different degrees of substitution of octadecylsilyl groups, etc. In the present invention, a high-purity silica gel is used, and it is preferable to use an ODS column (an end-capped ODS column) in which residual silanol obtained after octadecylation is treated with a low-molecular-weight silylating agent.

It is possible to use various types of ODS columns with different silica gel particle sizes, different pore sizes, different types of bonding of octadecylsilyl groups, different degrees of substitution of octadecylsilyl groups, etc. The silica gel preferably has an average particle size of, for example, 3 μm. The average particle size of silica gel can be measured by, for example, laser diffractometry.

The silica gel preferably has an average pore size of, for example, 10 to 12 nm. The average pore size of silica

gel can be measured by, for example, a gas adsorption method.

The bonding type of octadecylsilyl groups in the silica gel is preferably, for example, monomeric or polymeric.

The degree of substitution of octadecylsilyl groups can be measured by various methods. The carbon content in the silica gel is preferably, for example, 14% or more.

The carbon content in the silica gel is preferably, for example, 20% or less. The carbon content in the silica gel can be measured by various methods.

In the present invention, a mixture of an organic phase and an aqueous phase is used as the mobile phase for HPLC.

The organic phase used in the mobile phase for HPLC is a liquid medium containing mainly an organic solvent. Examples of organic solvents include non-polar solvents, such as hexane, cyclohexane, heptane, diethyl ether, tetrahydrofuran, chloroform, and methylene chloride; aprotic polar solvents, such as acetone, dimethyl sulfoxide, and acetonitrile; acetic acid; methanol; ethanol; isopropanol; acetonitrile; and the like. These organic solvents may be used singly or in a combination of two or more (e.g., a mixture of solvents). The organic solvent contained in the organic phase in the present invention is preferably methanol or acetonitrile, and more preferably acetonitrile.

The organic phase may contain water in an amount of 20 volume% or less. The amount of water is preferably 10 volume% or less, more preferably 5 volume% or less, and particularly preferably 1 volume% or less, of the entire organic phase.

The aqueous phase used in the mobile phase for HPLC is a liquid medium containing mainly water. All the liquid contained in the aqueous phase may be water. The aqueous phase may contain an organic solvent in an amount of 50 volume% or less. The organic solvent used in this case is not particularly limited as long as it can be uniformly mixed with water. Examples of organic solvents include acetone, dimethyl sulfoxide, acetonitrile, formic acid, acetic acid, methanol, ethanol, isopropanol, and the like. The organic solvent is preferably acetonitrile or methanol, and more preferably acetonitrile. The amount of organic solvent contained in the aqueous phase is 50 volume% or less of the entire aqueous phase. The amount of organic solvent is preferably 30 volume% or less, and more preferably 5 to 30 volume%, of the entire aqueous phase.

The pH in the mobile phase for HPLC is not particularly limited as long as the related substances described above can be detected and their content can be calculated. The pH in the mobile phase for HPLC is preferably a pH excluding 6.9 to 7.1. The pH in the mobile phase for HPLC is more preferably 6.1 to 6.8 and 7.2 to 7.5, even more preferably 6.4 to 6.8, and particularly preferably 6.6. The pH in the mobile phase for HPLC can be adjusted by adding a buffer described below.

A buffer may be added to the mobile phase for HPLC in order to reduce the effect of the pH on the measurement and improve reproducibility. For example, it is possible to add as a buffer acetic acid or a salt thereof, citric acid or a salt thereof, tartaric acid or a salt thereof, and phosphoric acid or a salt thereof. Examples of acetic acid or a salt thereof include acetic acid and sodium acetate. Examples of citric acid or a salt thereof include citric acid, monosodium citrate, disodium citrate, and trisodium citrate. Examples of tartaric acid or a salt thereof include tartaric acid and sodium tartrate. Examples of phosphoric acid or a salt thereof include phosphoric acid, sodium dihydrogenphosphate, disodium hydrogenphosphate, potassium dihydrogenphosphate, and dipotassium hydrogenphosphate. As buffers in the present invention, phosphoric acid and phosphoric acid salts are preferable, sodium dihydrogenphosphate, disodium hydrogenphosphate, potassium dihydrogenphosphate, and dipotassium hydrogenphosphate are more preferable, and potassium dihydrogenphosphate is particularly preferable, from the viewpoint of the properties of the substances to be measured and the shape of the peaks obtained by the measurement, as well as the measurement reproducibility. These buffers may be used singly or in a combination of two or more.

The buffer may be added to the aqueous phase and the organic phase. Preferably, the buffer is added to the aqueous phase.

The concentration of the buffer that can be used in the present invention may be suitably adjusted within a concentration range in which the buffer does not undergo precipitation during HPLC measurement. The concentration of the buffer is preferably 5 to 10 mM.

In general, in HPLC measurement, target related substances can be appropriately separated by varying the composition of the mixture of the organic phase and the aqueous phase in the mobile phase. In doing so, the related substances described above can be measured by keeping the proportion of each component of the mixture in the mobile phase constant (isocratic) or varying the composition continuously (gradient).

In reversed-phase chromatography, the variation in the composition of the mixture of the organic phase and the aqueous phase in the mobile phase is usually plotted on a two-dimensional graph in which the vertical axis shows the percentage (%) of the organic phase in the entire mobile phase, and the horizontal axis shows the measurement time (min). That is, when the composition of the mobile phase is under isocratic conditions, the graph is represented by a linear function with a slope of 0; when the composition of the mobile phase is under gradient conditions in which the percentage of the organic phase is increased over time at a constant increase rate, the graph is represented by a linear function with a positive slope; and when the composition of the mobile phase is under gradient conditions in which the

percentage of the organic phase is decreased over time at a constant decrease rate, the graph is represented by a linear function with a negative slope. By combining these, related substances with low polarity can also be eluted efficiently and measured with short retention times while maintaining the resolution of peaks. The increase rate of the organic phase in the mobile phase, which is the slope of the above linear functions, can be expressed, for example, as the increase rate of the organic phase per unit time, and can be calculated as follows.

Increase rate of organic phase per unit time = ((percentage of organic phase in mobile phase at ending time point of gradient)−(percentage of organic phase in mobile phase at starting time point of gradient))÷(length of time between starting time point and ending time point of gradient)

For example, when the percentage of the organic phase at the starting time point of the measurement (0 minutes) is 10 volume% of the mobile phase, and the percentage of the organic phase is continuously increased and reaches 30 volume% of the mobile phase 10 minutes after the start of the measurement, the increase rate of the organic phase per unit time is expressed as 2 volume%/minute.

In the present invention, the composition of the mobile phase and the presence or absence of the change in the mobile phase are not particularly limited as long as the related substances described above can be measured. A preferred embodiment of the present invention includes three gradients in which the percentage of the organic phase in the mobile phase is increased over time. The three gradients may be referred to below as a "first gradient," a "second gradient," and a "third gradient," in order starting from the one closest to the starting time point of the measurement.

The first gradient, second gradient, and third gradient in one preferred embodiment of the present invention are described below.

The increase rate of the organic phase in the first gradient is 0.7 to 2.0 volume%/minute, preferably 0.7 to 1.5 volume%/minute, more preferably 0.7 to 1.3 volume%/minute, and even more preferably 1.0 volume%/minute.

The starting time point of the first gradient is 0 to 5 minutes, preferably 0 to 2 minutes, and more preferably 0 to 1 minute, after the starting time point of the measurement. The starting time point of the first gradient is even more preferably the starting time point of the measurement (0 minutes after the starting time point of the measurement).

The ending time point of the first gradient is 5 to 15 minutes, preferably 7 to 12 minutes, and more preferably 10 minutes, after the starting time point of the first gradient.

The percentage of the organic phase at the starting time point of the first gradient is 0 to 15 mass%, preferably 0 to 10 mass%, more preferably 0 to 5 mass%, and particularly preferably 0 mass%, of the entire mobile phase.

The percentage of the organic phase at the ending time point of the first gradient is 5 to 15 mass%, preferably 7 to 12 mass%, and particularly preferably 10 mass%, of the entire mobile phase.

The increase rate of the organic phase in the second gradient is 0.3 to 2.0 volume%/minute, preferably 0.3 to 1.8 volume%/minute, more preferably 0.7 to 1.8 volume%/minute, and even more preferably 1.3 to 1.4 volume%/minute. In another embodiment, the increase rate of the organic phase in the second gradient is preferably 0.5 volume%/minute.

The starting time point of the second gradient is 0 to 10 minutes, and preferably 0 to 5 minutes, after the ending time point of the first gradient. The starting time point of the second gradient is more preferably the ending time point of the first gradient (0 minutes after the ending time point of the first gradient).

The ending time point of the second gradient is 10 to 20 minutes, preferably 12 to 18 minutes, and more preferably 15 minutes, after the starting time point of the second gradient. In another embodiment, the ending time point of the second gradient is preferably 20 minutes after the starting time point of the second gradient.

The percentage of the organic phase at the starting time point of the second gradient is the same as the percentage of the organic phase at the ending time point of the first gradient.

The percentage of the organic phase at the ending time point of the second gradient is 15 to 45 mass%, preferably 20 to 40 mass%, more preferably 28 to 38 mass%, and particularly preferably 30 mass%, of the entire mobile phase. In another embodiment, the percentage of the organic phase at the ending time point of the second gradient is preferably 20 mass%.

The increase rate of the organic phase in the third gradient is 1.0 to 6.0 volume%/minute, preferably 1.0 to 2.0 volume%/minute, more preferably 1.0 to 1.5 volume%/minute, and even more preferably 1.3 to 1.4 volume%/minute. In another embodiment, the increase rate of the organic phase in the third gradient is preferably 5.0 volume%/minute.

The starting time point of the third gradient is 0 to 10 minutes, and preferably 0 to 5 minutes, after the ending time point of the second gradient. The starting time point of the third gradient is more preferably the ending time point

of the second gradient (0 minutes after the ending time point of the second gradient).

The ending time point of the third gradient is 10 to 20 minutes, preferably 13 to 18 minutes, and more preferably 15 minutes, after the starting time point of the third gradient. In another embodiment, the ending time point of the third gradient is preferably 10 minutes after the starting time point of the third gradient.

The percentage of the organic phase at the starting time point of the third gradient is the same as the percentage of the organic phase at the ending time point of the second gradient.

The percentage of the organic phase at the ending time point of the third gradient is 45 to 75 mass%, preferably 45 to 70 mass%, more preferably 45 to 55 mass%, and particularly preferably 50 mass%, of the entire mobile phase. In another embodiment, the percentage of the organic phase at the ending time point of the third gradient is preferably 70 mass%.

The starting time point of the measurement may or may not coincide with the starting time point of the first gradient. When the starting time point of the measurement does not coincide with the starting time point of the first gradient, the mobile phase may be isocratic during the time between the starting time point of the measurement and the starting time point of the first gradient.

When a period of time in which the mobile phase is isocratic is provided between the ending time point of the first gradient and the starting time point of the second gradient, the length of the period is greater than 0 minutes and 5 minutes or less, preferably greater than 0 minutes and 2 minutes or less, and more preferably greater than 0 minutes and 1 minute or less.

The ending time point of the first gradient may or may not coincide with the starting time point of the second gradient. When the ending time point of the first gradient does not coincide with the starting time point of the second gradient, the mobile phase may be isocratic during the time between the ending time point of the first gradient and the starting time point of the second gradient.

When a period of time in which the mobile phase is isocratic is provided between the ending time point of the first gradient and the starting time point of the second gradient, the length of the period is greater than 0 minutes and 10 minutes or less, and preferably greater than 0 minutes and 5 minutes or less.

The ending time point of the second gradient may or may not coincide with the starting time point of the third gradient. When the ending time point of the second gradient does not coincide with the starting time point of the third gradient, the mobile phase may be isocratic during the time between the ending time point of the second gradient and the starting time point of the third gradient.

When a period of time in which the mobile phase is isocratic is provided between the ending time point of the second gradient and the starting time point of the third gradient, the length of the period is greater than 0 minutes and 10 minutes or less, and preferably greater than 0 minutes and 5 minutes or less.

The starting time point of the measurement and the starting time point of the first gradient may coincide, the ending time point of the first gradient and the starting time point of the second gradient may coincide, and the ending time point of the second gradient and the starting time point of the third gradient may coincide.

In such an embodiment, the first gradient, the second gradient, and the third gradient may be as follows.

The ending time point of the first gradient is 5 to 15 minutes, preferably 7 to 12 minutes, and more preferably 10 minutes, after the starting time point of the measurement.

The ending time point of the second gradient is 20 to 35 minutes, preferably 22 to 28 minutes, and more preferably 25 minutes, after the starting time point of the measurement. In another embodiment, the ending time point of the second gradient is preferably 30 minutes after the starting time point of the measurement.

The ending time point of the third gradient is 35 to 50 minutes, preferably 38 to 48 minutes, and more preferably 40 minutes, after the starting time point of the measurement.

Even in such a case, it is not precluded to provide a period of time in which the mobile phase is isocratic, during at least one time selected from the group consisting of time between the starting time point of the measurement and the starting time point of the first gradient, time between the ending time point of the first gradient and the starting time point of the second gradient, and time between the ending time point of the second gradient and the starting time point of the third gradient.

In the present invention, the increase rates of the organic phase in the first gradient, the second gradient, and the third gradient may be all the same, partially the same, or different from each other. In one preferred embodiment of the present invention, the increase rate of the organic phase in the second gradient is the same as that in the third gradient. Two or three gradients with the same increase rate of the organic phase means that the gradients together form one gradient if a period of time in which the mobile phase is isocratic is not provided between them.

A preferred embodiment of the gradients for HPLC measurement in the present invention includes first to third gradients as described above. Preferably, the increase rate of the organic phase in the first gradient is 0.7 to 2.0 volume%/minute, the starting time point of the first gradient is 0 to 5 minutes after the starting time point of the measurement, the ending time point of the first gradient is 5 to 15 minutes after the starting time point of the first gradient, the percentage of the organic phase at the starting time point of the first gradient is 0 to 15 mass% of the entire mobile

phase, the percentage of the organic phase at the ending time point of the first gradient is 5 to 15 mass% of the entire mobile phase, the increase rate of the organic phase in the second gradient is 0.3 to 2.0 volume%/minute, the starting time point of the second gradient is 0 to 10 minutes after the ending time point of the first gradient, the ending time point of the second gradient is 10 to 20 minutes after the starting time point of the second gradient, the percentage of the organic phase at starting time point of the second gradient is the same as the percentage of the organic phase at the ending time point of the first gradient, the percentage of the organic phase at the ending time point of the second gradient is 15 to 45 mass% of the entire mobile phase, the increase rate of the organic phase in the third gradient is 1.0 to 6.0 volume%/minute, the starting time point of the third gradient is 0 to 10 minutes after the ending time point of the second gradient, the ending time point of the third gradient is 10 to 20 minutes after the starting time point of the third gradient, the percentage of the organic phase at the starting time point of the third gradient is the same as the percentage of the organic phase at the ending time point of the second gradient, and the percentage of the organic phase at the ending time point of the third gradient is 45 to 75 mass% of the entire mobile phase.

More preferably, the increase rate of the organic phase in the first gradient is 0.7 to 1.5 volume%/minute, the starting time point of the first gradient is 0 to 2 minutes after the starting time point of the measurement, the ending time point of the first gradient is 7 to 12 minutes after the starting time point of the first gradient, the percentage of the organic phase at the starting time point of the first gradient is 0 to 10 mass% of the entire mobile phase, the percentage of the organic phase at the ending time point of the first gradient is 7 to 12 mass% of the entire mobile phase, the increase rate of the organic phase in the second gradient is 0.3 to 2.0 volume%/minute, the starting time point of the second gradient is 0 to 10 minutes after the ending time point of the first gradient, the ending time point of the second gradient is 10 to 20 minutes after the starting time point of the second gradient, the percentage of the organic phase at the starting time point of the second gradient is the same as the percentage of the organic phase at the ending time point of the first gradient, the percentage of the organic phase at the ending time point of the second gradient is 15 to 45 mass% of the entire mobile phase, the increase rate of the organic phase in the third gradient is 1.0 to 6.0 volume%/minute, the starting time point of the third gradient is 0 to 10 minutes after the ending time point of the second gradient, the ending time point of the third gradient is 10 to 20 minutes after the starting time point of the third gradient, the percentage of the organic phase at the starting time point of the third gradient is the same as the percentage of the organic phase at the ending time point of the second gradient, and the percentage of the organic phase at the ending time point of the third gradient is 45 to 75 mass% of the entire mobile phase.

More preferably, the increase rate of the organic phase in the first gradient is 0.7 to 1.3 volume%/minute, the starting time point of the first gradient is 0 to 1 minute after the starting time point of the measurement, the ending time point of the first gradient is 10 minutes after the starting time point of the first gradient, the percentage of the organic phase at the starting time point of the first gradient is 0 to 5 mass% of the entire mobile phase, the percentage of the organic phase at the ending time point of the first gradient is 10 mass% of the entire mobile phase, the increase rate of the organic phase in the second gradient is 0.3 to 2.0 volume%/minute, the starting time point of the second gradient is 0 to 10 minutes after the ending time point of the first gradient, the ending time point of the second gradient is 10 to 20 minutes after the starting time point of the second gradient, the percentage of the organic phase at the starting time point of the second gradient is the same as the percentage of the organic phase at the ending time point of the first gradient, the percentage of the organic phase at the ending time point of the second gradient is 15 to 45 mass% of the entire mobile phase, the increase rate of the organic phase in the third gradient is 1.0 to 6.0 volume%/minute, the starting time point of the third gradient is 0 to 10 minutes after the ending time point of the second gradient, the ending time point of the third gradient is 10 to 20 minutes after the starting time point of the third gradient, the percentage of the organic phase at the starting time point of the third gradient is the same as the percentage of the organic phase at the ending time point of the second gradient, and the percentage of the organic phase at the ending time point of the third gradient is 45 to 75 mass% of the entire mobile phase.

More preferably, the increase rate of the organic phase in the first gradient is 1.0 volume%/minute, the starting time point of the first gradient is the starting time point of the measurement (0 minutes after the starting time point of the measurement), the ending time point of the first gradient is 10 minutes after the starting time point of the measurement, the percentage of the organic phase at the starting time point of the first gradient is 0 mass%, the percentage of the organic phase at the ending time point of the first gradient is 10 mass%, the increase rate of the organic phase in the second gradient is 0.3 to 1.8 volume%/minute, the starting time point of the second gradient is 0 to 5 minutes after the ending time point of the first gradient, the ending time point of the second gradient is 12 to 18 minutes after the starting time point of the second gradient, the percentage of the organic phase at the starting time point of the second gradient is the same as the percentage of the organic phase at the ending time point of the first gradient, the percentage of the organic phase at the ending time point of the second gradient is 20 to 40 mass% of the entire mobile phase, the increase rate of the organic phase in the third gradient is 1.0 to 2.0 volume%/minute, the starting time point of the third gradient is 0 to 5 minutes after the ending time point of the second gradient, the ending time point of the third gradient is 13 to 18 minutes after the starting time point of the third gradient, the percentage of the organic phase at the starting time point of the third gradient is the same as the percentage of the organic phase at the ending time point of the second

gradient, and the percentage of the organic phase at the ending time point of the third gradient is 45 to 70 mass% of the entire mobile phase.

In another embodiment, more preferably, the increase rate of the organic phase in the first gradient is 0.7 to 1.5 volume%/minute, the starting time point of the first gradient is 0 to 2 minutes after the starting time point of the measurement, the ending time point of the first gradient is 7 to 12 minutes after the starting time point of the first gradient, the percentage of the organic phase at the starting time point of the first gradient is 0 to 10 mass% of the entire mobile phase, the percentage of the organic phase at the ending time point of the first gradient is 7 to 12 mass% of the entire mobile phase, the increase rate of the organic phase in the second gradient is 0.3 to 1.8 volume%/minute, the starting time point of the second gradient is 0 to 5 minutes after the ending time point of the first gradient, the ending time point of the second gradient is 12 to 18 minutes after the starting time point of the second gradient, the percentage of the organic phase at the starting time point of the second gradient is the same as the percentage of the organic phase at the ending time point of the first gradient, the percentage of the organic phase at the ending time point of the second gradient is 20 to 40 mass% of the entire mobile phase, the increase rate of the organic phase in the third gradient is 1.0 to 2.0 volume%/minute, the starting time point of the third gradient is 0 to 5 minutes after the ending time point of the second gradient, the ending time point of the third gradient is 13 to 18 minutes after the starting time point of the third gradient, the percentage of the organic phase at the starting time point of the third gradient is the same as the percentage of the organic phase at the ending time point of the second gradient, and the percentage of the organic phase at the ending time point of the third gradient is 45 to 70 mass% of the entire mobile phase.

In another embodiment, more preferably, the increase rate of the organic phase in the first gradient is 0.7 to 1.3 volume%/minute, the starting time point of the first gradient is 0 to 1 minute from the starting time point of the measurement, the ending time point of the first gradient is 10 minutes after the starting time point of the first gradient, the percentage of the organic phase at the starting time point of the first gradient is 0 to 5 mass% of the entire mobile phase, the percentage of the organic phase at the ending time point of the first gradient is 10 mass% of the entire mobile phase, the increase rate of the organic phase in the second gradient is 0.7 to 1.8 volume%/minute, the starting time point of the second gradient is 0 minutes after the ending time point of the first gradient, the ending time point of the second gradient is 15 minutes after the starting time point of the second gradient, the percentage of the organic phase at the starting time point of the second gradient is the same as the percentage of the organic phase at the ending time point of the first gradient, the percentage of the organic phase at the ending time point of the second gradient is 28 to 38 mass% of the entire mobile phase, the increase rate of the organic phase in the third gradient is 1.0 to 1.5 volume%/minute, the starting time point of the third gradient is 0 minutes after the ending time point of the second gradient, the ending time point of the third gradient is 15 minutes after the starting time point of the third gradient, the percentage of the organic phase at the starting time point of the third gradient is the same as the percentage of the organic phase at the ending time point of the second gradient, and the percentage of the organic phase at the ending time point of the third gradient is 45 to 55 mass% of the entire mobile phase.

Particularly preferably, the increase rate of the organic phase in the first gradient is 1.0 volume%/minute, the starting time point of the first gradient is the starting time point of the measurement (0 minutes after the starting time point of the measurement), the ending time point of the first gradient is 10 minutes after the starting time point of the measurement, the percentage of the organic phase at the starting time point of the first gradient is 0 mass%, the percentage of the organic phase at the ending time point of the first gradient is 10 mass%, the increase rate of the organic phase in the second gradient is 1.3 to 1.4 volume%/minute, the starting time point of the second gradient is 0 minutes after the ending time point of the first gradient, the ending time point of the second gradient is 15 minutes after the starting time point of the second gradient, the percentage of the organic phase at the starting time point of the second gradient is the same as the percentage of the organic phase at the ending time point of the first gradient, the percentage of the organic phase at the ending time point of the second gradient is 30 mass%, the increase rate of the organic phase in the third gradient is 1.3 to 1.4 volume%/minute, the starting time point of the third gradient is 0 minutes after the ending time point of the second gradient, the ending time point of the third gradient is 15 minutes after the starting time point of the third gradient, the percentage of the organic phase at the starting time point of the third gradient is the same as the percentage of the organic phase at the ending time point of the second gradient, and the percentage of the organic phase at the ending time point of the third gradient is 50 mass%.

Another preferred embodiment of the gradients for HPLC measurement in the present invention includes first to third gradients as described above. Preferably, the increase rate of the organic phase in the first gradient is 1.0 volume%/minute, the starting time point of the first gradient is 0 minutes after the starting time point of the measurement, the ending time point of the first gradient is 10 minutes after the starting time point of the first gradient, the percentage of the organic phase at the starting time point of the first gradient is 0 mass% of the entire mobile phase, the percentage of the organic phase at the ending time point of the first gradient is 10 mass% of the entire mobile phase, the increase rate of the organic phase in the second gradient is 0.5 volume%/minute, the starting time point of the second gradient is 0 minutes after the ending time point of the first gradient, the ending time point of the second gradient is 20 minutes after the starting time point of the second gradient, the percentage of the organic phase at the starting time point of the

second gradient is the same as the percentage of the organic phase at the ending time point of the first gradient, the percentage of the organic phase at the ending time point of the second gradient is 20 mass% of the entire mobile phase, the increase rate of the organic phase in the third gradient is 5.0 volume%/minute, the starting time point of the third gradient is 0 minutes after the ending time point of the second gradient, the ending time point of the third gradient is 10 minutes after the starting time point of the third gradient, the percentage of the organic phase at the starting time point of the third gradient is the same as the percentage of the organic phase at the ending time point of the second gradient, and the percentage of the organic phase at the ending time point of the third gradient is 70 mass% of the entire mobile phase.

After the end of the third gradient, if necessary, a period of time in which the mobile phase is isocratic may be provided, and then a gradient in which the percentage of the organic phase in the mobile phase is decreased over time may be provided.

The duration of time for the gradient in which the percentage of the organic phase in the mobile phase is decreased over time may be 1 minute or less, preferably 0.5 minutes or less, and more preferably 0.1 minutes or less.

By the gradient in which the percentage of the organic phase in the mobile phase is decreased over time, the percentage of the organic phase may be decreased to 0 to 15 mass%, preferably 0 to 10 mass%, more preferably 0 to 5 mass%, and even more preferably 0 mass% of the entire mobile phase.

A mobile phase cleaner may also be used for HPLC in the present invention as appropriate. The cleaner uses activated carbon in a cartridge and can remove impurities and dust in the mobile phase. As a result, the chromatogram baseline noise is reduced; thus, even a small amount of related substances derived from compound A can be appropriately detected and quantified.

In the present invention, the amount of solution injected into the mobile phase at the starting time point of the HPLC measurement is not particularly limited as long as it is a measurable amount, and is preferably 2 μL or more, more preferably 4 to 22 μL, and particularly preferably 8 to 22 μL.

## Examples

The present invention is described in more detail below with reference to Examples. However, the present invention is not limited to the Examples. Although the present invention is sufficiently described by the Examples, a person skilled in the art will understand that various changes and modifications are available. Thus, such changes and modifications are included in the present invention unless they depart from the spirit and principal concept of the present invention.

The reagents used in the Examples are commercially available products unless indicated otherwise.

The yield in the Production Examples was calculated as below:

the yield (%) = ((the amount of a desired product obtained)/(the theoretical amount of the desired product obtained)) × 100.

The LCMS spectra in the Production Examples were measured by using an ACQUITY SQD (quadrupole, produced by Waters Corporation) under the following conditions.

Column: ACQUITY UPLC® BEH C18, produced by Waters Corporation, 2.1 × 50 mm, 1.7 μm
MS Detection: ESI positive
UV Detection: 254 and 280 nm
Column Flow Rate: 0.5 mL/minute
Mobile Phase: Water/acetonitrile (0.1% formic acid)
Injection Volume: 1 μL

The conditions for performing HPLC in each step of the Production Examples are as described below.

Condition 1

Column: InertSustain C18 (4.6 mm I.D. × 150 mm, 3 μm)
Detector: Ultraviolet absorption photometer (measurement wavelength: 287 nm)
Column Temperature: Constant temperature around 40°C

Flow Rate: 1.0 mL/minute
Injection Volume: 10 μL
Analysis Time: 25 minutes (area measurement range: 15 minutes) Mobile Phase:

Solution A: 10 mmol/L aqueous phosphoric acid solution
Solution B: Acetonitrile

Gradient Program

Table 4

| Time after Injection (min) | Mobile Phase A (vol%) | Mobile Phase B (vol%) |
|---|---|---|
| 0 to 8 | 95 → 20 | 5 → 80 |
| 8 to 15 | 20 | 80 |
| 15 to 15.01 | 20 → 95 | 80 → 5 |
| 15.01 to 25 | 95 | 5 |

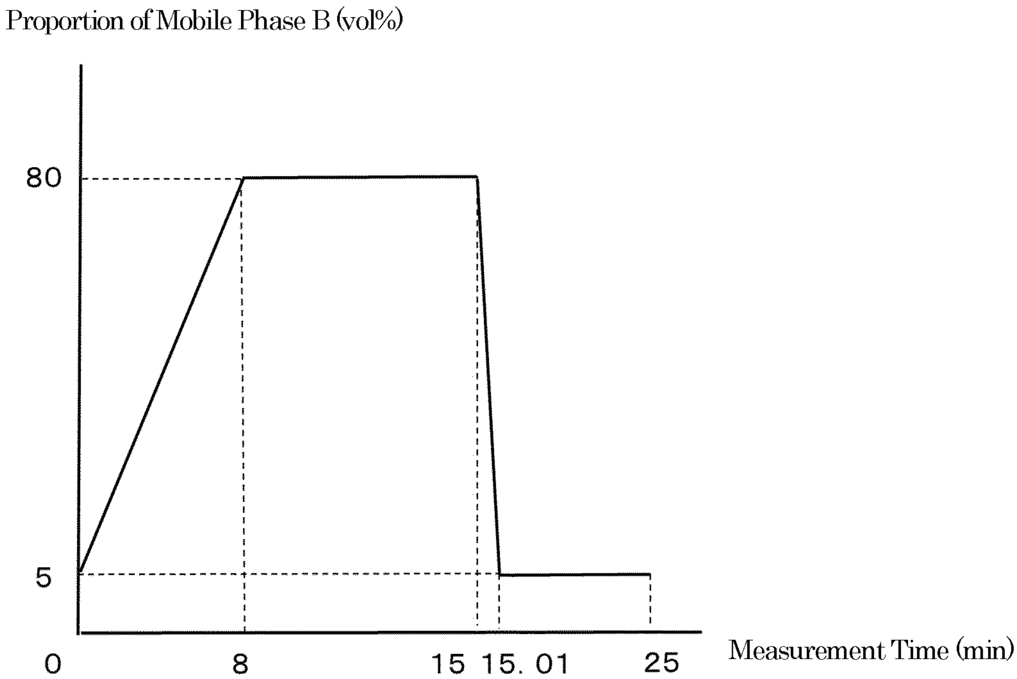


Condition 2

Column: InertSustain C18 (4.6 mm I.D. × 150 mm, 3 μm)
Detector: Ultraviolet absorption photometer (measurement wavelength: 287 nm)
Column Temperature: Constant temperature around 40°C
Flow Rate: 1.0 mL/minute
Injection Volume: 10 μL
Analysis Time: 30 minutes (area measurement range: 15 minutes)
Mobile Phase:

Solution A: 10 mmol/L aqueous phosphoric acid solution
Solution B: Acetonitrile

Gradient Program

Table 5

| Time after Injection (min) | Mobile Phase A (vol%) | Mobile Phase B (vol%) |
|---|---|---|
| 0 to 0.5 | 95 → 68 | 5 → 32 |
| 0.5 to 3.5 | 68 | 32 |
| 3.5 to 8 | 68 → 40 | 32 → 60 |
| 8 to 9 | 40 → 20 | 60 → 80 |
| 9 to 20 | 20 | 80 |
| 20 to 20.01 | 20 → 95 | 80 → 5 |
| 20.01 to 30 | 95 | 5 |

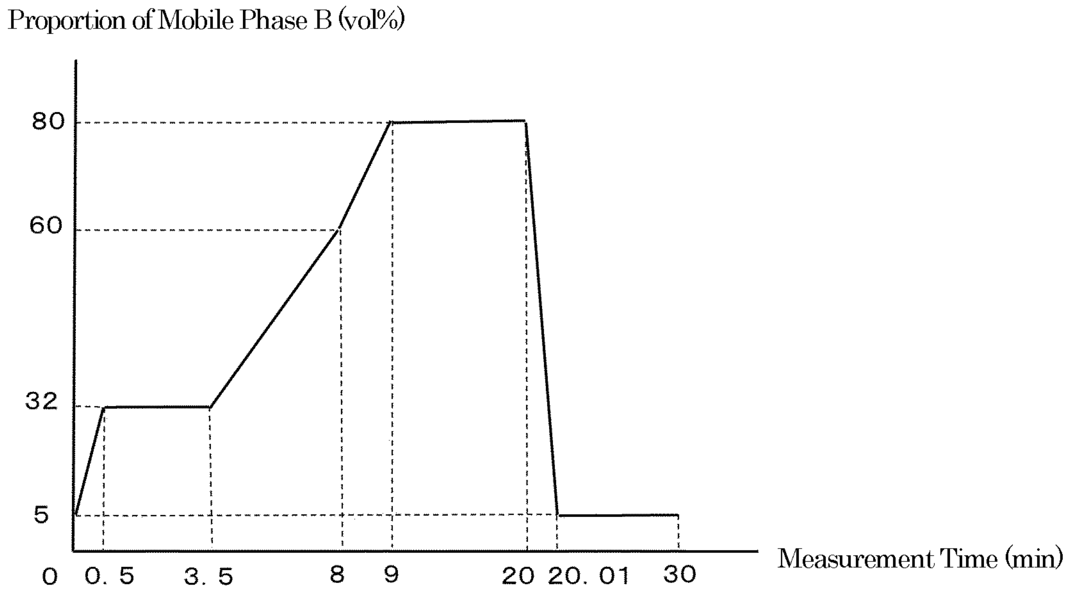


Condition 3

Column: InertSustain C18 (4.6 mm I.D. × 150 mm, 3 μm)
Detector: Ultraviolet absorption photometer (measurement wavelength: 220 nm)
Column Temperature: Constant temperature around 40°C
Flow Rate: 1.0 mL/minute
Injection Volume: 10 μL
Analysis Time: 78 minutes (area measurement range: 58 minutes)
Mobile Phase:

Solution A: 10 mmol/L aqueous phosphoric acid solution
Solution B: Acetonitrile

Gradient Program

Table 6

| Time after Injection (min) | Mobile Phase A (vol%) | Mobile Phase B (vol%) |
|---|---|---|
| 0 to 5 | 95 → 70 | 5 → 30 |

(continued)

| Time after Injection (min) | Mobile Phase A (vol%) | Mobile Phase B (vol%) |
|---|---|---|
| 5 to 40 | 70 | 30 |
| 40 → 53 | 70 → 20 | 30 → 80 |
| 53 to 68 | 20 | 80 |
| 68 to 68. 01 | 20 → 95 | 80 → 5 |
| 68. 01 to 78 | 95 | 5 |

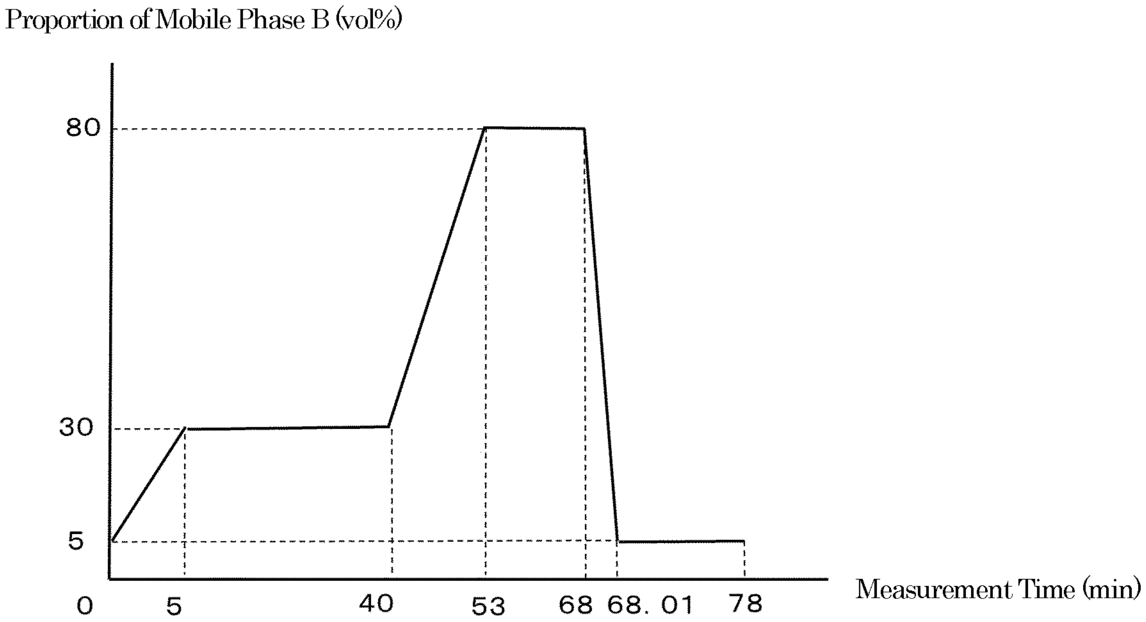


Production Example 1: Synthesis of (S)-3-((3,5-dimethoxyphenyl)ethynyl)-1-(pyrrolidin-3-yl)-1H-pyrazolo[3,4-d]pyrimidine-4-amine·2methanesulfonate

Toluene (2165 g), tert-butyl(S)-3-(4-amino-3-iodo-1H-pyrazolo[3,4-d]pyrimidin-1-yl)pyrrolidine-1-carboxylate (500 g), 1-ethynyl-3,5-dimethoxybenzene (207.5 g, PTL 1), and triethylamine (176.35 g) were dissolved, and the inside of the reaction system was purged with nitrogen. Copper iodide (885 mg), bis(triphenylphosphine palladium dichloride (3.264 g), and triphenylphosphine (1.2195 g) were added to the dissolved mixture, and the mixture was stirred at an internal temperature of 75°C for 18 hours in a nitrogen atmosphere. Thereafter, the reaction solution was partially taken out and measured by HPLC (condition 1). The peak area of tert-butyl(S)-3-(4-amino-3-iodo-1H-pyrazolo[3,4-d]pyrimidin-1-yl)pyrrolidine-1-carboxylate was confirmed to be 1.0% or less of the total peak area.

The internal temperature was cooled to 50°C, and ethyl acetate (2255 g), Scavenger SH Silica (250 g), and refined Shirasagi activated carbon (50 g) were added to the mixture, followed by stirring for 21 hours. The Scavenger SH Silica and refined Shirasagi activated carbon were removed from the mixture by suction filtration with a Nutsche filter. The residue was washed with 4 L of ethyl acetate and then mixed with the filtrate. The solvent was distilled off from the resulting filtrate under reduced pressure. 2.5 L of acetonitrile was added thereto when 6 L was distilled. The solvent was further distilled under reduced pressure. After distillation of 2.4 L, 2.5 L of acetonitrile was added. The solvent was further distilled under reduced pressure. After distillation of 2.6 L, acetonitrile was added so that the total amount was 5-fold by volume (v/w) relative to tert-butyl(S)-3-(4-amino-3-iodo-1H-pyrazolo[3,4-d]pyrimidin-1-yl)pyrrolidine-1-carboxylate.

Purified water (500 g) and methanesulfonic acid (234.5 g) were added to the obtained mixture, and the mixture was stirred at an internal temperature of 60°C or more for 2 hours. 8 L of acetonitrile was added to the mixture over a period of 5 minutes to crystalize (S)-3-((3,5-dimethoxyphenyl)ethynyl)-1-(pyrrolidin-3-yl)-1H-pyrazolo[3,4-d]pyrimidine-4-amine.2 methanesulfonate. The mixture was cooled from an internal temperature of 52°C to 25°C over a period of 3 hours, and then stirred for 11 hours. Wet crystals of (S)-3-((3,5-dimethoxyphenyl)ethynyl)-1-(pyrrolidin-3-yl)-1H-pyrazolo[3,4-d]pyrimidine-4-amine·2 methanesulfonate were obtained from the mixture by suction filtration using a Nutsche

filter. The wet crystals were washed with 2 L of acetonitrile and dried under reduced pressure at 60°C, thereby obtaining the title compound (571.56 g, yield 88.4%) as light yellow white crystals.

1H-NMR (400 MHz, D2O) δ8.358 (s, 1H), 6.741 (d, 2.4 Hz, 2H), 6.539 (t, 2.2 Hz, 1H), 5.715-5.662 (m, 1H), 3.955-3.860 (m, 2H), 3.828 (s, 6H), 3.799-3.645 (m, 2H), 2.838 (s, 6H), 2.766-2.666 (m, 1H), 2.536-2.471 (m, 1H)

Production Example 2: Synthesis of (S)-N-(1-(1-acryloylpyrrolidin-3-yl)-3-((3,5-dimethoxyphenyl)ethynyl)-1H-pyrazolo[3,4-d]pyrimidin-4-yl)acrylamide

Compound A (348 mg, PTL 1) and DMF (6 mL) were placed in a reaction vessel, and 60% mineral oil-containing sodium hydride (49 mg) was added thereto. Thereafter, 3-chloropropionyl chloride (120 μL) was added dropwise, and the mixture was stirred at room temperature for 30 minutes. The reaction solution was added to a saturated aqueous solution of sodium hydrogen carbonate, and extraction was performed with ethyl acetate, followed by distilling off the solvent of the organic layer. The residue was purified by column chromatography (Biotage SNAP Ultra HP-Sphere, chloroform/methanol), thereby obtaining the title compound (49mg).

1H-NMR (400 MHz, CDCl3) δ8.84 (1H, s), 8.71 (1H, d, J=1.2 Hz, NH), 7.37 (1H, ddd, J=17.0, 10.2, 5.1 Hz), 6.86 (2H, dd, J=2.2, 0.7 Hz), 6.74-6.48 (1H, m), 6.60-6.42 (1H, m), 6.56 (1H, dd, J=4.4, 2.2 Hz), 6.46-6.39 (1H, m), 5.96 (1H, ddd, J=10.4, 4.2, 1.2 Hz), 5.77-5.69 (1H, m), 5.68-5.56 (1H, m), 4.20-4.01 (2H, m), 4.15-3.70 (2H, m), 3.84 (6H, s), 2.75-2.43 (2H, m); m/z 473 [M+H]+

Production Example 3: Synthesis of (S)-1-(3-(4-amino-3-((3,5-dimethoxyphenyl)ethynyl)-1H-pyrazolo[3,4-d]pyrimidin-1-yl) pyrrolidin-1-yl)-3-chloropropan-1-one

(S)-3-((3,5-dimethoxyphenyl)ethynyl)-1-(pyrrolidin-3-yl)-1H-pyrazolo[3,4-d]pyrimidine-4-amine·2 methanesulfonate (1.0 g) and dimethyl sulfoxide (25 mL) were placed in a reaction vessel, and then a 1M aqueous sodium hydroxide solution (3.6 mL) was added thereto. 3-Chloropropanecarboxylic acid (297 mg) and DMT-MM hydrate (694 mg) were added thereto, followed by stirring at room temperature for 2 hours. 3-Chloropropanecarboxylic acid (67 mg) and DMT-MM hydrate (272 mg) were further added, and the mixture was stirred at room temperature for 1 hour. The reaction solution was added to an aqueous solution of sodium hydrogen carbonate, and extraction was performed with ethyl acetate, followed by distilling off the solvent of the organic layer. The residue was purified by column chromatography (Biotage SNAP Ultra HP-Sphere, chloroform/methanol), thereby obtaining the title compound (713 mg).

1H-NMR (400 MHz, DMSO-d6) δ8.26 (1H, s), 7.98 (1H, brs), 6.90 (2H, m), 6.78 (1H, brs), 6.60 (1H, m), 5.55-5.38 (1H, m), 4.05-3.76 (2H, m), 3.85-3.74 (2H, m), 3.80-3.49 (2H, m), 3.77 (6H, s), 2.79 (2H, dt, J=25.8, 6.8 Hz), 2.49-2.28 (2H, m); m/z 455,457 [M+H]+

Production Example 4: Synthesis of (S)-1-(3-(4-amino-3-((3,5-dimethoxyphenyl)ethynyl)-1H-pyrazolo[3,4-d]pyrimidin-1-yl) pyrrolidin-1-yl)-2-propen-1-one

(S)-3-((3,5-dimethoxyphenyl)ethynyl)-1-(pyrrolidin-3-yl)-1H-pyrazolo[3,4-d]pyrimidine-4-amine·2 methanesulfonate (10 g), water (56 mL), and acetonitrile (50 mL) were placed in a reaction vessel, and a 5N aqueous sodium hydroxide solution (14 mL) was added thereto. A solution prepared by diluting 3-chloropropionyl chloride (2.51 g) with acetonitrile (20 mL) was added, and the mixture was stirred at a temperature of 20 to 30°C for 2 hours. The reaction solution was partially taken out and measured by HPLC (condition 2). The peak area of compound B was confirmed to be less than 0.1% of the total peak area. At this stage, the diamide compound and the 3CP diamide compound were not detected in HPLC. Thereafter, a 5N aqueous sodium hydroxide solution (4 mL) was further added, and the mixture was stirred at a temperature of 20 to 30°C for 2 hours. Thereafter, a 5N aqueous sodium hydroxide solution (2 mL) was further added, and the mixture was stirred at a temperature of 20 to 30°C for 2 hours. The reaction solution was partially taken out and measured by HPLC (condition 2). The peak area of the A-1-3CP compound was confirmed to be less than 0.1% of the total peak area. After completion of the reaction, water (150 mL) was added, and the insoluble matter was collected by filtration, followed by washing with water (50 mL) and acetonitrile (50 mL). The collected matter was dried under reduced pressure at 60°C, thereby obtaining the title compound (5.60 g, yield 74.5%).

Analysis of the obtained title compound by HPLC detected no diamide compound.

Production Example 5: Synthesis of (S)-1-(3-(4-amino-3-((3,5-dimethoxyphenyl)ethynyl)-1H-pyrazolo[3,4-d]pyrimidin-1-yl) pyrrolidin-1-yl)-2-propen-1-one

(S)-3-((3,5-dimethoxyphenyl)ethynyl)-1-(pyrrolidin-3-yl)-1H-pyrazolo[3,4-d]pyrimidine-4-amine·2 methanesulfonate (25.0 g), water (69 mL), and acetonitrile (158 mL) were placed in a reaction vessel, and a 5N aqueous sodium hydroxide solution (35 mL) was added thereto. A solution prepared by diluting 3-chloropropionyl chloride (6.27 g) with

acetonitrile (50 mL) was added over a period of 10 minutes. After completion of the dropwise addition, the mixture was stirred at 30°C for 30 minutes. The reaction solution was partially taken out and measured by HPLC (condition 3). The peak area of compound B was confirmed to be less than 0.1% of the total peak area. At this stage, the diamide compound and the 3CP diamide compound were not detected in HPLC. Thereafter, a 5N aqueous sodium hydroxide solution (25 mL) was further added, and the mixture was stirred at 30°C for 4 hours. The reaction solution was partially taken out and measured by HPLC (condition 3). The peak area of the A-1-3CP compound was confirmed to be less than 0.1% of the total peak area. After completion of the reaction, water (550 mL) was added over a period of 2 hours. After completion of the dropwise addition, the internal temperature was adjusted to 25°C, and the mixture was stirred for 1.5 hours. The insoluble matter was collected by filtration and washed with water (125 mL), followed by drying the washed matter at 60°C under reduced pressure, thereby obtaining the title compound (16.02 g, yield 85.3%).

Analysis of the obtained title compound by HPLC detected no diamide compound.

Production Example 6: Synthesis of (S)-1-(3-(4-amino-3-((3,5-dimethoxyphenyl)ethynyl)-1H-pyrazolo[3,4-d]pyrimidin-1-yl) pyrrolidin-1-yl)-2-propen-1-one by using 3.1 equivalents of sodium hydroxide

(S)-3-((3,5-dimethoxyphenyl)ethynyl)-1-(pyrrolidin-3-yl)-1H-pyrazolo[3,4-d]pyrimidine-4-amine·2 methanesulfonate (3.0 g), water (9.15 mL), and acetonitrile (18.96 mL) were placed in a reaction vessel, and then a 5N aqueous sodium hydroxide solution (3.34 mL) was added thereto, followed by adjusting the internal temperature to 30°C. A solution prepared by diluting 3-chloropropionyl chloride (0.753 g) with acetonitrile (6 mL) was added over a period of 10 minutes. After completion of the dropwise addition, the mixture was stirred at 30°C for 30 minutes. The reaction solution was partially taken out and measured by HPLC (condition 3). The peak area of compound B was confirmed to be less than 0.1% of the total peak area. At this stage, the diamide compound and the 3CP diamide compound were not detected in HPLC. Thereafter, a 5N aqueous sodium hydroxide solution (3.56 mL) was further added, and the mixture was stirred at 30°C for 4 hours. The reaction solution was partially taken out and measured by HPLC (condition 3). The peak area of the A-1-3CP compound was confirmed to be less than 0.1% of the total peak area. After completion of the reaction, water (66 mL) was added over a period of 30 minutes, and the internal temperature was adjusted to 25°C, followed by stirring for 1 hour. The insoluble matter was collected by filtration and washed with water (15 mL), followed by drying the collected matter under reduced pressure at 60°C, thereby obtaining the title compound (1.874 g, yield 83.1%).

Analysis of the obtained title compound by HPLC detected no diamide compound.

Production Example 7: Synthesis of (S)-1-(3-(4-amino-3-((3,5-dimethoxyphenyl)ethynyl)-1H-pyrazolo[3,4-d]pyrimidin-1-yl) pyrrolidin-1-yl)-2-propen-1-one by using N,N-diisopropylethylamine

(S)-3-((3,5-dimethoxyphenyl)ethynyl)-1-(pyrrolidin-3-yl)-1H-pyrazolo[3,4-d]pyrimidine-4-amine·2 methanesulfonate (3.0 g), water (12.48 mL), and acetonitrile (18.96 mL) were placed in a reaction vessel, and N,N-diisopropylethylamine (2.26 g) was added thereto, followed by adjusting the internal temperature to 30°C. A solution prepared by diluting 3-chloropropionyl chloride (0.753 g) with acetonitrile (6 mL) was added over a period of 10 minutes. After completion of the dropwise addition, the mixture was stirred at 30°C for 30 minutes. The reaction solution was partially taken out and measured by HPLC (condition 3). The peak area of compound B was confirmed to be less than 1.0% of the total peak area. At this stage, the diamide compound and the 3CP diamide compound were not detected in HPLC. Thereafter, a 5N aqueous sodium hydroxide solution (6.47 mL) was further added, and the mixture was stirred at 30°C for 4 hours and 30 minutes. After completion of the reaction, water (66 mL) was added over a period of 30 minutes, and the internal temperature was adjusted to 25°C, followed by stirring for 1 hour and 30 minutes. The insoluble matter was collected by filtration and washed with water (15 mL), followed by drying the washed matter under reduced pressure at 60°C, thereby obtaining the title compound (1.897 g, yield 84.2%).

Analysis of the obtained title compound by HPLC detected no diamide compound.

Production Example 8: Synthesis of (S)-1-(3-(4-amino-3-((3,5-dimethoxyphenyl)ethynyl)-1H-pyrazolo[3,4-d]pyrimidin-1-yl) pyrrolidin-1-yl)-2-propen-1-one by using potassium hydroxide

(S)-3-((3,5-dimethoxyphenyl)ethynyl)-1-(pyrrolidin-3-yl)-1H-pyrazolo[3,4-d]pyrimidine-4-amine·2 methanesulfonate (500 mg), water (3.5 mL), and acetonitrile (3.5 mL) were placed in a reaction vessel, and then a 5N potassium hydroxide (equivalent to 280.7 mg) was added thereto. A solution prepared by diluting 3-chloropropionyl chloride (205.2 mg) with acetonitrile (1 mL) was added, and the mixture was stirred at a temperature of 20 to 30°C for 30 minutes. The reaction solution was partially taken out and measured by HPLC (condition 2). The peak area of compound B was confirmed to be less than 1.0% of the total peak area. At this stage, the diamide compound and the 3CP diamide compound were not detected in HPLC.

This suggests that due to its ability to suppress the formation of related substances such as the diamide

compound, the method of Production Example 8 is excellent in maintaining the high quality of compound A and is suitable for mass production of a pharmaceutical product.

Production Example 9: Synthesis of (S)-1-(3-(4-amino-3-((3,5-dimethoxyphenyl)ethynyl)-1H-pyrazolo[3,4-d]pyrimidin-1-yl) pyrrolidin-1-yl)-2-propen-1-one by using sodium hydroxide

(S)-3-((3,5-dimethoxyphenyl)ethynyl)-1-(pyrrolidin-3-yl)-1H-pyrazolo[3,4-d]pyrimidine-4-amine·2 methanesulfonate (500 mg), water (3.5 mL), and acetonitrile (3.5 mL) were placed in a reaction vessel, and a 5N sodium hydroxide (1.0 mL) was added thereto. A solution prepared by diluting 3-chloropropionyl chloride (205.2 mg) with acetonitrile (1 mL) was added, and the mixture was stirred at a temperature of 20 to 30°C for 30 minutes. The reaction solution was partially taken out and measured by HPLC (condition 2). The peak area of compound B was confirmed to be less than 1.0% of the total peak area. At this stage, the peak area of the 3CP diamide compound was 0.31% of the total peak area.

This suggests that due to its ability to suppress the formation of the diamide compound induced from the 3CP diamide compound, the method of Production Example 9 is excellent in maintaining the quality of compound A and is suitable for mass production of a pharmaceutical product.

Production Example 10: Synthesis of (S)-1-(3-(4-amino-3-((3,5-dimethoxyphenyl)ethynyl)-1H-pyrazolo[3,4-d]pyrimidin-1-yl) pyrrolidin-1-yl)-2-propen-1-one by using acryloyl chloride

(S)-3-((3,5-dimethoxyphenyl)ethynyl)-1-(pyrrolidin-3-yl)-1H-pyrazolo[3,4-d]pyrimidine-4-amine·2 methanesulfonate (5.0 g), N-methyl-2-pyrrolidone (50 mL), and potassium phosphate (3.43 g) were placed in a reaction vessel, and the internal temperature was adjusted to 10°C or less. Acryloyl chloride (1.219 g) was added thereto, and the mixture was stirred for 3 hours. The reaction solution was partially taken out and measured by HPLC (condition 2). The peak area of compound B was confirmed to be less than 1.0% of the total peak area, and the peak area of the diamide compound was confirmed to be 2% or more.

Thereafter, water (25 g) and phosphoric acid (880 mg) were added, and the mixture was stirred at a temperature of 20 to 30°C for 3 hours. The reaction solution was partially taken out and measured by HPLC (condition 2). The peak area of the diamide compound was confirmed to be less than 2% of the total peak area.

Thereafter, the pH of the reaction solution was adjusted to 7 to 8 with a 20% aqueous potassium hydroxide solution. Ethanol (40 mL) was then added thereto, and the mixture was heated to an internal temperature of 50 to 60°C to dissolve it. At an internal temperature of 50°C, crystal II of compound A (25.2 mg, PTL 8) was added, and the mixture was stirred for 3 hours. Thereafter, water (176.3 mL) was added dropwise over a period of 3 hours. The internal temperature was cooled from 50°C to 25°C over a period of 15 hours. At this stage, the entire solution, containing a solution and insoluble matter, was 375 mL. From this solution, the insoluble matter was collected by filtration and washed with water (30 mL), followed by drying the washed matter under reduced pressure at 60°C, thereby obtaining the title compound (2.936 g, yield 78.2%).

Analysis of the obtained title compound by HPLC detected no diamide compound.

However, when the insoluble matter was filtered off from the entire solution (375 mL), containing a solution and insoluble matter, by using a Nutsche filter with a filtration area of 12.56 $cm^2$, filtration took 1716 seconds, and the filtration rate was 0.2 mL/second. This filtration rate was about 1/20 of the filtration rate in the Examples when compared in terms of the time period that it took for filtration on the same scale as that of the Examples.

This suggests that the method of Production Example 10 took time for filtration of compound A, and indicates that the conditions may be insufficient as a method for mass production.

Production Example 11

(S)-3-((3,5-dimethoxyphenyl)ethynyl)-1-(pyrrolidin-3-yl)-1H-pyrazolo[3,4-d]pyrimidine-4-amine·2 methanesulfonate (500 mg), water (2.5 mL), and acetonitrile (2.5 mL) were placed in a reaction vessel, and a 5N aqueous sodium hydroxide solution (1 mL) was added thereto. A solution prepared by diluting acryloyl chloride (162.9 mg) with acetonitrile (1 mL) was then added thereto, followed by stirring the mixture at ice cooling temperature for 30 minutes. The reaction solution was partially taken out and measured by HPLC (condition 2). The peak area of compound B was 1.94% of the total peak area, and the peak area of the diamide compound was 3.02% of the total peak area.

This suggests that the method of Production Example 11 may be insufficient as a method for producing a pharmaceutical product from the standpoint of product quality.

Production Example 12

(S)-3-((3,5-dimethoxyphenyl)ethynyl)-1-(pyrrolidin-3-yl)-1H-pyrazolo[3,4-d]pyrimidine-4-amine·2 methanesulfonate (500 mg), water (3.5 mL), and acetonitrile (2.5 mL) were placed in a reaction vessel, and then potassium phosphate (343.8 mg) was added thereto. A solution prepared by diluting acryloyl chloride (97.8 mg) with acetonitrile (1 mL) was then added thereto, followed by stirring the mixture at ice cooling temperature for 60 minutes. The reaction solution was partially taken out and measured by HPLC (condition 2). The peak area of compound B was 17.79% of the total peak area, and the peak area of the diamide compound was 20.85% of the total peak area.

This suggests that the method of Production Example 12 may be insufficient as a method for mass production of compound A.

Production Example 13

(S)-3-((3,5-dimethoxyphenyl)ethynyl)-1-(pyrrolidin-3-yl)-1H-pyrazolo[3,4-d]pyrimidine-4-amine·2 methanesulfonate (500 mg), water (3.5 mL), and acetonitrile (2.5 mL) were placed in a reaction vessel, and potassium phosphate (343.8 mg) was added thereto. A solution prepared by diluting acryloyl chloride (146.6 mg) with acetonitrile (1 mL) was then added thereto, followed by stirring the mixture at ice cooling temperature for 60 minutes. The reaction solution was partially taken out and measured by HPLC (condition 2). Although the peak area of compound B was confirmed to be less than 1.0% of the total peak area, the peak area of the diamide compound was 44.23% of the total peak area.

This suggests that the method of Production Example 13 may be insufficient as a method for mass production of compound A.

Production Example 14

(S)-3-((3,5-dimethoxyphenyl)ethynyl)-1-(pyrrolidin-3-yl)-1H-pyrazolo[3,4-d]pyrimidine-4-amine·2 methanesulfonate (500 mg), water (3.5 mL), and acetonitrile (2.5 mL) were placed in a reaction vessel, and diisopropylethylamine (406.2 mg) was added thereto. A solution prepared by diluting acryloyl chloride (171 mg) with acetonitrile (1 mL) was then added thereto, followed by stirring the mixture at ice cooling temperature for 60 minutes. The reaction solution was partially taken out and measured by HPLC. Although the peak area of compound B was less than 0.1% of the total peak area, the peak area of the diamide compound was 2.04%. At this stage, despite the recrystallization in various ways, the content of the diamide compound in compound A could not be reduced to less than 0.1%.

This suggests that the method of Production Example 14 may be insufficient as a method for producing a pharmaceutical product from the standpoint of product quality.

Production Example 15

(S)-3-((3,5-dimethoxyphenyl)ethynyl)-1-(pyrrolidin-3-yl)-1H-pyrazolo[3,4-d]pyrimidine-4-amine·2 methanesulfonate (500 mg), water (3.5 mL), and acetonitrile (3.5 mL) were placed in a reaction vessel, and potassium carbonate (620.6 mg) was added thereto. A solution prepared by diluting 3-chloropropionyl chloride (205.2 mg) with acetonitrile (1 mL) was then added, followed by stirring the mixture at a temperature of 20 to 30°C for 30 minutes. The reaction solution was partially taken out and measured by HPLC (condition 2). Although the peak area of compound B was less than 1.0% of the total peak area, the peak area of the 3CP diamide compound was 3.41% of the total peak area.

This suggests that the method of Production Example 15 may be insufficient as a method for producing a pharmaceutical product from the standpoint of product quality.

Production Example 16

(S)-3-((3,5-dimethoxyphenyl)ethynyl)-1-(pyrrolidin-3-yl)-1H-pyrazolo[3,4-d]pyrimidine-4-amine·2 methanesulfonate (500 mg), water (3.5 mL), and acetonitrile (3.5 mL) were placed in a reaction vessel, and potassium phosphate (343.1 mg) was added thereto. A solution prepared by diluting 3-chloropropionyl chloride (205.2 mg) with acetonitrile (1 mL) was then added, followed by stirring the mixture at a temperature of 20 to 30°C for 30 minutes. The reaction solution was partially taken out and measured by HPLC (condition 2). Although the peak area of compound B was less than 1.0% of the total peak area, the peak area of the 3CP diamide compound was 6.58% of the total peak area.

This suggests that the method of Production Example 16 may be insufficient as a method for producing a pharmaceutical product from the standpoint of product quality.

Production Example 17

(S)-3-((3,5-dimethoxyphenyl)ethynyl)-1-(pyrrolidin-3-yl)-1H-pyrazolo[3,4-d]pyrimidine-4-amine·2 methanesulfonate (500 mg), water (3.5 mL), and acetonitrile (3.5 mL) were placed in a reaction vessel, and triethylamine (318.0 mg) was added thereto. A solution prepared by diluting 3-chloropropionyl chloride (171.0 mg) with acetonitrile (1 mL)

was then added, followed by stirring the mixture at a temperature of 20 to 30°C for 30 minutes. The reaction solution was partially taken out and measured by HPLC (condition 2). Although the peak area of compound B was less than 1.0% of the total peak area, the peak area of the 3CP diamide compound was 1.68% of the total peak area, with many other kinds of related substances being detected.

This suggests that the method of Production Example 17 may be insufficient as a method for producing a pharmaceutical product from the standpoint of product quality.

Production Example 18

(S)-3-((3,5-dimethoxyphenyl)ethynyl)-1-(pyrrolidin-3-yl)-1H-pyrazolo[3,4-d]pyrimidine-4-amine·2 methanesulfonate (500 mg), water (3.5 mL), and acetonitrile (3.5 mL) were placed in a reaction vessel, and pyridine (3551.6 mg) was added thereto. A solution prepared by diluting 3-chloropropionyl chloride (205.2 mg) with acetonitrile (1 mL) was then added, followed by stirring the mixture at a temperature of 20 to 30°C for 30 minutes. The reaction solution was partially taken out and measured by HPLC (condition 2). The peak area of compound B was 43.72%, indicating that the reaction did not sufficiently proceed.

This suggests that the method of Production Example 18 may be insufficient as a method for mass production of compound A.

Production Example 19

(S)-3-((3,5-dimethoxyphenyl)ethynyl)-1-(pyrrolidin-3-yl)-1H-pyrazolo[3,4-d]pyrimidine-4-amine·2 methanesulfonate (500 mg), water (3.5 mL), and acetonitrile (3.5 mL) were placed in a reaction vessel, and potassium tert-butoxide (503.8 mg) was added thereto. A solution prepared by diluting 3-chloropropionyl chloride (205.2 mg) with acetonitrile (1 mL) was then added, followed by stirring the mixture at a temperature of 20 to 30°C for 30 minutes. The reaction solution was partially taken out and measured by HPLC (condition 2). Although the peak area of compound B was less than 1.0% of the total peak area, the peak area of the 3CP diamide compound was 4.66% of the total peak area.

This suggests that the method of Production Example 19 may be insufficient as a method for producing a pharmaceutical product from the standpoint of product quality.

The following table illustrates the results of production of the compound represented by formula (A-1) in which $L^2$ is a chlorine atom from 2 methanesulfonate of compound B.

Table 7

| | | Production Example 4 | Production Example 5 | Production Example 6 | Production Example 7 | Production Example 8 | Production Example 9 | Production Example 10 | Production Example 11 |
|---|---|---|---|---|---|---|---|---|---|
| Reaction Conditions | Reagent | 3-Chloropropionyl Chloride | 3-Chloropropionyl Chloride | 3-Chloropropionyl Chloride | 3-Chloropropionyl Chloride | 3-Chloropropionyl Chloride | 3-Chloropropionyl Chloride | Acryloyl Chloride | Acryloyl Chloride |
| | Reagent Equivalent | 1.10 | 1.10 | 1.10 | 1.10 | 1.80 | 1.80 | 1.50 | 2.00 |
| | Base (Valence) | Sodium Hydroxide (Monovalent) | Sodium Hydroxide (Monovalent) | Sodium Hydroxide (Monovalent) | Diisopropylethylamine (Monovalent) | Potassium Hydroxide (Monovalent) | Sodium Hydroxide (Monovalent) | Potassium Phosphate (Trivalent) | Sodium Hydroxide (Monovalent) |
| | Base Equivalent (After Acid Addition Salt Is Subtracted) | 1.90 | 1.90 | 1.10 | 1.24 | 3.57 | 3.57 | 1.14 | 3.57 |
| | Solvent | Acetonitrile/Water (1:1) | Acetonitrile/Water (2:1) | Acetonitrile/Water (2:1) | Acetonitrile/Water (10:1) | Acetonitrile/Water (1:1) | Acetonitrile/Water (1:1) | N-Methylpyrrolidone | Acetonitrile/Water (1:1) |
| | Temperature | 20-30°C | 20-30°C | 30°C | 30°C | 20-30°C | 20-30°C | 20-30°C | Ice Cooling Temperature |
| HPLC during Reaction | Compound B | Less than 1.0% | Less than 1.0% | Less than 1.0% | Less than 1.0% | Less than 1.0% | Less than 1.0% | N.A. | 1.94% |
| | Diamide+3CP Diamide | N.D. | N.D. | N.D. | N.D. | N.D. | 0.31% | N.A. | 3.02% |
| Yield of Compound A (%) | | 74.5 | 85.3 | 83.1 | 84.2 | N.A. | N.A. | 78.2 | N.A. |

| | | Production Example 12 | Production Example 13 | Production Example 14 | Production Example 15 | Production Example 16 | Production Example 17 | Production Example 18 | Production Example 19 |
|---|---|---|---|---|---|---|---|---|---|
| Reaction Conditions | Reagent | Acryloyl Chloride | Acryloyl Chloride | Acryloyl Chloride | 3-Chloropropionyl Chloride | 3-Chloropropionyl Chloride | 3-Chloropropionyl Chloride | 3-Chloropropionyl Chloride | 3-Chloropropionyl Chloride |
| | Reagent Equivalent | 1.20 | 1.80 | 1.40 | 1.80 | 1.80 | 1.50 | 1.80 | 1.80 |
| | Base (Valence) | Potassium Phosphate (Trivalent) | Potassium Phosphate (Trivalent) | Diisopropylethylamine (Monovalent) | Potassium Carbonate (Divalent) | Potassium Phosphate (Trivalent) | Triethylamine (Monovalent) | Pyridine (Monovalent) | Potassium Tert-Butoxide (Monovalent) |
| | Base Equivalent (After Acid Addition Salt Is Subtracted) | 1.14 | 1.14 | 1.50 | 4.00 | 1.14 | 1.50 | 48.0 | 3.00 |
| | Solvent | Acetonitrile/Water (1:1) | Acetonitrile/Water (1:1) | Acetonitrile/Water (1:1) | Acetonitrile/Water (1:1) | Acetonitrile/Water (1:1) | Acetonitrile/Water (1:1) | Acetonitrile/Water (1:1) | Acetonitrile/Water (1:1) |
| | Temperature | Ice Cooling Temperature | Ice Cooling Temperature | Ice Cooling Temperature | 30°C | 20-30°C | 20-30°C | 20-30°C | 20-30°C |
| HPLC during Reaction | Compound B | 17.79% | Less than 1.0% | Less than 1.0% | Less than 1.0% | Less than 1.0% | Less than 1.0% | 43.72% | Less than 1.0% |
| | Diamide+3CP Diamide | 20.85% | 44.23% | 2.04% | 3.41% | 6.58% | 1.68% | N.D. | 4.66% |
| Yield of Compound A (%) | | N.A. | N.A. | N.A. | N.A. | N.A. | N.A. | N.A. | N.A. |

The phrase "After Acid Addition Salt Is Subtracted" written for the base equivalent refers to a value determined by subtracting the number of equivalents of the base required for neutralization of methanesulfonic acid (acid addition part) of 2 methanesulfonate of compound B. The expression "N.D." means "below the detection limit," and the expression "N.A." means that measurement was not performed.

The results indicate that the probability of containing diamide in compound A is low when 1.1 equivalents of 3-chloropropionyl chloride is used per equivalent of compound B. The results also indicate that when sodium hydroxide or potassium hydroxide is used as a base, and when 1.8 equivalents of 3-chloropropionyl chloride per equivalent of compound B is used, the probability of containing diamide in compound A is low.

Test Example 1: Confirmation of Resolution

In accordance with the method in Example 1 of PTL 4, compound A obtained in Production Examples 4, 5, and 6 was crystallized. Analysis of the filtrate obtained at this stage by a mass spectrum and HPLC detected the CE compound (retention time: 18.3 minutes), the OH compound (retention time: 23.1 minutes), the CDA1 compound (retention time: 24.7 minutes), the CHO compound (retention time: 27.3 minutes), the diamide compound (retention time: 47.9 minutes), the UK compound (retention time: 48.5 minutes), the MA compound (retention time: 55.9 minutes), and the Dimer compound (retention time: 58.6 minutes). The CDA1 compound and diamide compound coincided with the separately synthesized CDA1 compound and diamide compound in retention time in HPLC analysis. The conditions for the analysis by HPLC are as described below.

Detector: Ultraviolet absorption photometer (wavelength: 220 nm) Column: A stainless steel tube (inner diameter: 4.6 mm, length: 15 cm) was filled with a 3-μm octadecylsilanized silica gel for liquid chromatography (InertSustain C18, produced by GL Sciences Inc.)
Column Temperature: 40°C
Injection Volume: 10 μL
Flow Rate: 1.0 mL/minute
Mobile Phase A: 950 mL of a phosphate buffer solution was prepared by adding 0.685 mL of phosphoric acid to 1000 mL of water, and then adding a 45% potassium hydroxide solution thereto to adjust the pH to 6.8. 50 mL of acetonitrile was then added thereto.
Mobile Phase B: Acetonitrile

The proportion of mobile phase A and mobile phase B was changed as shown below to control the concentration gradient.

Table 8

| Time after Injection (min) | Mobile Phase A (vol%) | Mobile Phase B (vol%) |
|---|---|---|
| 0 to 10 | 70 → 25 | 30 → 75 |
| 10 to 35 | 25 | 75 |
| 35 to 35. 1 | 25 → 70 | 75 → 30 |
| 35. 1 to 50 | 70 | 30 |

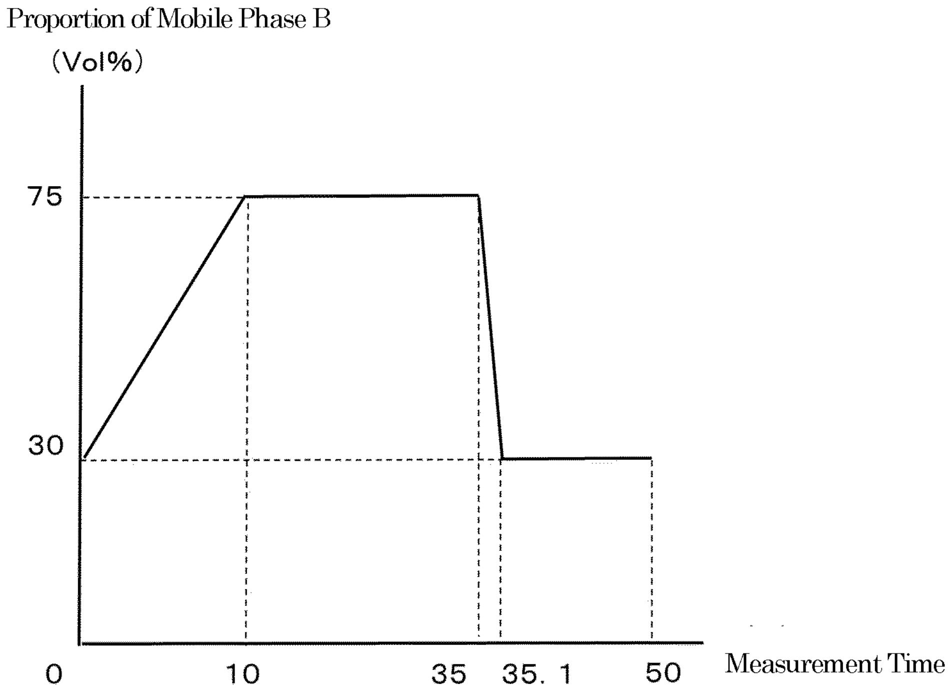


This indicates that not only compound B and the A-1-3CP compound (intermediates in production of compound A) but also the CE compound, OH compound, CDA1 compound, CHO compound, diamide compound, UK compound, MA compound, and Dimer compound are related substances that could be contained in the drug substance and/or preparation of compound A. Additionally, this also indicates that these compounds are usable as a standard for maintaining the quality of compound A.

Production Example 1: Synthesis of (S)-1-(3-(4-amino-3-((3,5-dimethoxyphenyl)ethynyl)-1H-pyrazolo[3,4-d]pyrimidin-1-yl) pyrrolidin-1-yl)-3-hydroxypropan-1-one (OH compound)

Compound B·2 methanesulfonate (1.004 g) obtained in Production Example 1 was dissolved in DMSO (25 mL), and a 1M aqueous sodium hydroxide solution (3.610 mL) was added thereto at room temperature. 3-Hydroxypropanoic acid (30% aqueous solution, 1.50 mL) and DMT-MM·monohydrate (1.067 g) were added to the obtained solution at room temperature, followed by stirring for 1 hour. An aqueous solution of sodium hydrogen carbonate was added to the reaction solution, followed by extraction with ethyl acetate. The extract was dried over anhydrous sodium sulfate and concentrated under reduced pressure. The thus-obtained residue was purified by silica gel chromatography (Biotage SNAP Ultra HP-Sphere, 50 g, followed by SNAP Isolute Flash-NH, 110 g, eluate: chloroform/methanol), thereby obtaining the title compound.

1H-NMR (400 MHz, DMSO-d6) δ 8.26 (1H, d, J=3.4 Hz), 7.97 (1H, brs), 6.91 (2H, d, J=2.4 Hz), 6.76 (1H, brs), 6.60 (1H, t, J=2.4 Hz), 5.45 (1H, m), 4.52 (1H, dt, J=5.4, 17.6 Hz), 4.05-3.80 (1H, m), 3.80-3.62 (4H, m), 3.78 (6H, s), 3.62-3.47 (1H, m), 2.52-2.27 (5H, m); m/z 473.3 [M+H]+.

Production Example 2: Synthesis of (S)-3-(4-amino-3-((3,5-dimethoxyphenyl)ethynyl)-1H-pyrazolo[3,4-d]pyrimidin-1-yl) pyrrolidine-1-carbaldehyde (CHO compound)

Compound B·2 methanesulfonate (520.4 mg) obtained in Production Example 1 was dissolved in DMSO (10 mL), and a 1M aqueous sodium hydroxide solution (1.870 mL) was added thereto at room temperature. Formic acid (42.35 μL) and DMT-MM·monohydrate (412.8 mg) were added to the obtained solution at room temperature, followed by stirring for 20 minutes. An aqueous solution of sodium hydrogen carbonate was added to the reaction solution, followed by extraction with ethyl acetate. The extract was dried over anhydrous sodium sulfate and concentrated under reduced pressure. Ethyl acetate was added to the thus-obtained residue to precipitate the title compound from the solution. The generated solid was collected by filtration and washed with ethyl acetate, followed by drying 65°C under reduced pressure, thereby obtaining the CHO compound (239.0 mg).

1H-NMR (400 MHz, CDCl3) δ 8.37 (1H, s), 8.30 (1H, d, J=9.5 Hz), 6.76-6.72 (2H, m), 6.56-6.52 (1H, m), 5.92 (2H, brs), 5.62-5.45 (1H, m), 4.09-3.97 (2H, m), 3.94-3.63 (2H, m), 3.82 (6H, s), 2.71-2.41 (2H, m); m/z 393.3[M+H]+.

Production Example 3: Synthesis of 1,3-bis((S)-3-(4-amino-3-((3,5-dimethoxyphenyl)ethynyl)-1H-pyrazolo[3,4-d]pyrimidin-1-yl) pyrrolidin-1-yl)propan-1-one (MA compound)

A 50% aqueous acetonitrile solution (4 mL) was added to the compound B·2 methanesulfonate (100.4 mg) obtained in Production Example 1 to dissolve the compound B·2 methanesulfonate. A 1M aqueous sodium hydroxide solution (360.7 mg) was added thereto at room temperature. Compound A (75.4 mg) disclosed in PTL 1 and DBU (26.9 μL) were added to the obtained suspension. After the mixture was stirred at 80°C for 11 hours, heating was ended, followed by further stirring until it returned to room temperature. The precipitated solid was collected by filtration and washed with a 50% aqueous acetonitrile solution, followed by drying at 65°C under reduced pressure, thereby obtaining a crude product of the title compound. The obtained crude product was purified by silica gel column chromatography (Biotage SNAP Isolute Flash-NH, 25 g, eluate: ethyl acetate/methanol), thereby obtaining the MA compound (109.1 mg). 1H-NMR (400 MHz, CDCl3) δ 8.42-8.29 (2H, m), 6.81-6.64 (2H, m), 6.52 (2H, s), 6.18-5.90 (4H, m), 5.61-5.41 (1H, m), 4.08-3.59 (4H, m), 3.81 (12H, s), 3.17 (1H, dt, J=9.0, 23.6 Hz), 3.08-2.78 (5H, m), 2.71-2.30 (6H, m); m/z 783.5 [M+H]+.

Production Example 4: Synthesis of (S)-3-((1-(1-acryloylpyrrolidin-3-yl)-6-((3,5-dimethoxyphenyl)ethynyl)-1H-pyrazolo[3,4-d]pyrimidin-4-yl)amino)propanoic acid (CE compound)

Compound A disclosed in PTL 1 (502.0 mg) was dissolved in DMF (10 mL), and 60% sodium hydride (powder dispersed in liquid paraffin, 60.3 mg) was added thereto at ice cooling temperature, followed by stirring for 10 minutes. 3-Bromopropanoic acid tert-butyl ester (220.2 μL) was added at icecooling temperature, and the mixture was stirred for 15 minutes, followed by heating to room temperature and stirring for 20 hours. A saturated aqueous solution of ammonium chloride was added to the reaction solution, followed by extraction with ethyl acetate. The extract was dried over anhydrous sodium sulfate and concentrated under reduced pressure. The thus-obtained residue was purified by silica gel column chromatography (Biotage SNAP Ultra HP-Sphere, 50 g, eluate: ethyl acetate/methanol), thereby obtaining tert-butyl(S)-3-((1-acryloylpyrrolidin-3-yl)-3-((3,5-dimethoxyphenyl)ethynyl-1H-pyrazolo[3,4-d]pyrimidin-4-yl) amino) propanoate (a protected carboxylic acid with tert-butyl group of the title compound, 510.0 mg).

The obtained protected carboxylic acid with tert-butyl group (510.0 mg) was dissolved in formic acid (8 mL), followed by stirring at room temperature for 20 hours, and stirring at 50°C for 3 hours. The reaction solution was concentrated under reduced pressure to obtain a residue, and methanol was added to the residue to precipitate the title compound from the solution. The precipitated solid was collected by filtration and washed with a small amount of methanol, followed by drying at 65°C under reduced pressure, thereby obtaining the CE compound (342.3 mg).

1H-NMR (400 MHz, CDCl3) δ 8.35 (1H, d, J=7.3 Hz), 6.84 (2H, t, J=2.4 Hz), 6.84-6.75 (1H, m), 6.53-6.36 (3H, m), 5.74-5.66 (1H, m), 5.55-5.40 (1H, m), 4.10-3.85 (5H, m), 3.82-3.68 (1H, m), 3.79 (6H, s), 2.79-2.72 (2H, m), 2.64-2.32 (2H, m); m/z 491.3 [M+H]+.

Production Example 5: Synthesis of 1-((S)-3-(4-((3-((S)-3-(4-amino-3-((3,5-dimethoxyphenyl)ethynyl)-1H-pyrazolo[3,4-d]pyrimidin-1-yl)pyrrolidin-1-yl)-3-oxopropyl)amino)-3-((3,5-dimethoxyphenyl)ethynyl)-1H-pyrazolo[3,4-d]pyrimidin-1-yl) pyrrolidin-1-yl)prop-2-en-1-one (Dimer compound)

2 Mesylate of compound B (2.751 g) obtained in Production Example 1 was dissolved in a 50% aqueous acetonitrile solution, and the obtained solution was added dropwise to a 0.5M sodium hydroxide solution. The obtained suspension was extracted with chloroform, and the extract was dried over anhydrous sodium sulfate and concentrated under reduced pressure, thereby obtaining a desalted product of compound B obtained in Production Example 4 (1.753 g).

The thus-obtained desalted product (268.2 mg) and the CE compound (301.0 mg) obtained in Production Example 104 were dissolved in a solvent mixture of DMSO (15 mL) and water (1.5 mL), and then DMT-MM·monohydrate (274.4 mg) was added thereto, followed by stirring at room temperature for 2 hours and then stirring at 50°C for 1 hour. DMT-MM·monohydrate (38.5 mg) was further added, and the mixture was further stirred at 50°C for 1 hour, followed by cooling to room temperature. Water was added to the reaction solution, followed by extraction with chloroform. After the extract was dried over anhydrous sodium sulfate, the residue obtained by concentration under reduced pressure was purified by silica gel chromatography (Biotage, SNAP Ultra HP-Sphere, 110 g, eluate: chloroform/methanol), thereby obtaining the Dimer compound (360.7 mg).

1H-NMR (400 MHz, CDCl3) δ 8.38-8.27 (2H, m), 7.30-7.13 (1H, m), 7.08 (1H, dd, J=2.2, 7.8 Hz), 7.03 (1H, d, J=2.2 Hz), 6.71 (1H, d, J= 2.4 Hz), 6.70 (1H, dd, J=2.2, 5.1 Hz), 6.56-6.47 (2H, m), 6.45-6.36 (2H, m), 6.00-5.87 (2H, m), 5.74-5.65 (1H, m), 5.58-5.43 (2H, m), 4.17-3.56 (10H, m), 3.86-3.75 (12H, m), 2.75-2.31 (6H, m); m/z 837.6 [M+H]+.

Production Example 6: Synthesis of (S)-8-(1-acryloylpyrrolidin-3-yl)-10-((3,5-dimethoxyphenyl)ethynyl)-3,4-dihydropyrazolo[4,3-e]pyrimido[1,2-c]pyrimidin-2(8H)-one (CDA1 compound)

Compound A (500.1 mg) disclosed in PTL 1 was dissolved in chloroform (10 mL), and 4-dimethylaminopyridine (358.9 mg) and acryloyl chloride (234.0 μL) were added thereto, followed by stirring at room temperature for 2 hours. A saturated aqueous solution of sodium hydrogen carbonate was added to the reaction solution, followed by extraction with chloroform. The extract was dried over anhydrous sodium sulfate and concentrated under reduced pressure. The obtained residue was purified by silica gel chromatography (Biotage, SNAP Ultra HP-Sphere, 50 g, eluate: chloroform/methanol), thereby obtaining the CDA1 compound (232.8 mg) .

1H-NMR (400 MHz, DMSO-d6) δ 8.41 (1H, d, J=2.0 Hz), 6.82 (2H, d, J=2.4 Hz), 6.61 (1H, ddd, J=10.2, 17.0, 37.1 Hz), 6.58 (1H, t, J= 2.4 Hz), 6.16 (1H, ddd, J=2.4, 6.3, 17.0 Hz), 5.69 (1H, ddd, J= 2.4, 10.2, 16.1 Hz), 5.56-5.42 (1H, m), 4.36 (2H, t, J=7.3 Hz), 4.14-3.92 (1H, m), 3.96-3.73 (1H, m), 3.86-3.57 (1H, m), 3.78 (6H, s), 2.64 (2H, t, J=7.3 Hz), 2.56-2.23 (2H, m); m/z 473.3 [M+H]+.

Production Example 7: Synthesis of (S)-N-(1-(1-acryloylpyrrolidin-3-yl)-3-((3,5-dimethoxyphenyl)ethynyl)-1H-pyrazolo[3,4-d]pyrimidin-4-yl)acrylamide (diamide compound)

Compound A disclosed in PTL 1 (3.000 g) was dissolved in DMF (50 mL). While the mixture was stirred at room temperature, 60% sodium hydride (powder dispersed in liquid paraffin, 440.0 mg) was added thereto, followed by stirring for 5 minutes. 3-Chloropropanoic acid chloride (1.40 mL) was added, followed by stirring at room temperature for 20 minutes. A saturated aqueous solution of ammonium chloride was added to the obtained gel-like reaction solution until the gel state cleared away, followed by adding a saturated aqueous solution of sodium hydrogen carbonate and extraction with ethyl acetate. The extract was washed with a saturated sodium chloride solution and dried over anhydrous sodium sulfate and concentrated under reduced pressure. The thus-obtained residue was purified by silica gel chromatography (Biotage, SNAP Ultra HP-Sphere, 100 g, eluate: ethyl acetate/methanol), thereby obtaining the diamide compound (374.3 mg).

1H-NMR (400 MHz, CDCl3) δ 8.84 (1H, s), 8.72 (1H, s), 7.43-7.31 (1H, m), 6.85 (2H, d, J=2.4 Hz), 6.75-6.34 (3H, m), 6.56 (1H, q, J=2.4 Hz), 5.96 (1H, ddd, J=1.2, 4.1, 10.5 Hz), 5.77-5.69 (1H, m), 5.67-5.56 (1H, m), 4.48-3.74 (4H, m), 3.84 (6H, s), 2.75-2.42 (2H, m); m/z 473.3 [M+H]+.

Production Example 8: Isolation of (S)-1-(1-acryloylpyrrolidin-3-yl)-5-amino-3-((3,5-dimethoxyphenyl)ethynyl)-1H-pyrazole-4-carbonitrile (UK compound)

In accordance with the method disclosed in Example 1 of PTL 4, compound A obtained in Production Examples 4, 5, and 6 was crystallized and filtered. Chloroform (40 mL) was added to the filtrate (40 mL), followed by extraction twice. The extracts were combined and concentrated under reduced pressure, followed by purifying the obtained residue by silica gel column chromatography (Biotage, SNAP Isolute Flash-NH, 11 g, eluate: ethyl acetate/methanol) and silica gel preparative thin-layer chromatography (Merck, Cat. No. 1.05744, developing solvent: chloroform/methanol), thereby obtaining the UK compound (2.5 mg).

1H-NMR (400 MHz, CDCl3) δ 6.75-6.71 (2H, m), 6.53-6.43 (2H, m), 6.42-6.35 (1H, m), 5.78-5.70 (1H, m), 4.80-4.60 (1H, m), 4.73 (2H, s), 4.07-3.86 (2H, m), 3.86-3.60 (2H, m), 3.80 (6H, s), 2.78-2.27 (2H, m); m/z 392.3 [M+H]+.

Example 1: Analysis of Compound A by Liquid Chromatography

Measurement by HPLC was performed under the test conditions described below. The solution of compound A used in the measurement by HPLC was prepared in the following manner. 50 mL of a mixture of water and acetonitrile (1:1) was added to compound A (20 mg: its preparation also being usable) obtained in Production Example 4, 5, or 6 to dissolve compound A. 1 mg of the CE compound, the OH compound, the MA compound, the Dimer compound, and the CHO compound was weighed, and 2 mL of the solution of compound A in a mixture of water and acetonitrile (1:1) was added thereto. A mixture of water and acetonitrile (1:1) was further added to precisely form 100 mL of the solution.

Conditions: Example 1

Detector: Ultraviolet absorption photometer (wavelength: 220 nm) Column: A stainless steel tube (inner diameter: 4.6 mm, length: 15 cm) was filled with 3-μm octadecylsilanized silica gel for liquid chromatography (InertSustain C18HP, produced by GL Sciences Inc.)

Column Temperature: 40°C
Injection Volume: 10 μL
Flow Rate: 1.0 mL/min
Mobile Phase A: 2.04 g of potassium dihydrogenphosphate was dissolved in 1500 mL of water, and an 8 mol/L potassium hydroxide reagent was added thereto to adjust the pH to 6.6, followed by adding 500 mL of acetonitrile.
Mobile Phase B: Acetonitrile

The proportion of mobile phase A and mobile phase B in the mixture was changed as shown below to control the concentration gradient.

Table 9

| Time after Injection (min) | Mobile Phase A (vol%) | Mobile Phase B (vol%) |
|---|---|---|
| 0 to 10 | 100 → 90 | 0 → 10 |
| 10 to 25 | 90 → 70 | 10 → 30 |
| 25 to 40 | 70 → 50 | 30 → 50 |
| 40 to 40.1 | 50 → 100 | 50 → 0 |
| 40.1 to 50 | 100 | 0 |

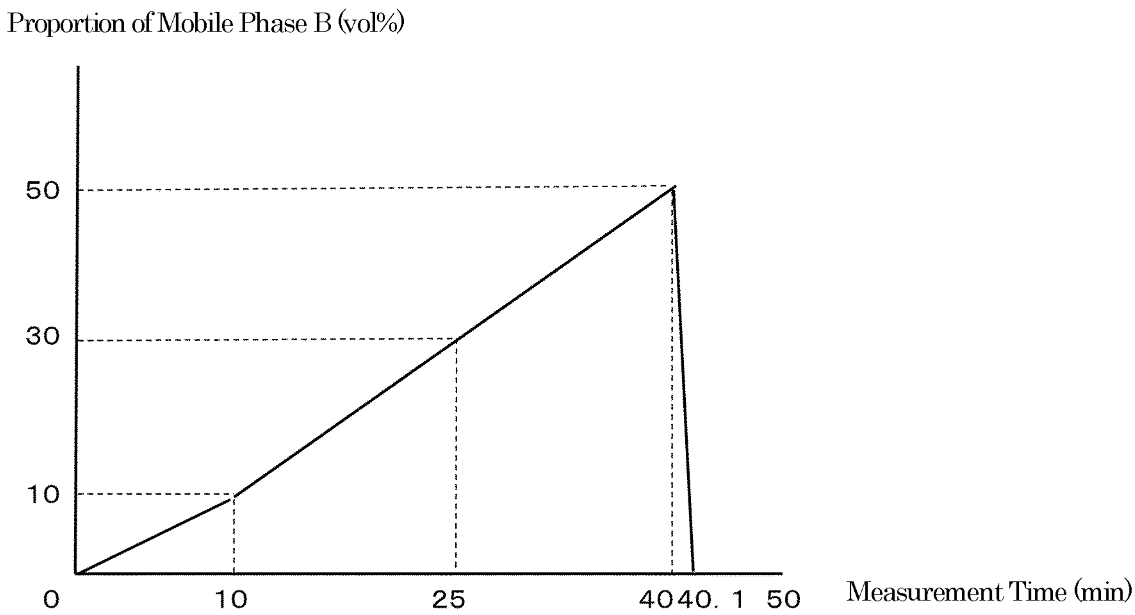


Fig. 1 illustrates the measurement results. This measurement method confirmed that the retention time of compound A was about 18.9 minutes. The measurement method also confirmed that the retention time of the CE compound was about 8.7 minutes, the retention time of the OH compound was about 12.6 minutes, the retention time of the MA compound was about 24.1 minutes, the retention time of the Dimer compound was about 27.3 minutes, and the retention time of the CHO compound was about 15.2 minutes.
Additionally, Table 10 illustrates the results of evaluation of the resolution between the peaks of these related substances and compound A.

Table 10

| Compound | CE Compound | OH Compound | CHO Compound | Compound A | MA Compound | Dimer Compound |
|---|---|---|---|---|---|---|
| Retention Time (min) | 8.7 | 12.6 | 15.2 | 18.9 | 24.1 | 27.3 |
| Resolution | | >1.5 | >1.5 | >1.5 | >1.5 | >1.5 |

In the resolution row in the table, the field of a compound that has a longer retention time than another compound with which the compound was compared for resolution indicates the resolution between their peaks. For example, the field of resolution of the OH compound indicates the resolution between the OH compound, which has a longer retention time, and the CE compound, which has a shorter retention time. The field of resolution of the CHO compound indicates the resolution between the CHO compound, which has a longer retention time, and the OH compound, which has a shorter retention time.

The results indicate that the resolution was 1.5 or higher between any peaks, and that peaks were completely separated. Thus, the conditions for HPLC in Example 1 were confirmed to be able to control the quality of compound A.

Example 2: Analysis of Compound A by Liquid Chromatography

Measurement by HPLC was performed under the test conditions described below. The solution of compound A used in the measurement by HPLC was the same as in Example 1.

Conditions: Example 2

Detector: Ultraviolet absorption photometer (wavelength: 220 nm) Column: A stainless steel tube (inner diameter: 4.6 mm, length: 15 cm) was filled with 3-μm octadecylsilanized silica gel for liquid chromatography (InertSustain C18HP, produced by GL Sciences Inc.)
Column Temperature: 40°C
Injection Volume: 5 μL
Flow Rate: 1.0 mL/minute
Mobile Phase A: 2.04 g of potassium dihydrogenphosphate was dissolved in 1500 mL of water, and an 8 mol/L potassium hydroxide reagent was added thereto to adjust the pH to 6.6, followed by adding 500 mL of acetonitrile.
Mobile Phase B: Acetonitrile

The proportion of mobile phase A and mobile phase B in the mixture was changed as shown below to control the concentration gradient.

Table 11

| Time after Injection (min) | Mobile Phase A (vol%) | Mobile Phase B (vol%) |
|---|---|---|
| 0 to 10 | 100 → 90 | 0 → 10 |
| 10 to 25 | 90 → 70 | 10 → 30 |
| 25 to 40 | 70 → 50 | 30 → 50 |
| 40 to 40.1 | 50 → 100 | 50 → 0 |
| 40.1 to 50 | 100 | 0 |

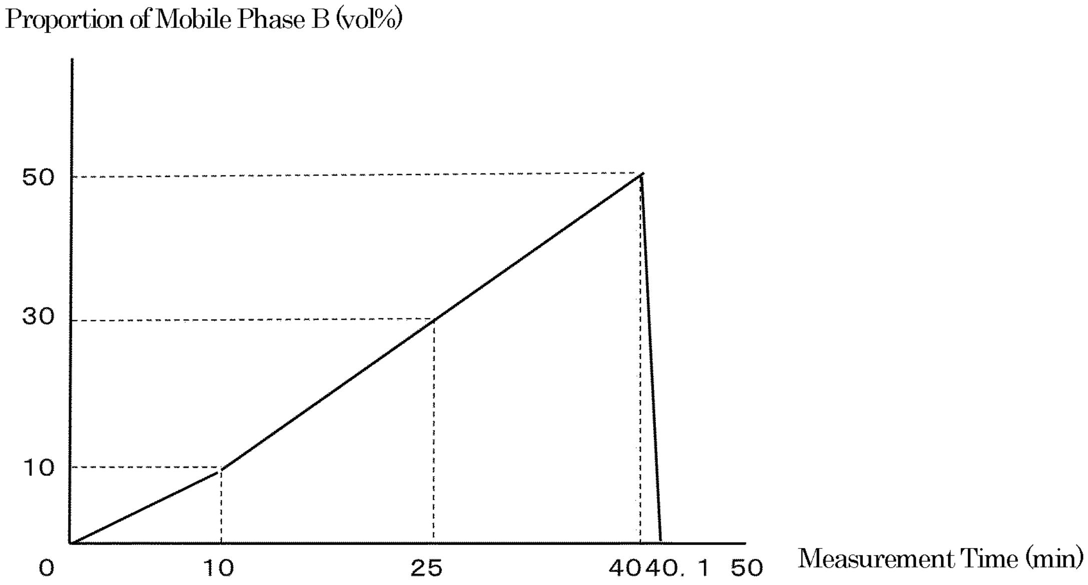


This measurement method confirmed that the retention time of compound A was about 18.7 minutes. The retention time of the other related substances was equivalent to that in Example 1. The results indicate that the measurement conditions can separate every related substance, including compound A.

Example 3: Analysis of Compound A by Liquid Chromatography

Measurement by HPLC was performed under the test conditions described below. The solution of compound A used in the measurement by HPLC was the same as in Example 1.

Conditions: Example 3

Detector: Ultraviolet absorption photometer (wavelength: 220 nm) Column: A stainless steel tube (inner diameter 4.6 mm, length 15 cm) was filled with 3-μm octadecylsilanized silica gel for liquid chromatography (InertSustain C18HP, produced by GL Sciences Inc.) Column Temperature: 40°C
Injection Volume: 5 μL
Flow Rate: 1.0 mL/minute
mobile phase A: 2.04 g of potassium dihydrogenphosphate was dissolved in 1500 mL of water, and an 8 mol/L potassium hydroxide reagent was added thereto to adjust the pH to 6.6, followed by adding 500 mL of acetonitrile.
Mobile Phase B: Acetonitrile

The proportion of mobile phase A and mobile phase B in the mixture was changed as shown below to control the concentration gradient.

Table 12

| Time after Injection (min) | Mobile Phase A (vol%) | Mobile Phase B (vol%) |
|---|---|---|
| 0 to 10 | 100 → 90 | 0 → 10 |
| 10 to 25 | 90 → 65 | 10 → 35 |
| 25 to 50 | 65 → 35 | 35 → 65 |
| 50 to 50.1 | 35 → 100 | 65 → 0 |
| 50.1 to 60 | 100 | 0 |

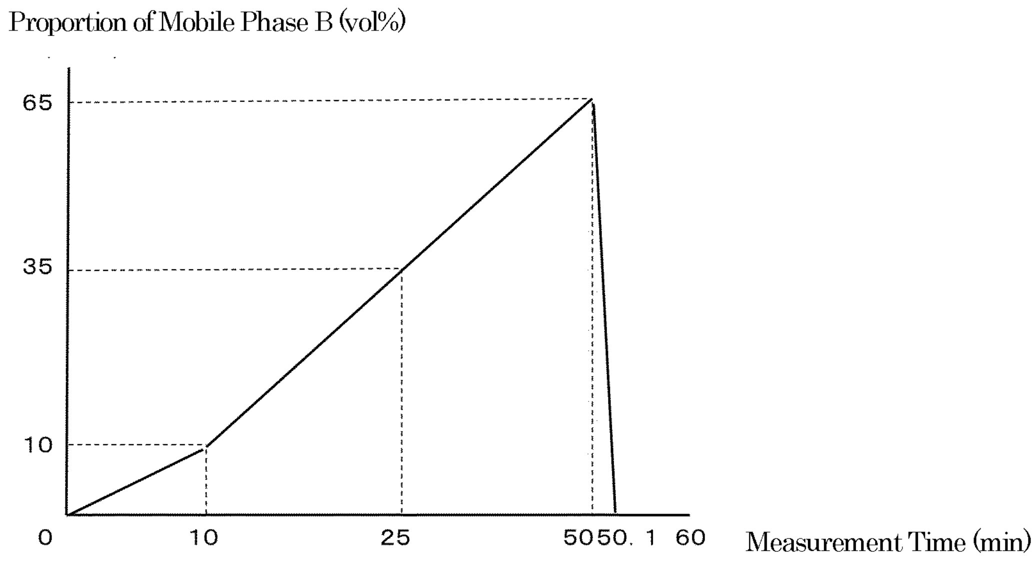


This measurement method confirmed that the retention time of compound A was about 18.1 minutes. The measurement method also confirmed that the retention time of the CE compound was about 8.5 minutes, the retention time of the OH compound was about 12.3 minutes, the retention time of the MA compound was about 22.6 minutes, the retention time of the Dimer compound was about 25.4 minutes, and the retention time of the CHO compound was about 14.7 minutes. The results indicate that the measurement conditions can separate every related substance, including compound A.

Example 4: Analysis of Compound A by Liquid Chromatography

Measurement by HPLC was performed under the test conditions described below. The solution of compound A used in the measurement by HPLC was the same as in Example 1.

Conditions: Example 4

Detector: Ultraviolet absorption photometer (wavelength: 220 nm) Column: A stainless steel tube (inner diameter: 4.6 mm, length: 15 cm) was filled with 3-μm octadecylsilanized silica gel for liquid chromatography (InertSustain C18HP, produced by GL Sciences Inc.)
Column Temperature: 40°C
Injection Volume: 5 μL
Flow Rate: 1.0 mL/minute
Mobile Phase A: 2.04 g of potassium dihydrogenphosphate was dissolved in 1500 mL of water, and an 8 mol/L potassium hydroxide reagent was added thereto to adjust the pH to 6.6, followed by adding 500 mL of acetonitrile.
Mobile Phase B: Acetonitrile

The proportion of mobile phase A and mobile phase B in the mixture was changed as shown below to control the concentration gradient.

Table 13

| Time After Injection (min) | Mobile Phase A (vol%) | Mobile Phase B (vol%) |
|---|---|---|
| 0 to 10 | 100 → 90 | 0 → 10 |
| 10 to 30 | 90 → 80 | 10 → 20 |
| 30 to 40 | 80 → 30 | 20 → 70 |
| 40 to 45 | 30 | 70 |
| 45 to 45. 1 | 30 → 100 | 70 → 0 |
| 45. 1 to 60 | 100 | 0 |

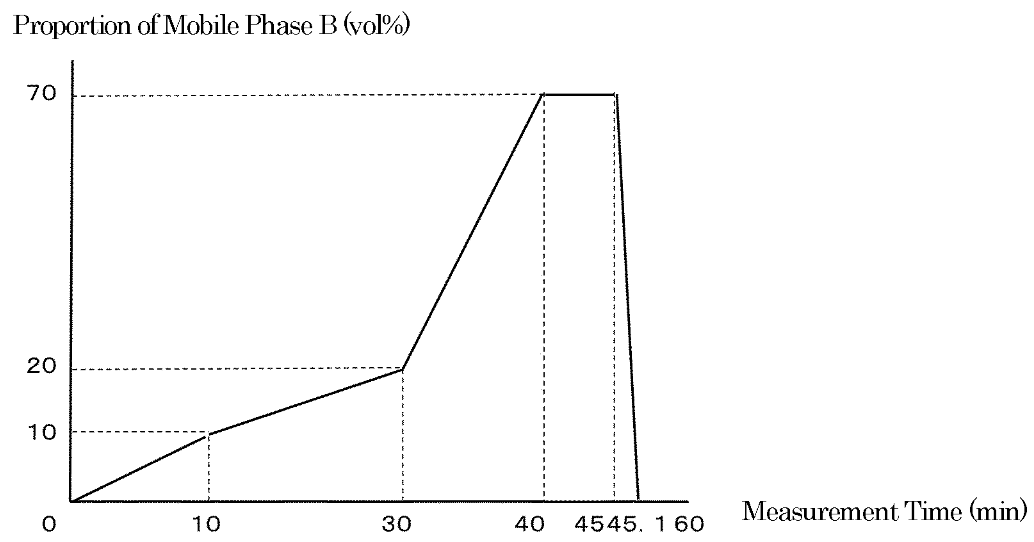


This measurement method confirmed that the retention time of compound A was about 18.1 minutes. The measurement method also confirmed that the retention time of the CE compound was about 8.5 minutes, the retention time of the OH compound was about 12.3 minutes, the retention time of the MA compound was about 22.6 minutes, the retention time of the Dimer compound was about 25.4 minutes, and the retention time of the CHO compound was about 14.7 minutes. The results indicate that the measurement conditions can separate every related substance, including compound A.

Example 5: Analysis of Compound A by Liquid Chromatography

Measurement by HPLC was performed under the test conditions described below. The analysis was performed in the same manner as in Example 4 except for the HPLC conditions described below.

Conditions: Example 5

Mobile Phase A: 2.72 g of potassium dihydrogenphosphate was dissolved in 1800 mL of water, and a 0.2 mol/L sodium hydroxide reagent was added thereto to adjust the pH to 6.6, followed by adding 500 mL of acetonitrile to 1500 mL of this solution.
The proportion of mobile phase A and mobile phase B in the mixture was changed as shown below to control the concentration gradient.

Table 14

| Time after Injection (min) | Mobile Phase A (vol%) | Mobile Phase B (vol%) |
|---|---|---|
| 0 to 10 | 100 → 90 | 0 → 10 |
| 10 to 30 | 90 → 80 | 10 → 20 |
| 30 to 40 | 80 → 30 | 20 → 70 |
| 40 to 45 | 30 | 70 |
| 45 to 45. 1 | 30 → 100 | 70 → 0 |
| 45. 1 to 55 | 100 | 0 |

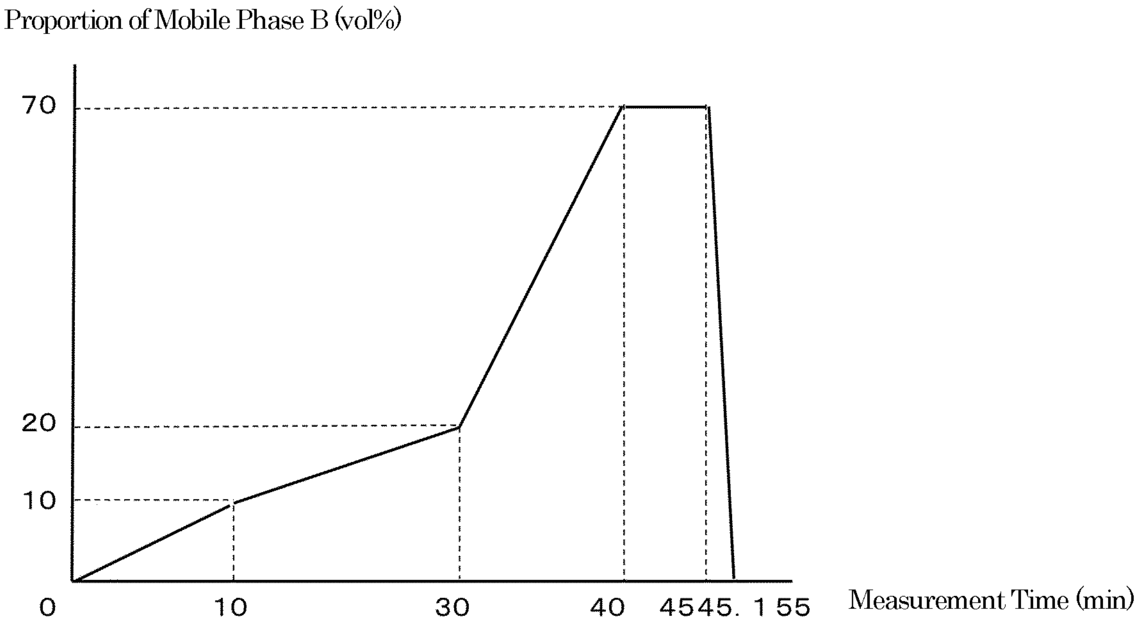


This measurement method confirmed that the retention time of compound A was about 19.9 minutes. The measurement method also confirmed that the retention time of the CE compound was about 8.3 minutes, the retention time of the OH compound was about 12.2 minutes, the retention time of the MA compound was about 30.2 minutes, the retention time of the Dimer compound was about 35.1 minutes, and the retention time of the CHO compound was about 14.9 minutes. The results indicate that the measurement conditions can separate every related substance, including compound A.

Example 6: Analysis of Compound A by Liquid Chromatography

Measurement by HPLC was performed under the test conditions described below. The solution of compound A used in the measurement by HPLC was the same as in Example 1.

Conditions: Example 6

Detector: Ultraviolet absorption photometer (wavelength: 220 nm) Column: A stainless steel tube (inner diameter: 4.6 mm, length: 15 cm) was filled with 3-μm octadecylsilanized silica gel for liquid chromatography (InertSustain C18HP, produced by GL Sciences Inc.)
Column Temperature: 40°C
Injection Volume: 5 μL
Flow Rate: 1.0 mL/minute
Mobile Phase A: 2.04 g of potassium dihydrogenphosphate was dissolved in 1500 mL of water, and an 8 mol/L potassium hydroxide reagent was added thereto to adjust the pH to 6.6, followed by adding 500 mL of acetonitrile.
Mobile Phase B: Acetonitrile

The proportion of mobile phase A and mobile phase B in the mixture was changed as shown below to control the concentration gradient.

Table 15

| Time after Injection (min) | Mobile Phase A (vol%) | Mobile Phase B (vol%) |
|---|---|---|
| 0 to 10 | 100 → 90 | 0 → 10 |
| 10 to 30 | 90 → 80 | 10 → 20 |
| 30 to 40 | 80 → 30 | 20 → 70 |
| 40 to 45 | 30 | 70 |

(continued)

| Time after Injection (min) | Mobile Phase A (vol%) | Mobile Phase B (vol%) |
|---|---|---|
| 45 to 45. 1 | 30 → 100 | 70 → 0 |
| 45. 1 to 55 | 100 | 0 |

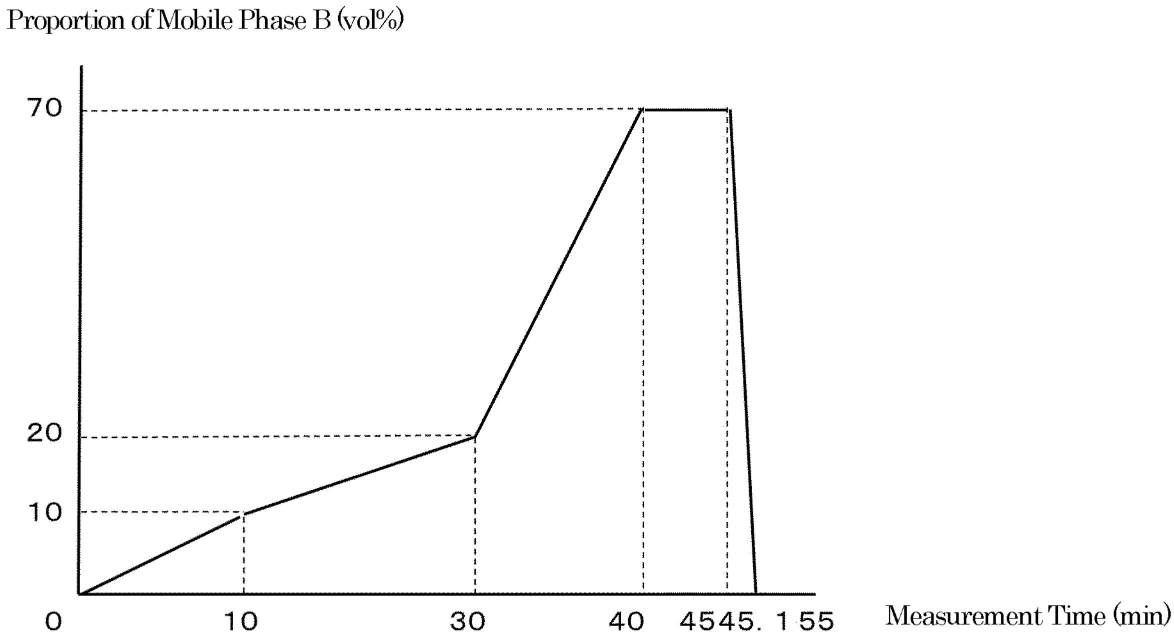


This measurement method confirmed that the retention time of compound A was about 19.8 minutes. The related substances were also well separated. This indicates that the measurement conditions can separate every related substance, including compound A.

Test Example 2: Confirmation of Resolution

At a pH of 6.8 under the conditions in Example 1, the behavior of compound B was confirmed while the pH was changed to 6.3, 6.6, 7.0, and 7.3 without changing other conditions. Table 16 illustrates the results.

Table 16

| Compound | Parameter | CE Compound | Compound B |
|---|---|---|---|
| pH 6.3 | Retention Time (min) | 8.3 minutes | 6.7 minutes |
| | Resolution | 7.1 | |
| pH 6.6 | Retention Time (min) | 8.4 minutes | 7.5 minutes |
| | Resolution | 4.8 | |
| pH 6.8 | Retention Time (min) | 8.5 minutes | 8.1 minutes |
| | Resolution | 2.9 | |
| pH 7.0 | Retention Time (min) | 8.2 minutes | 8.2 minutes |
| | Resolution | | - |
| pH 7.3 | Retention Time (min) | 8.5 minutes | 10.0 minutes |
| | Resolution | | 4.8 |

The field of a compound that has a longer retention time than another compound with which the compound was compared for resolution indicates the resolution between their peaks. The symbol "-" indicates that the peaks of

the CE compound and compound B overlapped, and their resolution was not calculated. This indicates that if compound B is contained in the drug substance or preparation of compound A, the conditions are acceptable for HPLC measuring compound A except for the condition of pH at 7.0.

## Claims

1. A compound represented by any one of the following formulas (1) to (5) or a salt thereof, or a combination thereof

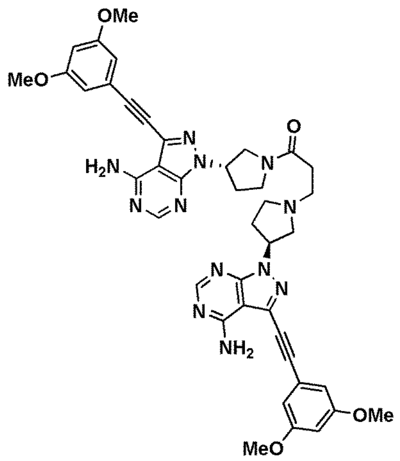

(1)

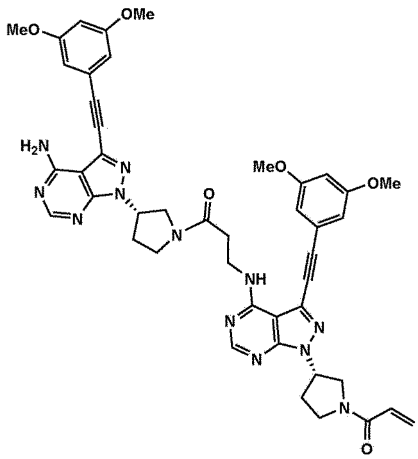

(2)

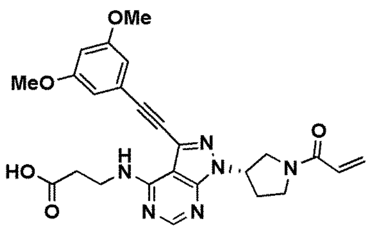

(3)

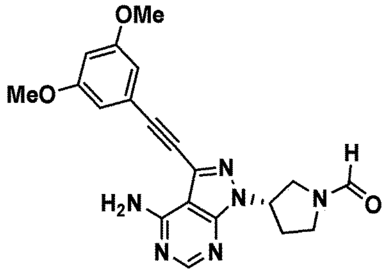

(4)

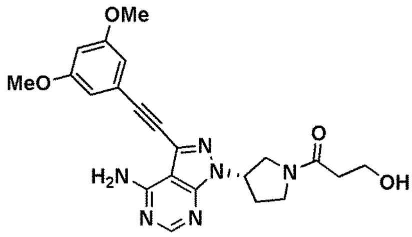

(5)

.

2. The compound or a salt thereof, or a combination thereof according to claim 1, wherein the compound is represented by formula (1) or (2).

3. A compound represented by any one of the following formulas (1) to (5) or a salt thereof, or a combination thereof, the compound being for use as a standard for controlling quality of (S)-1-(3-(4-amino-3-((3,5-dimethoxyphenyl)ethynyl)-1H-pyrazolo[3,4-d]pyrimidin-1-yl)pyrrolidin-1-yl)-2-propen-1-one

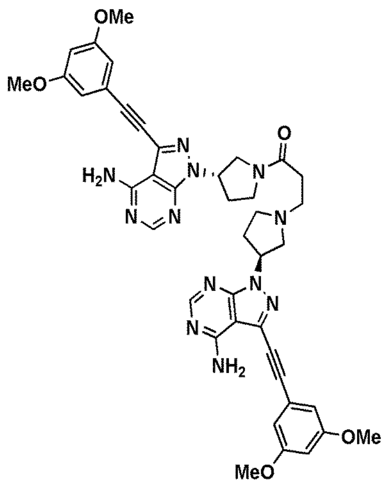

(1)

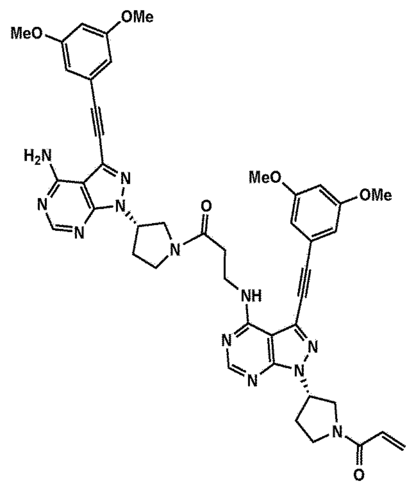

(2)

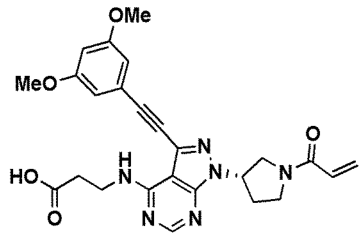

(3)

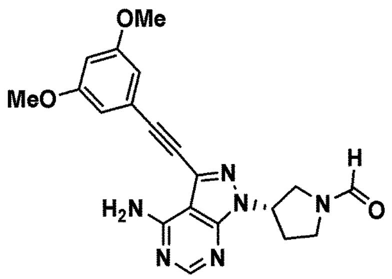

(4)

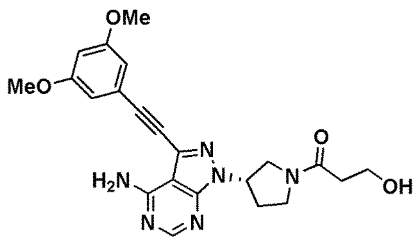

(5)

.

4. The compound or a salt thereof, or a combination thereof according to claim 3, wherein the compound is represented by formula (1) or (2).

5. A method for analysis of (S)-1-(3-(4-amino-3-((3,5-dimethoxyphenyl)ethynyl)-1H-pyrazolo[3,4-d]pyrimidin-1-yl)pyrrolidin-1-yl)-2-propen-1-one by high-performance liquid chromatography, wherein resolutions between compounds of the group consisting of a compound represented by the following formula (1), a compound represented by the following formula (2), and (S)-1-(3-(4-amino-3-((3,5-dimethoxyphenyl)ethynyl)-1H-pyrazolo[3,4-d]pyrimidin-1-yl)pyrrolidin-1-yl)-2-propen-1-one are 1.5 or more as measured by high-performance liquid chromatography

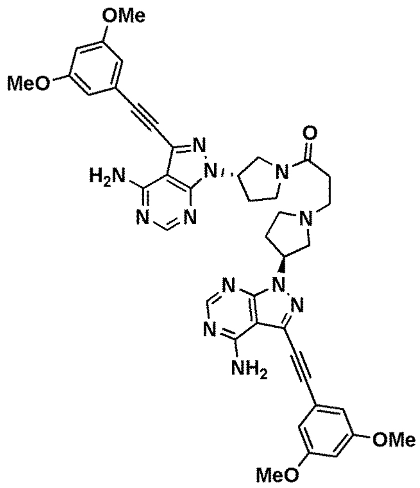

(1)

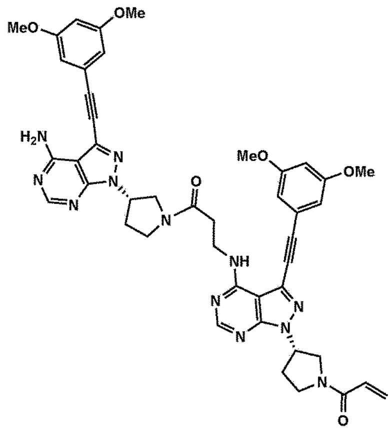

(2)

.

6. A method for analysis of (S)-1-(3-(4-amino-3-((3,5-dimethoxyphenyl)ethynyl)-1H-pyrazolo[3,4-d]pyrimidin-1-yl)pyrrolidin-1-yl)-2-propen-1-one by high-performance liquid chromatography, wherein resolutions between compounds of the group consisting of a compound represented by the following formula (1), a compound represented by the following formula (2), a compound represented by the following formula (3), a compound represented by the following formula (4), a compound represented by the following formula (5), and (S)-1-(3-(4-amino-3-((3,5-dimethoxyphenyl)ethynyl)-1H-pyrazolo[3,4-d]pyrimidin-1-yl)pyrrolidin-1-yl)-2-propen-1-one are 1.5 or more as measured by high-performance liquid chromatography

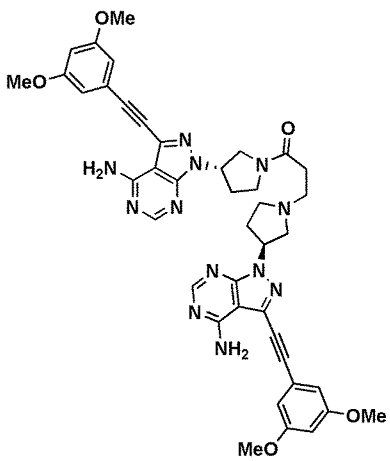

(1)

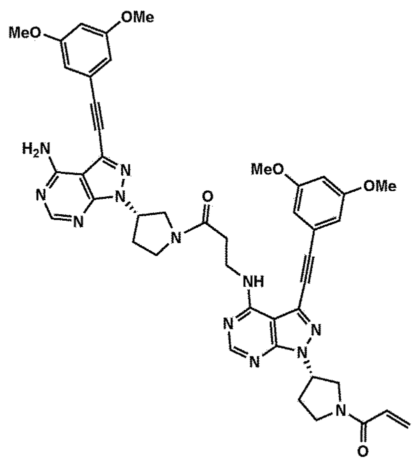

(2)

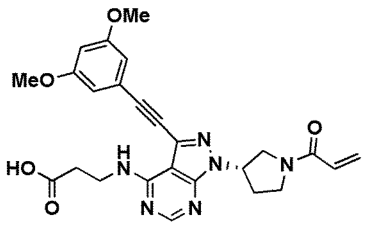

(3)

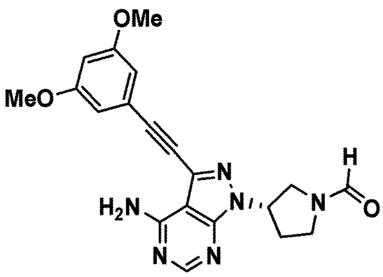

(4)

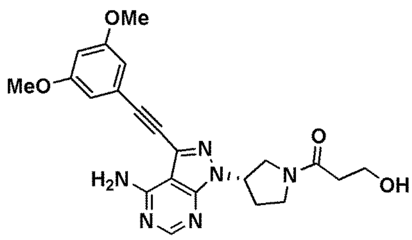

(5)

.

7. The analysis method according to claim 5 or 6, wherein a mobile phase comprises a buffer solution whose pH is adjusted to 6.4 or more and 6.8 or less using a phosphoric acid salt.

8. The analysis method according to any one of claims 5 to 7, wherein the mobile phase is a mixture of an organic phase and an aqueous phase and comprises a first gradient, a second gradient, and a third gradient, in each of which the percentage of the organic phase in the mobile phase is increased over time at a constant increase rate.

9. The analysis method according to any one of claims 5 to 8, wherein the increase rate of the organic phase in the first gradient is 0.7 to 2.0 volume%/minute, a starting time point of the first gradient is 0 to 5 minutes after a starting time point of the measurement, an ending time point of the first gradient is 5 to 15 minutes after the starting time point of the first gradient, the percentage of the organic phase at the starting time point of the first gradient is 0 to 15 mass% of the entire mobile phase, and the percentage of the organic phase at the ending time point of the first

gradient is 5 to 15 mass% of the entire mobile phase;

the increase rate of the organic phase in the second gradient is 0.3 to 2.0 volume%/minute, a starting time point of the second gradient is 0 to 10 minutes after the ending time point of the first gradient, an ending time point of the second gradient is 10 to 20 minutes after the starting time point of the second gradient, the percentage of the organic phase at the starting time point of the second gradient is the same as the percentage of the organic phase at the ending time point of the first gradient, and the percentage of the organic phase at the ending time point of the second gradient is 15 to 45 mass% of the entire mobile phase; and
the increase rate of the organic phase in the third gradient is 1.0 to 6.0 volume%/minute, a starting time point of the third gradient is 0 to 10 minutes after the ending time point of the second gradient, an ending time point of the third gradient is 10 to 20 minutes after the starting time point of the third gradient, the percentage of the organic phase at the starting time point of the third gradient is the same as the percentage of the organic phase at the ending time point of the second gradient, and the percentage of the organic phase at the ending time point of the third gradient is 45 to 75 mass% of the entire mobile phase.

**10.** The analysis method according to any one of claims 5 to 9, wherein an octadecylsilanized silica gel column is used.

**11.** The analysis method according to any one of claims 5 to 10, wherein an aqueous phase comprising acetonitrile in an amount of 15 to 40 volume% based on the total amount of the aqueous phase is used.

**12.** The analysis method according to any one of claims 5 to 11, wherein a mobile phase cleaner is used.

Fig.1

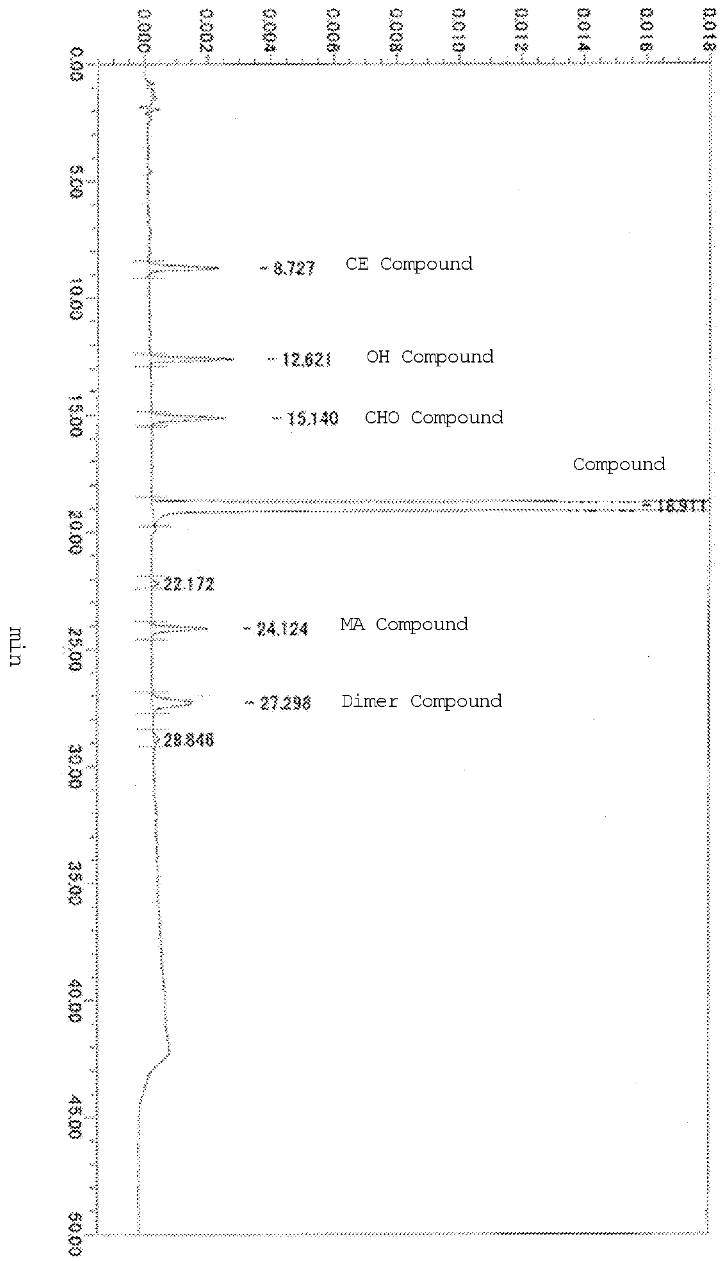

8.727 CE Compound
12.621 OH Compound
15.140 CHO Compound
Compound
18.911
22.172
24.124 MA Compound
27.298 Dimer Compound
28.846
min

**INTERNATIONAL SEARCH REPORT**

International application No. PCT/JP2019/043926

A. CLASSIFICATION OF SUBJECT MATTER
Int.Cl. C07D487/04(2006.01)i, C07D519/00(2006.01)i, G01N30/26(2006.01)i, G01N30/34(2006.01)i, G01N30/88(2006.01)i

According to International Patent Classification (IPC) or to both national classification and IPC

B. FIELDS SEARCHED

Minimum documentation searched (classification system followed by classification symbols)
Int.Cl. C07D487/04, C07D519/00, G01N30/26, G01N30/34, G01N30/88

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY(STN), CASREACT(STN)

C. DOCUMENTS CONSIDERED TO BE RELEVANT

| Category* | Citation of document, with indication, where appropriate, of the relevant passages | Relevant to claim No. |
|---|---|---|
| A | WO 2013/108809 A1 (TAIHO PHARMACEUTICAL CO., LTD.) 25 July 2013, claims, example 2 & US 2014/0343035 A1, claims, example 2 & EP 2657233 A1 & CA 2854093 A1 & KR 10-2014-0080551 A & CN 103958512 A | 1-12 |
| A | CN 105859721 A (ZHEJIANG JINGXIN PHARMACEUTICAL CO., LTD.) 17 August 2016, claims, paragraphs [0011]-[0032], examples (Family: none) | 1-12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10.12.2019 | 24.12.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/043926

C (Continuation). DOCUMENTS CONSIDERED TO BE RELEVANT

| Category* | Citation of document, with indication, where appropriate, of the relevant passages | Relevant to claim No. |
|---|---|---|
| A | JP 2017-518276 A (ZHEJIANG DTRM BIOPHARMA CO., LTD.) 06 July 2017, examples 45, 58 & WO 2015/165279 A1, examples 45, 58 & EP 3138842 A1 & CN 105017256 A | 1-12 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018041744 W
- JP 2019044236 A
- WO 2013108809 A
- WO 2015008844 A
- WO 2015008839 A
- WO 2016159327 A
- WO 2017150725 A